# EUROPEAN PATENT APPLICATION

(11) **EP 4 265 730 A1**
(43) Date of publication of application: **25.10.2023**
(21) Application number: 22168789.0
(22) Date of filing: 19.04.2022
(51) Int. Cl.: C12P 21/00, C12P 19/18, C12P 19/44

(54) **CELL-FREE ENZYMATIC METHOD FOR PREPARATION OF N-GLYCANS**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to a cell-free enzyme-catalyzed process for producing glycoproteins of general formula (I) from a lipid-linked oligosaccharide and a peptide. Further, said process includes the construction of the lipid-linked oligosaccharide from a mannose trisaccharide containing core structure. Particularly, the lipid-linked oligosaccharide is a high mannose-, complex-, or hybrid-type *N*-glycan.

## Description

### Field of the invention

The present invention relates to a cell-free enzyme-catalyzed process for producing glycoproteins of general formula (I) from a lipid-linked oligosaccharide and a peptide. Further, said process includes the construction of the lipid-linked oligosaccharide from a mannose trisaccharide containing core structure. Particularly, the lipid-linked oligosaccharide is a high mannose-, complex-, or hybrid-type *N*-glycan.

### Background of the invention

Glycosylation of peptides and proteins - the covalent attachment of glycans onto specific amino acid residues within a polypeptide chain - is crucial for their biological activity and significantly affects their physicochemical properties, stability, folding, subcellular localization, immunogenicity, antigenicity, pharmacokinetics and pharmacodynamics. Asparagine (*N*)-linked glycosylation is one of the most common co- and post-translational modifications of both intra- and extracellularly distributing proteins, which directly affects their functions, such as protein folding, stability and intercellular traffic (signal transduction). Furthermore, the majority of therapeutic proteins including monoclonal antibodies are glycosylated and the manner of glycosylation often determines protein drug stability besides the biological function. Therefore, obtaining diverse glycan structures is essential to study their function and to understand their biological roles.

Naturally occurring glycans are usually complex and are present as heterogeneous mixtures or glycoforms because glycan biosynthesis involves a series of glycosylation reactions catalyzed by specific glycosyltransferase (GT) enzymes that are co-expressed in different subcellular locations, thereby leading to multiple glycan structures in the glycoproteins. The structures of oligosaccharides are further diverse and complex due to branching of the glycan core, the addition of terminal sugars such as sialic acids, as well as the modification of carbohydrates with functional groups such as phosphate, sulfate, and acetate. Thus, glycoproteins are generally found in nature as a mixture of glycoforms sharing the same protein backbone but differ only in the glycan structure. Even though free glycans may be obtained from natural samples, the high diversity of glycan structures makes it very difficult to acquire highly pure compounds.

Thus, access to structurally homogenous glycoproteins at sufficient quantities is very limited, which also affects the fundamental understanding of glycosylation processes and their corresponding biotechnological applications. It has been proven to be a major impediment for the development of glycoprotein-based therapeutics as the consistent ratio and identity of glycoforms are essential for reproducible clinical efficacy and safety.

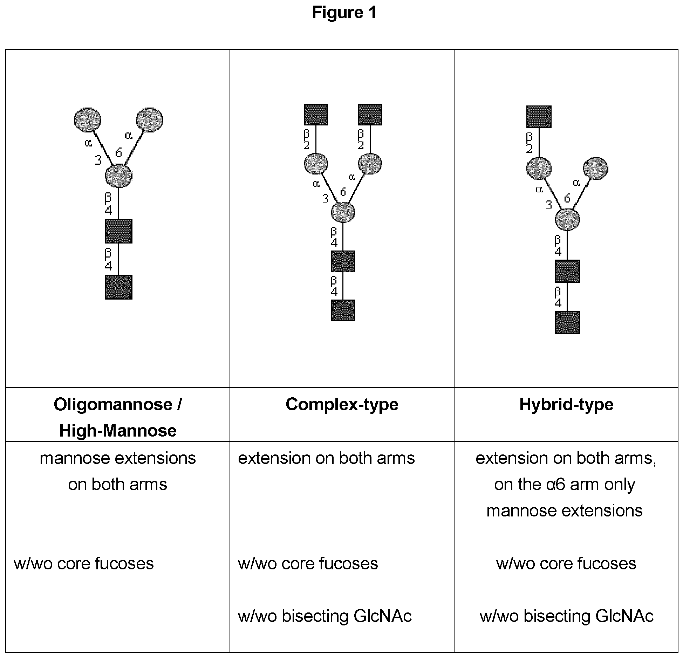

*N*-glycosylation of a peptide or protein is referred to the attachment of glycans to nitrogen of asparagine in a conserved amino acid sequence and can be achieved by using an oligosaccharyltransferase enzyme (OST). The OST transfers the assembled glycan to an asparagine residue within the N-X-T/S (wherein X represents any amino acid except proline) consensus sequon of the polypeptide chain. Starting point of the *N*-glycosylation in eukaryotes is the synthesis of lipid-linked oligosaccharides (LLOs). The LLO is assembled on a lipid tail embedded in the membrane of the endoplasmic reticulum (ER). The assembly occurs on the luminal and cytoplasmic site of the ER by a set of asparagine-linked glycosylation (ALG) glycosyltransferases and activated nucleotide sugars. Once assembled, the LLO precursor (LL-GlcNAc₂Man₉Glc₃) is transferred *en bloc* to the nascent polypeptide chain. *N*-glycans widely exist in eukaryotic cells, whose common monosaccharide building blocks include *N*-acetylglucosamine (GlcNAc), mannose (Man), glucose (Glc), galactose (Gal), fucose (Fuc), and sialic acid (Neu5Ac, Neu5Gc). They can be present as high mannose, hybrid or complex structures with a common core consisting of two GlcNAc and three mannose residues (GlcNAc₂Man₃) (see **Figure 1**).

In recent years, various synthetic methods including one-pot synthesis, solid-phase synthesis, cascade multi-enzymatic synthesis and chemo-enzymatic synthesis, have been well investigated to prepare structurally defined *N*-glycans. However, it is difficult to achieve a general synthetic method for *N*-glycans due to their complicated structures and inherent chemical properties. The chemical synthesis of highly complicated *N*-glycans typically involves performing iterative rounds of glycosylation reactions utilizing a protecting group scheme that enables functionalization of a single hydroxyl group for sugar attachment and is therefore very time-consuming and requires careful design in synthetic route and protecting groups.

To circumvent the need for protecting group manipulations, enzymatic *in vitro* approaches - cell-based or cell-free - represent a suitable alternative to chemical methods. Enzymatic glycosylation using glycosyltransferases permits precise stereo- and regio-controlled synthesis with high conversions using unprotected monosaccharides as substrates. Reactions generally proceed under mild, aqueous conditions without the need for toxic and harsh organic reagents. Thus, using bio- and/or chemoenzymatic synthesis tools, several natural and engineered glycoproteins can be in principle constructed (Jaroentomeechai et al. 2020, Front. Chem. 8:645). The two major approaches for enzymatic *in vitro* glycoengineering of proteins are transglycosylation using glycosynthases and glycomodification using Leloir glycosyltransferases. Most glycosynthases are genetically engineered glycosidases that catalyze the en-bloc transfer of oligosaccharides to *N*-acetylglucosamine (GlcNAc) and glucose moieties of glycoproteins, respectively. To achieve high product yields, oxazoline-activated oligosaccharides are used as substrates. Some oligosaccharides can be isolated from natural resources in large scales. However, if specific structures are required, tedious digestions by glycosidases, build-up of glycans by glycosyltransferases using nucleotide sugars as substrates, or elaborate product isolation are required. Glycosidases and glycosyltransferases can also be used directly to modify glycoproteins for *in vitro* glycoengineering (Li et al., Carbohydrate Research 2019, 472, 86-97). Although most glycosyltransferases are difficult to express membrane proteins, the use of glycosyltransferases at larger scales is hampered by the high costs of nucleotide sugars.

In eukaryotes, the core lipid-linked oligosaccharide GlcNAc₂Man₉Glc₃ from which the glycan is transferred to a nascent protein, is catalyzed by a cascade of membrane proteins residing in the Endoplasmic Reticulum (ER) membrane. The natural substrate for LLO synthesis in eukaryotes is dolichol (Burda and Aebi Biochimica et Biophysica Acta (BBA) - General Subjects 1999, 1426(2), 239-257). Depending on the species, dolichol consists of 14-25 isoprene units (Jones et al. Biochimica et Biophysica Acta (BBA) - General Subjects 2009, 1790(6), 485-494). Due to its lowsolubility in water, however, it cannot be used as a precursor for *in vitro* synthesis of lipid-linked oligosaccharides. Therefore, eukaryotic-type LLOs have recently been enzymatically synthesized from GDP-Mannose and a lipid-linked precursor (LL-(GlcNAc)₂) using purified mannosyltransferases from S. *cerevisiae* overexpressed in *E. coli* and HEK cells, respectively.

To date only a limited number of core lipid-linked high mannose glycans (LLOs), such as Man₃, Man₅ and Man₉ have been prepared *in vitro* by chemo-enzymatic methods using recombinant glycosyltransferases and transferred to small peptides of not more than 10 amino acids (Ramirez et al. Glycobiology 2017, 27, 726-733; Rexer et al. J. Biotechnol. 2020, 322, 54-65). In a one-pot, two compartment multi-enzyme cascade consisting of eight recombinant enzymes including the three Leloir glycosyltransferases, Alg1, Alg2 and Alg11, expressed in E. *coli* and S. *cerevisiae,* respectively, the lipid-linked oligosaccharide mannopentaose-di-(*N*-acetylglucosamine) was prepared. Nevertheless, the synthesis of eukaryotic-type lipid-linked oligosaccharides, i.e. high-mannose, complex- and hybrid-type glycans, needs to be improved significantly to synthesize milligram to gram quantities that are needed to generate viable amounts of glycoproteins. The two main challenges are: (a) the efficient synthesis of the lipid-linked precursor LL-(GlcNAc)₂ and (b) the circumvention of the use of or recycling of expensive nucleotide sugars serving as glycosyl donor, such as GDP-mannose. Typically, costs for 100 mg of GDP-Mannose are in excess of $500 from commercial suppliers. The Alg2 enzyme is only effective for LLOs having isoprenyl lipid chains longer than C₂₀-C₂₅.

Therefore, there is a need for chemo-enzymatic *in vitro* methods for the preparation of lipid-linked glycans, particularly complex- and hybrid-type glycans, as well as for the *N*-glycosylation of peptides.

Thus, it is the objective of the present invention to provide cost-effective and efficient chemo-enzymatic *in vitro* methods for the preparation of lipid-linked complex- and hybrid-type glycans as well as for the *N*-glycosylation of peptides with high-mannose, complex- and hybrid-type glycans.

The objective of the present invention is solved by the teaching of the independent claims. Further advantageous features, aspects and details of the invention are evident from the dependent claims, the description, the figures, and the examples of the present application.

### Description of the invention

The present invention is directed to an *in vitro* synthesis of synthetic lipid-linked oligosaccharides by using recombinantly expressed glycosyltransferase enzymes and the transfer of the oligosaccharides to a peptide or protein by using an oligosaccharyltransferase enzyme.

Starting from the lipid-linked core saccharide LL-GlcNAc₂Man₃ a synthetic oligosaccharide of high-mannose, complex-type or hybrid-type is constructed at the lipid moiety in solution by using recombinant glycosyltransferase enzymes and the respective activated nucleotide sugars serving as glycosyl donors. After assembling the lipid-linked oligosaccharide, the oligosaccharide is transferred from the lipid moiety to the asparagine γ-amido group of a peptide or protein thereby forming an *N*-glycan or a glycoprotein. The lipid is bound to the oligosaccharide via a pyrophosphate group, which provides the required energy for the saccharide transfer.

The inventors have surprisingly found that some glycosyltransferase enzymes show activity towards lipid-linked glycans. Those glycosyltransferases are in vivo located in the Golgi apparatus and act on glycans attached to proteins. So far, glycosyltransferase enzymes were only known for glycosylations of glycans linked to a protein or peptide. Based on this finding, a reaction matrix was developed for synthetic lipid-linked glycans of high-mannose-type, complex-type and hybrid-type by using recombinantly expressed glycosyltransferase enzymes *in vitro.* In some embodiments, transmembrane domain-deleted variants of glycosyltransferases were used that allow higher enzyme concentrations in the reaction solution, which improve efficiency of the synthesis and the production of glycoproteins in larger scale.

The inventors have further found that oligosaccharyltransferase enzyme (OST) is capable of *in vitro* transferring high-mannose-type, complex-type and hybrid-type glycans from the lipid moiety to the asparagine γ-amido group of a peptide or protein having a peptide backbone of more than 10 amino acids. So far, OST enzymes were only known for transferring high-mannose-type glycans from lipid moieties to peptides (see Ramirez et al. Glycobiology 2017, 27, 726-733; Rexer et al. J. Biotechnol. 2020, 322, 54-65). Thus, the OST enzymes used in the methods according to the invention are able to transfer complex-type and hybrid-type glycans from a lipid moiety to a peptide without the need to trim the glycan structure to GlcNAc₂Man₃. It is further remarkable, that the OST enzymes transfer oligosaccharides to longer peptides and protein exhibiting a secondary structure or a folding since due to the fact that OST enzymes operate co-translationally in protein biosynthesis, post-translational glycosylation of a target protein or peptide was not expected (see Jaroentomeechai et al. 2020, Front. Chem. 8:645).

Moreover, the inventive methods described herein, enable the preparation of glycoproteins with a well-defined homogeneous glycan structure on a milligram scale, which is crucial for investigation and elucidation of the impact of glycosylation on the functions and properties of proteins, for instance in clinical trials. In contrast, naturally occurring glycans are usually complex and are present as heterogeneous mixtures or glycoforms, such that isolation of single glycoforms is very tedious and often only possible in small amounts.

Thus, the present invention is directed to a method for producing a glycoprotein of general formula **(I)**

**[C-NH]ₒP** **( I )**

wherein C represents a carbohydrate of the following structure 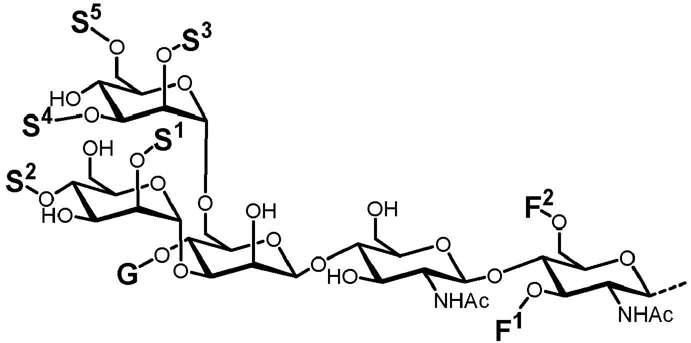
**o** is an integer representing the number of carbohydrates **C** which are bound to a peptide **P,**
**P** represents a peptide of at least 10 amino acids comprising at least o-times a consensus sequence of N-X-S/T, wherein X represents any amino acid except proline, and
**NH** represents an asparagine γ-amido group of the consensus sequence;
**F¹** and **F²** represent 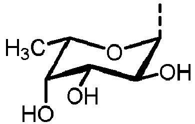 or -H; with the proviso that **F¹** and **F²** cannot be simultaneously 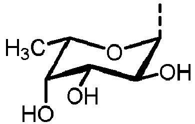
G represents 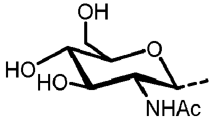 or -H;
**S¹** represents 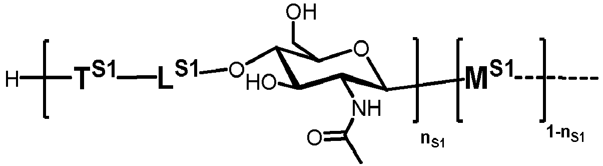
**S²** represents 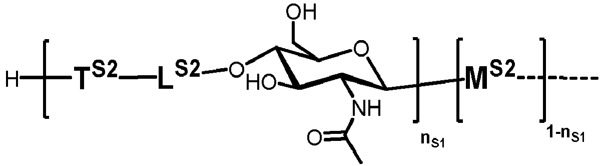
**S³** represents 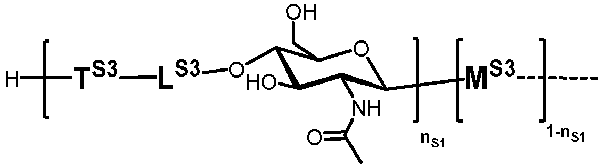
**S⁴** represents 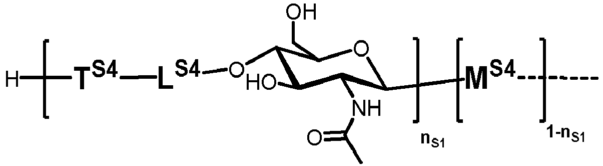
**S⁵** represents 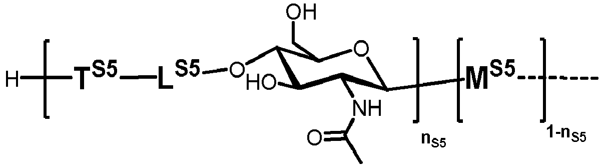
M^{S1} represents 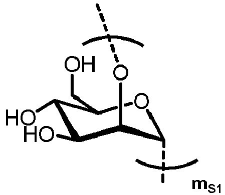
M^{S2} represents 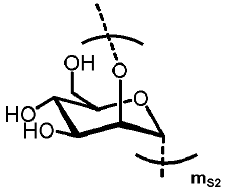
M^{S3} represents 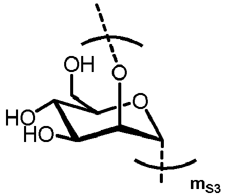
M^{S4} represents 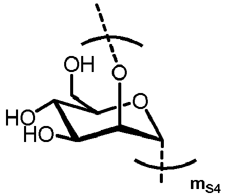
**M^{S5}** represents 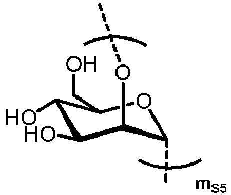
**L^{S1}** represents 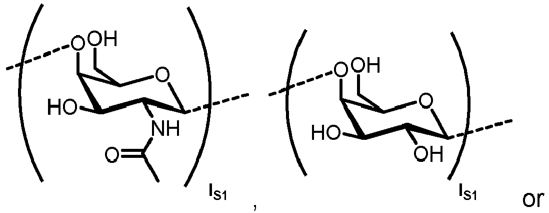 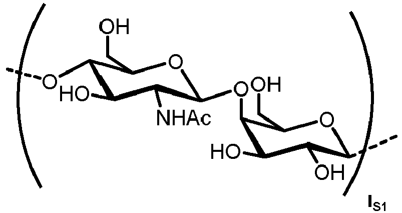
**L^{S2}** represents 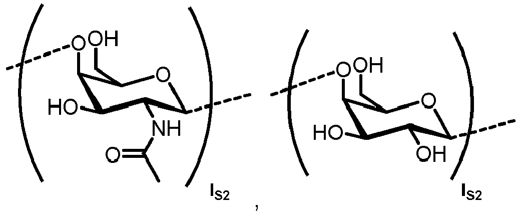 or 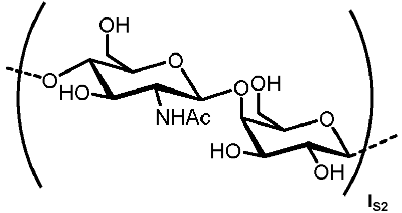
**L^{S3}** represents 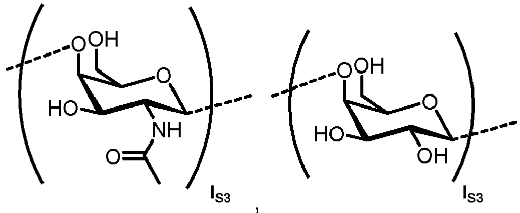 or 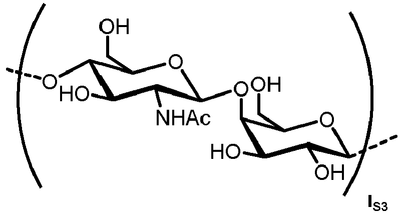
**L^{S4}** represents 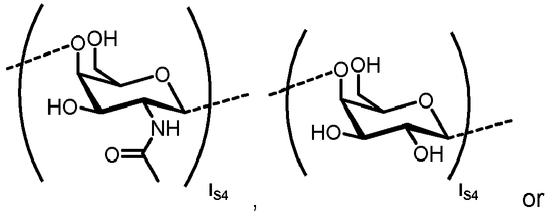 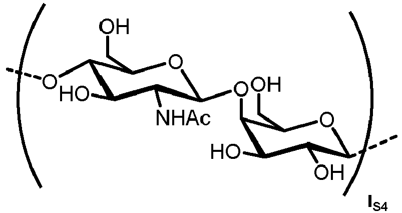
**L^{S5}** represents 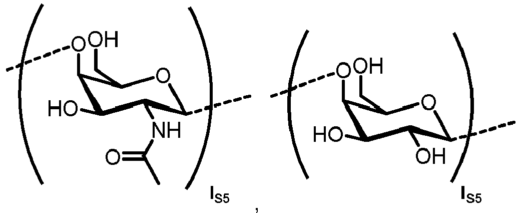 or 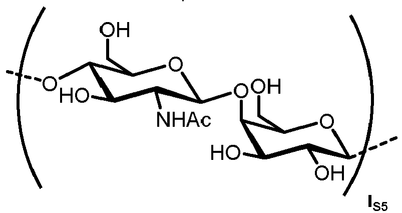
**T^{S1}**, **T^{S2}**, **T^{S3}**, **T^{S4}** and **T^{S5}** represent independently of each other: 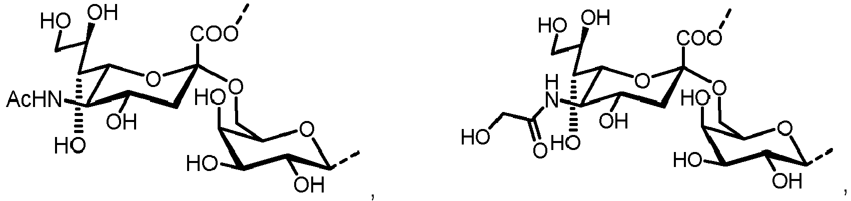 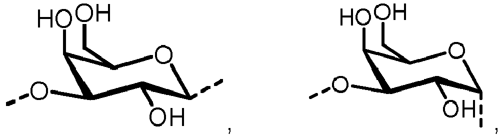 -SO₄- or a bond wherein m_{S1}, m_{S2}, m_{S3}, m_{S4}, m_{S5} I_{S1}, I_{S2}, I_{S3}, I_{S4}, and I_{S5} represent independently of each other an integer selected from 0, 1, 2, 3, 4, 5, 6, 7, 8, 9, 10, 11, 12, 13, 14 and 15,
n_{S1}, n_{S2}, n_{S3}, n_{S4} and n_{S5} represent an integer selected from 0 and 1 ,
   with the proviso that if n_{S3} = 1 then
   n_{S1} = 1 and m_{S2} = m_{S4} = m_{S5} = 0; and
if n_{S3} = 0 then
   i) n_{S2} = m_{S2} = m_{S3} = 0 , and
   ii) m_{S1} + m_{S4} + m_{S5} ≥ 0 ;
comprising the steps:
   A) providing a solution comprising a compound of formula **(II)** 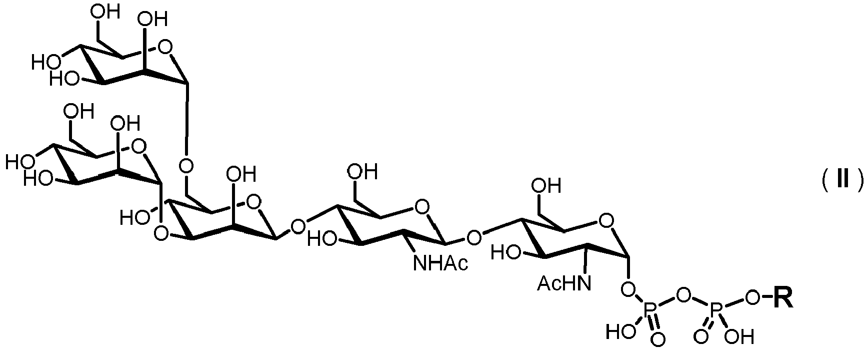 wherein R represents 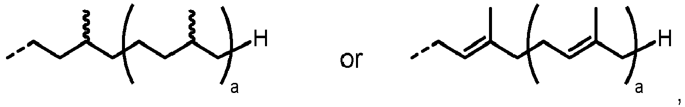 with a being an integer selected from 2, 3, 4, 5, 6, 7, 8, 9, 10, 11, 12, 13, 14 or 15,
   B) mixing the solution with at least one nucleotide sugar and at least one glycosyltransferase enzyme and reacting a resulting mixture to produce a compound of formula (III) 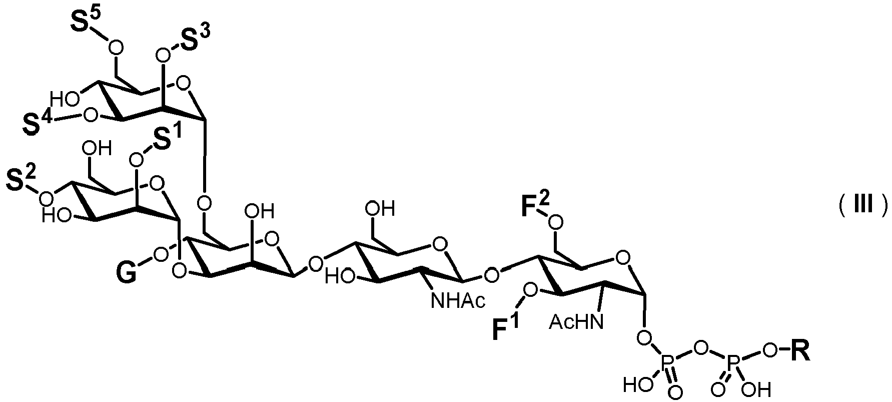 wherein the at least one nucleotide sugar is selected from GDP-mannose, UDP-galactose, UDP-GlcNAc, UDP-GaINAc, GDP-fucose, CMP-NeuAc and CMP-NeuGc,
      wherein the at least one glycosyltransferase enzyme is selected from N-acetylglucosaminyltransferase, mannosyltransferase, glucosyltransferase, galactosyltransferase, fucosyltransferase and sialyltransferase;
   C) reacting the compound of formula **(III)** with the peptide **P** in the presence of an oligosaccharyltransferase enzyme to produce the compound of formula **(I).**

The inventive methods described herein are directed to the *in vitro* preparation of glycoproteins of formula **(I)** which are *N*-glycans having an *N*-glycosidic bond between the terminal GlcNAc and an asparagine γ-amido group of the peptide **P.** The carbohydrate moiety **C** is prepared starting from a lipid-linked core structure of formula **(II).** Extension of this core structure is achieved by a set of different glycosylation reactions, which are performed *in vitro* and cell-free by using a set of recombinantly expressed glycosyltransferase enzymes, particularly *N*-acetyl-glucosaminyltransferases, mannosyltransferases, glucosyltransferases, galactosyl-transferases, fucosyltransferases and sialyltransferases, and the corresponding nucleotide sugars, which act as glycosyl donors. Suitable nucleotide sugars are GDP-mannose, UDP-galactose, UDP-GlcNAc, UDP-GaINAc, GDP-fucose, CMP-NeuAc and CMP-NeuGc. Apparently, the skilled person may readily select the appropriate glycosyltransferase enzymes and the corresponding nucleotide sugars depending on the saccharide composition of the desired glycoprotein.

The peptide **P** can be any type of a polypeptide having at least 10 amino acids and comprising at least one consensus sequence of N-X-S/T, wherein X represents any amino acid except proline, including proteins, folded peptides, folded proteins, therapeutic peptides, therapeutic proteins, aglycosylated peptides or aglycosylated proteins.

The oligosaccharyltransferase enzyme used in the inventive methods attaches the carbohydrate **C** to an asparagine γ-amido group of the consensus sequence of N-X-S/T, wherein X represents any amino acid except proline. Thus, the peptide **P** comprises at least one consensus sequence of N-X-S/T, wherein X represents any amino acid except proline. If the glycoprotein of formula **(I)** comprises **o** carbohydrate moieties **C,** the peptide **P** comprises at least o-times the consensus sequence of N-X-S/T, wherein **X** represents any amino acid except proline. The oligosaccharyltransferase enzyme can be of eukaryotic or bacterial origin.

The number **o** of carbohydrates **C** which are bound to a peptide **P** may depend on the length of the peptide **P** itself. For instance, for shorter peptides consisting of 10 to 20 amino acids **o** may be 1, thus only one carbohydrate is bound to the peptide, whereas for peptides consisting of 20 to 50 amino acids **o** may be 1 or 2, thus one or two carbohydrates are bound to the peptide, etc.

Thus, in one embodiment of the inventive method for producing a glycoprotein of general formula **(I),** o is an integer defined as follows:

| | |
|---|---|
| o is between 1 and 10 | if **P** consists of 10 to 50 amino acids |
| o is between 1 and 20 | if **P** consists of 51 to 100 amino acids |
| o is between 1 and 40 | if **P** consists of 101 to 200 amino acids |
| o is between 1 and 60 | if **P** consists of 201 to 300 amino acids |
| o is between 1 and 80 | if **P** consists of 301 to 400 amino acids |
| o is between 1 and 100 | if **P** consists of 401 to 500 amino acids |

the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
   wherein **C, NH** and **P** have the meanings as defined herein.

In a preferred embodiment, o is an integer defined as follows:

| | |
|---|---|
| o is 1 | if **P** consists of 10 to 50 amino acids |
| o is 1 or 2 | if **P** consists of 51 to 100 amino acids |
| o is 1, 2 or 3 | if **P** consists of 101 to 200 amino acids |
| o is 2, 3 or 4 | if **P** consists of 201 to 300 amino acids |
| o is 2, 3, 4 or 5 | if **P** consists of 301 to 400 amino acids |
| o is 2, 3, 4, 5 or 6 | if **P** consists of 401 to 500 amino acids. |

In a preferred embodiment, **o** is 1. Thus, the inventive method for producing a glycoprotein of general formula **(I),** comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
   wherein **C, NH** and **P** have the meanings as defined herein and **o** represents 1.

In one embodiment of the inventive method for producing a glycoprotein of general formula (I), wherein **C, NH** and **o** have the meanings as defined herein, **P** represents a peptide of at least 10 amino acids comprising at least **o-times** a consensus sequence of **N-V-T,**
the method comprises the steps:
**A)** providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

The inventors have found that the transfer of the carbohydrate **C** to the peptide **P** by an oligosaccharyltransferase enzyme is particularly efficient when the consensus sequence of peptide P is N-V-T or N-Y-T.

Thus, in one embodiment of the inventive method for producing a glycoprotein of general formula **(I),** wherein **C, NH** and **o** have the meanings as defined herein, **P** represents a peptide of at least 10 amino acids comprising at least **o-times** a consensus sequence of N-V-T or N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In one embodiment of the inventive method for producing a glycoprotein of general formula (I), wherein **C, NH** and o have the meanings as defined herein, **P** represents a peptide of at least 10 amino acids comprising at least o-times a consensus sequence of N-V-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from N-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In one embodiment of the inventive method for producing a glycoprotein of general formula (I), wherein **C, NH** and **o** have the meanings as defined herein, **P** represents a peptide of at least 10 amino acids comprising at least **o-times** a consensus sequence of N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula (III) with the peptide P in the presence of an oligosaccharyltransferase enzyme to produce the compound of formula **(I).**

In a further embodiment of the inventive method for producing a glycoprotein of general formula **(I),** wherein **C, NH** and **o** have the meanings as defined herein, **P** represents a peptide of at least 20 amino acids comprising at least **o-times** a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a preferred embodiment of the inventive method for producing a glycoprotein of general formula **(I),** wherein **C,** and **NH** have the meanings as defined herein and **o** represents 1, **P** represents a peptide of at least 20 amino acids comprising at least a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a preferred embodiment of the inventive method for producing a glycoprotein of general formula **(I),** wherein **C, NH** and **o** have the meanings as defined herein, **P** represents a peptide of at least 20 amino acids comprising at least **o-times** a consensus sequence of N-V-T or N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a further embodiment of the inventive method for producing a glycoprotein of general formula **(I),** wherein **C, NH** and **o** have the meanings as defined herein, **P** represents a folded peptide of at least 10 amino acids comprising at least **o-times** a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a further embodiment of the inventive method for producing a glycoprotein of general formula **(I),** wherein **C, NH** and **o** have the meanings as defined herein, **P** represents a folded peptide of at least 20 amino acids comprising at least **o-times** a consensus sequence of N-X-S/T, wherein X represents any amino acid except proline, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a preferred embodiment of the inventive method for producing a glycoprotein of general formula **(I),** wherein **C,** and **NH** have the meanings as defined herein and **o** represents 1, **P** represents a folded peptide of at least 20 amino acids comprising at least a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a preferred embodiment of the inventive method for producing a glycoprotein of general formula **(I),** wherein **C, NH** and **o** have the meanings as defined herein, **P** represents a folded peptide of at least 20 amino acids comprising at least **o-times** a consensus sequence of N-V-T or N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula (I).

In a preferred embodiment, **P** is an aglycosylated peptide or protein. Aglycosylated peptides or proteins can be recombinantly produced in cell-based bacterial and cell-free production systems. An aglycosylated peptide or aglycosylated protein, as used herein, is a peptide or protein which is not functionalized with a carbohydrate at an amino acid residue.

Thus, in a further embodiment of the inventive method for producing a glycoprotein of general formula (I), wherein **C, NH** and **o** have the meanings as defined herein, **P** represents an aglycosylated peptide or an aglycosylated protein of at least 10 amino acids comprising at least **o-times** a consensus sequence of N-X-S/T, wherein **X** represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a further embodiment of the inventive method for producing a glycoprotein of general formula (I), wherein **C, NH** and **o** have the meanings as defined herein, **P** represents an aglycosylated peptide or an aglycosylated protein of at least 20 amino acids comprising at least o-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a preferred embodiment of the inventive method for producing a glycoprotein of general formula **(I),** wherein **C,** and **NH** have the meanings as defined herein and **o** represents 1, **P** represents an aglycosylated peptide or an aglycosylated protein of at least 20 amino acids comprising at least a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a preferred embodiment of the inventive method for producing a glycoprotein of general formula **(I),** wherein **C, NH** and **o** have the meanings as defined herein, **P** represents an aglycosylated peptide or an aglycosylated protein of at least 20 amino acids comprising at least o-times a consensus sequence of N-V-T or N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a preferred embodiment, **P** is a therapeutic protein. Thus, in a further embodiment of the inventive method for producing a glycoprotein of general formula **(I),** wherein **C, NH** and **o** have the meanings as defined herein, P represents a therapeutic protein of at least 10 amino acids comprising at least **o-times** a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a further embodiment of the inventive method for producing a glycoprotein of general formula **(I),** wherein **C, NH** and **o** have the meanings as defined herein, **P** represents an aglycosylated therapeutic protein of at least 10 amino acids comprising at least **o-times** a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the aglycosylated therapeutic protein **P** in the presence of an oligosaccharyltransferase enzyme to produce the compound of formula **(I).**

In a further embodiment of the inventive method for producing a glycoprotein of general formula (I), wherein **C, NH** and **o** have the meanings as defined herein, **P** represents a therapeutic protein of at least 20 amino acids comprising at least o-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the therapeutic protein **P** in the presence of an oligosaccharyltransferase enzyme to produce the compound of formula **(I).**

In a preferred embodiment of the inventive method for producing a glycoprotein of general formula **(I),** wherein **C,** and **NH** have the meanings as defined herein and **o** represents 1, **P** represents a therapeutic protein of at least 20 amino acids comprising at least a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the therapeutic protein **P** in the presence of an oligosaccharyltransferase enzyme to produce the compound of formula **(I).**

In a preferred embodiment of the inventive method for producing a glycoprotein of general formula **(I),** wherein **C, NH** and **o** have the meanings as defined herein, **P** represents a therapeutic protein of at least 20 amino acids comprising at least o-times a consensus sequence of N-V-T or N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the therapeutic protein **P** in the presence of an oligosaccharyltransferase enzyme to produce the compound of formula **(I).**

In a preferred embodiment, the oligosaccharyltransferase enzyme is a eukaryotic oligosaccharyltransferase. In a more preferred embodiment, the oligosaccharyltransferase is a STT3A protein from *Trypanosoma brucei.* In contrast to oligosaccharyltransferases from higher eukaryotes, which are only present in two catalytically active isoforms STT3, the single-subunit OST of *Trypanosoma brucei* recognizes also human-like glycans and is thus more efficient for transferring glycans to a polypeptide.

In a further embodiment, the oligosaccharyltransferase enzyme is a bacterial oligosaccharyltransferase. In a further embodiment, the oligosaccharyltransferase enzyme is selected from one of the following organisms: *Campylobacter jejuni* strain RM1221, *Campylobacter lari strain* RM2100 / D67 / ATC BAA-1060, *Campylobacter coli, Campylobacter upsaliensis, Desulfovibrio desulfuricans* strain DSM 7057, *Megalodesulfovibrio gigas* DSM 1382, *Desulfovibrio vulgaris, Trypanosoma brucei, Saccharomyces cerevisiae* strain ATCC 204508 / S288c, *Mus musculus, Schizosaccharomyces pombe* strain 972 / ATCC 24843, *Canis lupus familiaris, Arabidopsis thaliana, Caenorhabditis elegans, Bos taurus, Pongo pygmaeus abelii, Oryza stiva japonica, Dictyostelium discoideum, Brachypodium distachyon, Brachypodium retusum, Rattus norvegicus, Brachypodium sylvaticum, Brachypodium pinnatum, Brachypodium rupestre,* and *Campylobacter jejuni* strain ATCC 700819 / NCTC 11168.

In a further embodiment, the oligosaccharyltransferase enzyme consists of an amino acid sequence as set forth in SEQ ID Nos 3 to 31.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a eukaryotic oligosaccharyltransferase to produce the compound of formula (I).

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of STT3A protein from *Trypanosoma brucei* to produce the compound of formula (I).

In a further embodiment of the inventive method for producing a glycoprotein of general formula **(I),** wherein **C, NH** and **o** have the meanings as defined herein, **P** represents a therapeutic protein of at least 10 amino acids comprising at least **o-**times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the therapeutic protein **P** in the presence of a eukaryotic oligosaccharyltransferase to produce the compound of formula **(I).**

In a further embodiment of the inventive method for producing a glycoprotein of general formula **(I),** wherein **C, NH** and **o** have the meanings as defined herein, **P** represents a therapeutic protein of at least 10 amino acids comprising at least **o-**times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the therapeutic protein **P** in the presence of STT3A protein from *Trypanosoma brucei* to produce the compound of formula **(I).**

In a further embodiment of the inventive method for producing a glycoprotein of general formula (I), wherein **C, NH** and **o** have the meanings as defined herein, **P** represents an aglycosylated peptide of at least 10 amino acids comprising at least **o-times** a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the aglycosylated peptide **P** in the presence of a eukaryotic oligosaccharyltransferase to produce the compound of formula **(I).**

In a further embodiment of the inventive method for producing a glycoprotein of general formula **(I),** wherein **C, NH** and **o** have the meanings as defined herein, **P** represents an aglycosylated peptide of at least 10 amino acids comprising at least **o-times** a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the aglycosylated peptide **P** in the presence of STT3A protein from *Trypanosoma brucei* to produce the compound of formula **(I).**

In a further embodiment of the inventive method for producing a glycoprotein of general formula **(I),** wherein **C, NH** and **o** have the meanings as defined herein, **P** represents a peptide of at least 20 amino acids comprising at least **o-times** a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a eukaryotic oligosaccharyltransferase to produce the compound of formula **(I).**

In a further embodiment of the inventive method for producing a glycoprotein of general formula **(I),** wherein **C, NH** and **o** have the meanings as defined herein, **P** represents a peptide of at least 20 amino acids comprising at least **o-times** a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of STT3A protein from *Trypanosoma brucei* to produce the compound of formula **(I).**

In a preferred embodiment of the inventive method for producing a glycoprotein of general formula **(I),** wherein **C,** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a eukaryotic oligosaccharyltransferase to produce the compound of formula **(I).**

In a preferred embodiment of the inventive method for producing a glycoprotein of general formula **(I),** wherein **C,** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of STT3A protein from *Trypanosoma brucei* to produce the compound of formula (I).

In a preferred embodiment of the inventive method for producing a glycoprotein of general formula **(I),** wherein **C, NH** and **o** have the meanings as defined herein, **P** represents a peptide of at least 20 amino acids comprising at least **o-times** a consensus sequence of N-V-T or N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III),** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a eukaryotic oligosaccharyltransferase to produce the compound of formula **(I).**

In a preferred embodiment of the inventive method for producing a glycoprotein of general formula (I), wherein **C, NH** and **o** have the meanings as defined herein, **P** represents a peptide of at least 20 amino acids comprising at least **o-**times a consensus sequence of N-V-T or N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III),** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of STT3A protein from *Trypanosoma brucei* to produce the compound of formula **(I).**

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N-*acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a eukaryotic oligosaccharyltransferase to produce the compound of formula (I),
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N-*acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a bacterial oligosaccharyltransferase to produce the compound of formula **(I).**

In a further embodiment of the inventive method for producing a glycoprotein of general formula **(I),** wherein **C, NH** and **o** have the meanings as defined herein, **P** represents a therapeutic protein of at least 10 amino acids comprising at least **o-**times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the therapeutic protein **P** in the presence of a bacterial oligosaccharyltransferase to produce the compound of formula **(I).**

In a further embodiment of the inventive method for producing a glycoprotein of general formula **(I),** wherein **C, NH** and **o** have the meanings as defined herein, **P** represents an aglycosylated peptide of at least 10 amino acids comprising at least **o-**times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the aglycosylated peptide **P** in the presence of a bacterial oligosaccharyltransferase to produce the compound of formula **(I).**

In a further embodiment of the inventive method for producing a glycoprotein of general formula **(I),** wherein **C, NH** and **o** have the meanings as defined herein, **P** represents a peptide of at least 20 amino acids comprising at least **o-**times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a bacterial oligosaccharyltransferase to produce the compound of formula **(I).**

In a preferred embodiment of the inventive method for producing a glycoprotein of general formula **(I),** wherein **C,** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a bacterial oligosaccharyltransferase to produce the compound of formula (I).

In a preferred embodiment of the inventive method for producing a glycoprotein of general formula **(I),** wherein **C, NH** and **o** have the meanings as defined herein, **P** represents a peptide of at least 20 amino acids comprising at least **o-**times a consensus sequence of N-V-T or N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III),** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a bacterial oligosaccharyltransferase to produce the compound of formula **(I).**

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a bacterial oligosaccharyltransferase to produce the compound of formula (I),
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N-*acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

Thus, in a further embodiment of the inventive method for producing a glycoprotein of general formula (I), wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of STT3A protein from *Trypanosoma brucei* to produce the compound of formula (I),
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N-*acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

In a further embodiment of the inventive method for producing a glycoprotein of general formula **(I),** wherein **C, NH** and **o** have the meanings as defined herein, **P** represents a therapeutic protein of at least 10 amino acids comprising at least **o-**times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the therapeutic protein **P** in the presence of an oligosaccharyltransferase enzyme to produce the compound of formula **(I),**
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N-*acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

In a further embodiment of the inventive method for producing a glycoprotein of general formula **(I),** wherein **C, NH** and **o** have the meanings as defined herein, **P** represents an aglycosylated peptide of at least 10 amino acids comprising at least **o-times** a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the aglycosylated peptide **P** in the presence of an oligosaccharyltransferase enzyme to produce the compound of formula **(I),**
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N-*acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

In a further embodiment of the inventive method for producing a glycoprotein of general formula (I), wherein C, NH and **o** have the meanings as defined herein, P represents a peptide of at least 20 amino acids comprising at least o-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N-*acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

In a preferred embodiment of the inventive method for producing a glycoprotein of general formula **(I),** wherein **C,** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N-*acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

In a preferred embodiment of the inventive method for producing a glycoprotein of general formula (I), wherein **C, NH** and **o** have the meanings as defined herein, **P** represents a peptide of at least 20 amino acids comprising at least **o-**times a consensus sequence of N-V-T or N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III),** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N-*acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

In a further embodiment, the at least one glycosyltransferase enzyme is transmembrane domain-deleted variant. Transmembrane domain-deleted variants of glycosyltransferases provide better solubilitiy and are therefore advantageous for *in vitro* processes as described herein. Thus, the inventive method for producing a glycoprotein of general formula **(I),** wherein **C, NH, P** and **o** have the meanings as defined herein, comprises the steps:
A) providing a solution comprising a compound of formula **(II)**
B) mixing the solution with at least one nucleotide sugar and at least one transmembrane domain-deleted glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one transmembrane domain-deleted glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the therapeutic protein **P** in the presence of an oligosaccharyltransferase enzyme to produce the compound of formula **(I).**

Thus, in a further embodiment of the inventive method for producing a glycoprotein of general formula (I), wherein **C, NH, P** and **o** have the meanings as defined herein, the method comprises the steps:
A) providing a solution comprising a compound of formula **(II)**
B) mixing the solution with at least one nucleotide sugar and at least one transmembrane domain-deleted glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one transmembrane domain-deleted glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a bacterial oligosaccharyltransferase to produce the compound of formula **(I).**

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one transmembrane domain-deleted glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one transmembrane domain-deleted glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of STT3A protein from *Trypanosoma brucei* to produce the compound of formula (I).

In a further embodiment of the inventive method for producing a glycoprotein of general formula (I), wherein **C, NH** and **o** have the meanings as defined herein, **P** represents an aglycosylated peptide of at least 10 amino acids comprising at least **o-**times a consensus sequence of N-X-S/T, wherein X represents any amino acid except proline,
the method comprises the steps:
A) providing a solution comprising a compound of formula **(II)**
B) mixing the solution with at least one nucleotide sugar and at least one transmembrane domain-deleted glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one transmembrane domain-deleted glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the aglycosylated peptide **P** in the presence of an oligosaccharyltransferase enzyme to produce the compound of formula **(I).**

In a further embodiment of the inventive method for producing a glycoprotein of general formula (I), wherein **C, NH** and **o** have the meanings as defined herein, **P** represents a peptide of at least 20 amino acids comprising at least **o-**times a consensus sequence of N-X-S/T, wherein X represents any amino acid except proline,
the method comprises the steps:
A) providing a solution comprising a compound of formula **(II)**
B) mixing the solution with at least one nucleotide sugar and at least one transmembrane domain-deleted glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one transmembrane domain-deleted glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a preferred embodiment of the inventive method for producing a glycoprotein of general formula **(I),** wherein **C,** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
A) providing a solution comprising a compound of formula **(II)**
B) mixing the solution with at least one nucleotide sugar and at least one transmembrane domain-deleted glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one transmembrane domain-deleted glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a preferred embodiment of the inventive method for producing a glycoprotein of general formula **(I),** wherein **C, NH** and **o** have the meanings as defined herein, **P** represents a peptide of at least 20 amino acids comprising at least **o-**times a consensus sequence of N-V-T or N-Y-T,
the method comprises the steps:
A) providing a solution comprising a compound of formula **(II)**
B) mixing the solution with at least one nucleotide sugar and at least one transmembrane domain-deleted glycosyltransferase enzyme and reacting a resulting mixture to produce a compound of formula **(III),** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one transmembrane domain-deleted glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

Thus, in a further embodiment of the inventive method for producing a glycoprotein of general formula **(I),** wherein **C** represents a carbohydrate of the following structure 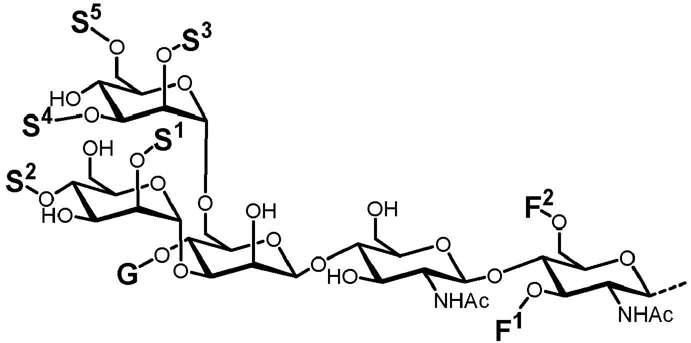
**o**, **P,** and **NH** have the meanings as defined herein;
**F¹** and **F²** represent 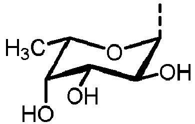 or -H; with the proviso that **F¹** and **F²** cannot be simultaneously 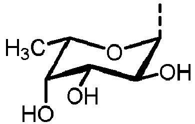
**G** represents 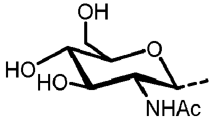 or -H;
**S¹** represents 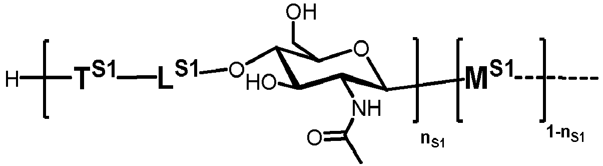
**S²** represents 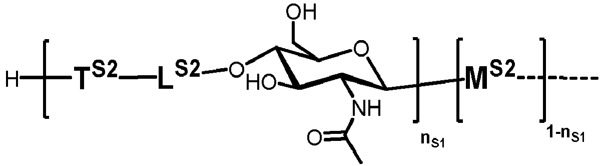
**S³** represents 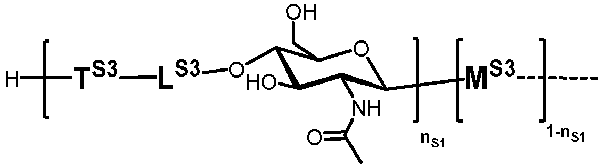
**S⁴** represents 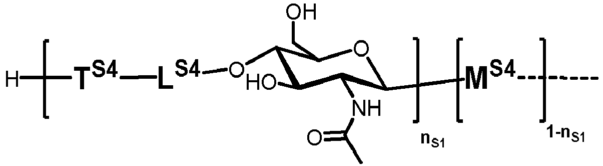
**S⁵** represents 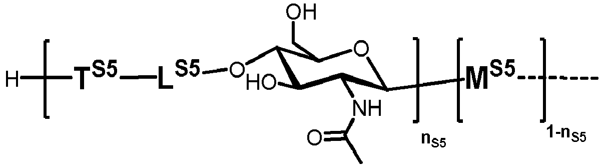
**M^{S1}** represents 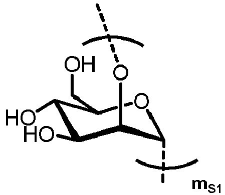
**M^{S2}** represents 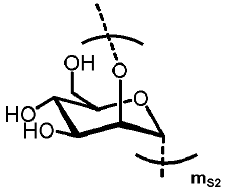
**M^{S3}** represents 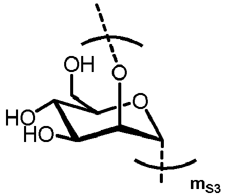
**M^{S4}** represents 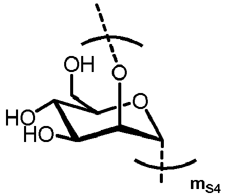
**M^{S5}** represents 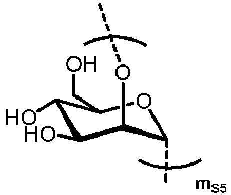
**L^{S1}** represents 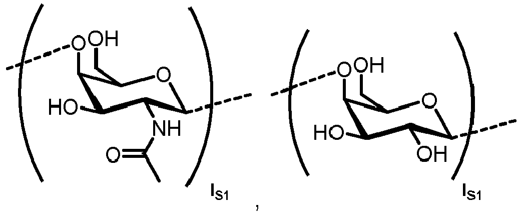 or 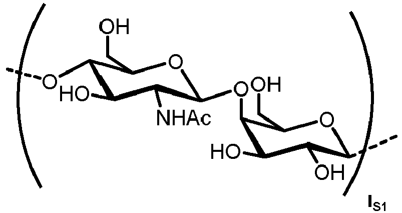
**L^{S2}** represents 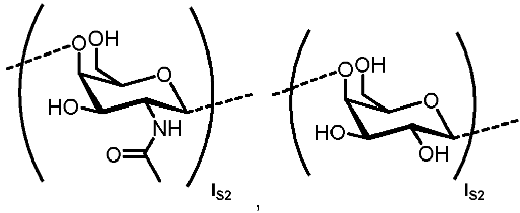 or 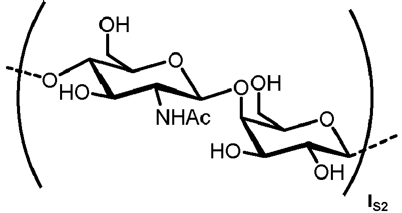
**L^{S3}** represents 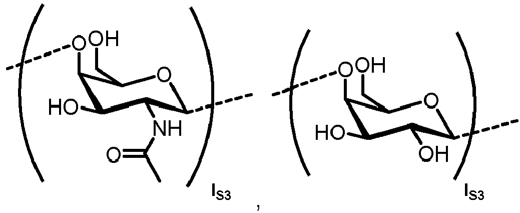 or 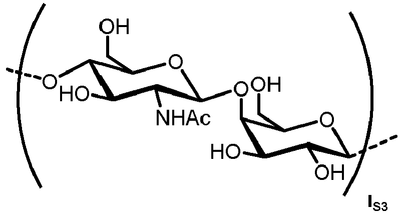
**L^{S4}** represents 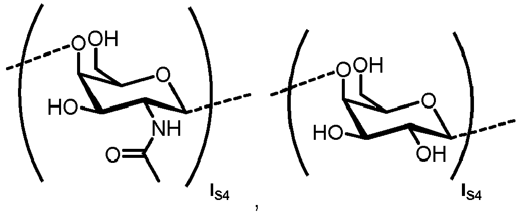 or 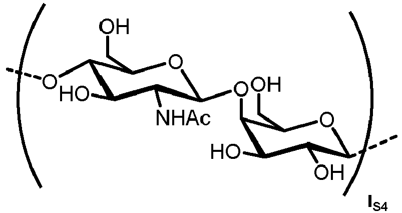
**L^{S5}** represents 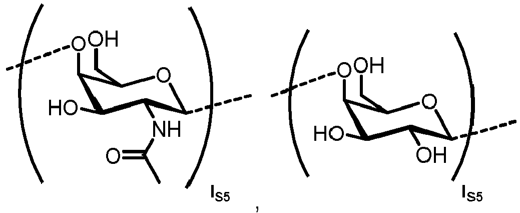 or 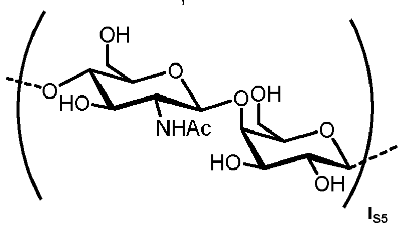
**T^{S1}**, **T^{S2}**, **T^{S3}**, **T^{S4}** and **T^{S5}** represent a bond
wherein m_{S1}, m_{S2}, m_{S3}, m_{S4}, m_{S5} I_{S1}, I_{S2}, I_{S3}, I_{S4}, and I_{S5} represent independently of each other an integer selected from 0, 1, 2, 3, 4, 5, 6, 7, 8, 9, 10, 11, 12, 13, 14 and 15,
n_{S1}, n_{S2}, n_{S3}, n_{S4} and n_{S5} represent an integer selected from 0 and 1 ,
with the proviso that if n_{S3} = 1 then
   n_{S1} = 1 and m_{S2} = m_{S4} = m_{S5} = 0; and
if n_{S3} = 0 then
   i) n_{S2} = m_{S2} = m_{S3} = 0 , and
   ii) m_{S1} + m_{S4} + m_{S5} ≥ 0 ;
the method comprises the steps:
   A) providing a solution comprising a compound of formula **(II)**
   B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
      wherein the at least one glycosyltransferase enzyme is selected from *N*-acetylglucosaminyltransferase, mannosyltransferase, glucosyltransferase, galactosyltransferase, fucosyltransferase and sialyltransferase; and
   C) reacting the compound of formula **(III)** with the peptide **P** in the presence of an oligosaccharyltransferase enzyme to produce the compound of formula **(I).**

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N-*acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase, and wherein **T^{S1}**, **T^{S2}**, **T^{S3}**, **T^{S4}** and **T^{S5}** represent a bond.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a eukaryotic oligosaccharyltransferase to produce the compound of formula (I),
and wherein **T^{S1}**, **T^{S2}**, **T^{S3}**, **T^{S4}** and **T^{S5}** represent a bond.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of STT3A protein from *Trypanosoma brucei* to produce the compound of formula **(I),**
and wherein **T^{S1}**, **T^{S2}**, **T^{S3}**, **T^{S4}** and **T^{S5}** represent a bond.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a bacterial oligosaccharyltransferase to produce the compound of formula **(I),**
and wherein **T^{S1}**, **T^{S2}**, **T^{S3}**, **T^{S4}** and **T^{S5}** represent a bond.

In a further embodiment of the inventive method for producing a glycoprotein of general formula **(I),** wherein **C, NH** and **o** have the meanings as defined herein, **P** represents a therapeutic protein of at least 10 amino acids comprising at least **o-**times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the therapeutic protein **P** in the presence of an oligosaccharyltransferase enzyme to produce the compound of formula **(I),**
and wherein **T^{S1}**, **T^{S2}**, **T^{S3}**, **T^{S4}** and **T^{S5}** represent a bond.

In a further embodiment of the inventive method for producing a glycoprotein of general formula **(I),** wherein **C, NH** and **o** have the meanings as defined herein, **P** represents an aglycosylated peptide of at least 10 amino acids comprising at least **o-times** a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the aglycosylated peptide **P** in the presence of an oligosaccharyltransferase enzyme to produce the compound of formula **(I),**
and wherein **T^{S1}**, **T^{S2}**, **T^{S3}**, **T^{S4}** and **T^{S5}** represent a bond.

In a further embodiment of the inventive method for producing a glycoprotein of general formula **(I),** wherein **C, NH** and **o** have the meanings as defined herein, **P** represents a peptide of at least 20 amino acids comprising at least **o-times** a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
and wherein **T^{S1}**, **T^{S2}**, **T^{S3}**, **T^{S4}** and **T^{S5}** represent a bond.

In a preferred embodiment of the inventive method for producing a glycoprotein of general formula **(I),** wherein **C,** and **NH** have the meanings as defined herein and **o** represents 1, P represents a peptide of at least 20 amino acids comprising at least one consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
and wherein **T^{S1}**, **T^{S2}**, **T^{S3}**, **T^{S4}** and **T^{S5}** represent a bond.

In a preferred embodiment of the inventive method for producing a glycoprotein of general formula **(I),** wherein **C, NH** and **o** have the meanings as defined herein, **P** represents a peptide of at least 20 amino acids comprising at least **o-**times a consensus sequence of N-V-T or N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III),** wherein the at least one nucleotide sugar is selected from GDP-mannose, UDP-galactose, UDP-GlcNAc, UDP-GaINAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
and wherein **T^{S1}**, **T^{S2}**, **T^{S3}**, **T^{S4}** and **T^{S5}** represent a bond.

In a further embodiment of the inventive method for producing a glycoprotein of general formula **(I),** wherein **C** represents a carbohydrate of the following structure 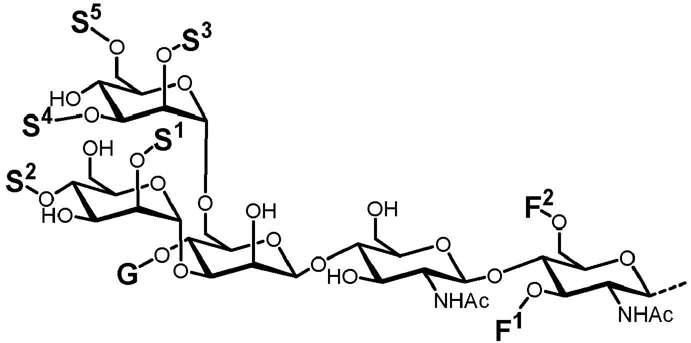
**o**, **P,** and **NH** have the meanings as defined herein;
**F¹** and **F²** represent 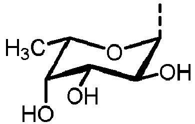 or -H; with the proviso that **F¹** and **F²** cannot be simultaneously 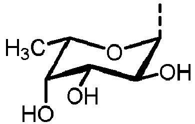
G represents 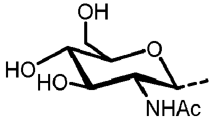 or -H;
**S¹** represents 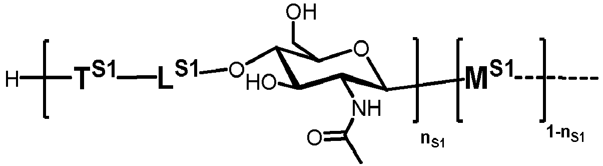
**S²** represents 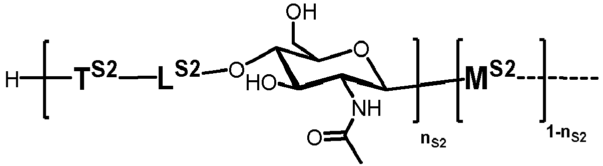
**S³** represents 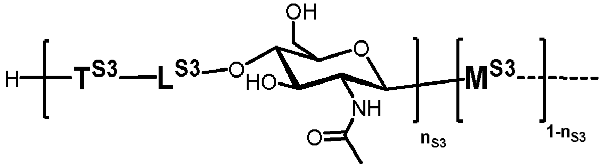
**S⁴** represents 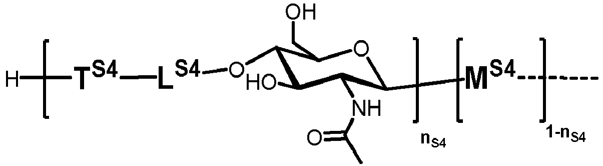
**S⁵** represents 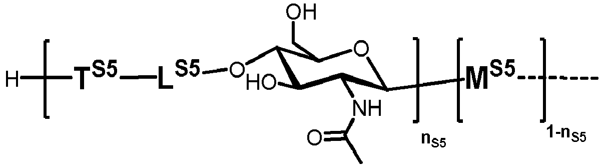
**M^{S1}** represents 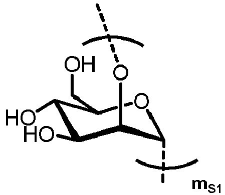
**M^{S2}** represents 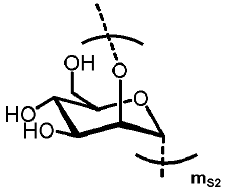
**M^{S3}** represents 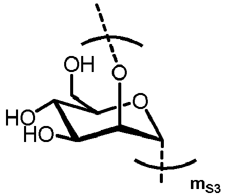
**M^{S4}** represents 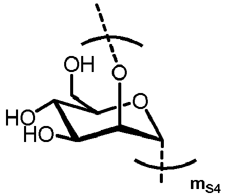
**M^{S5}** represents 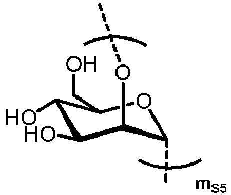
**L^{S1}** represents 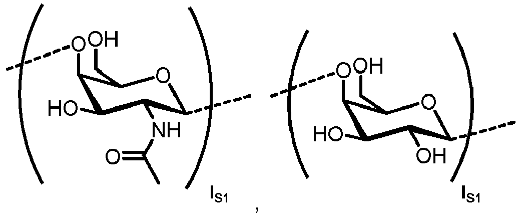 or 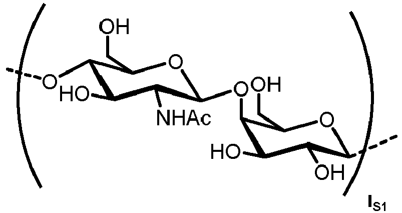
**L^{S2}** represents 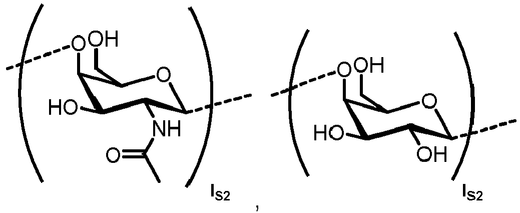 or 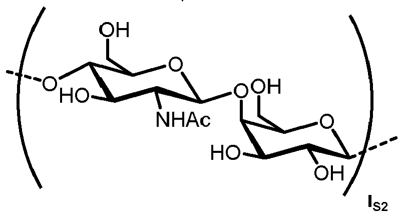
**L^{S3}** represents 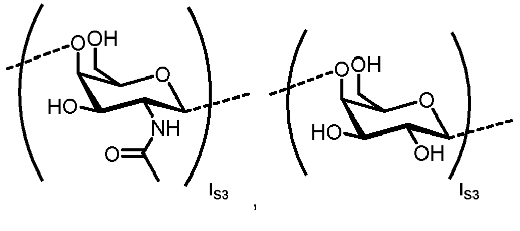 or 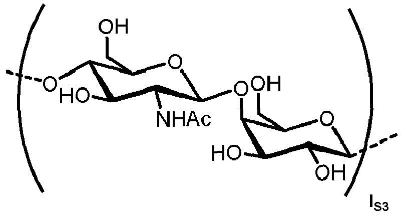
**L^{S4}** represents 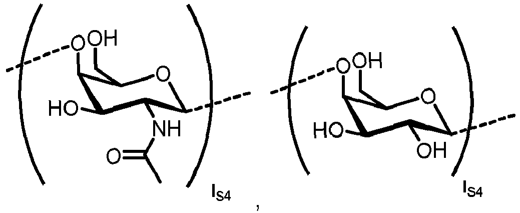 or 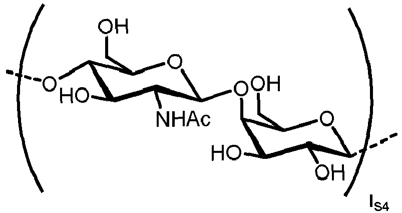
**L^{S5}** represents 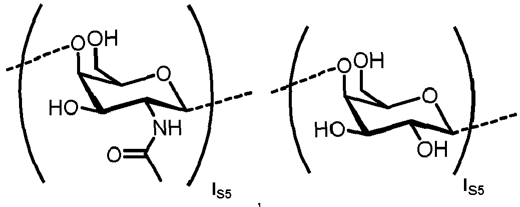 or 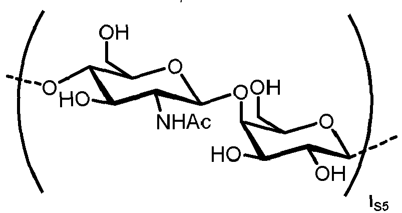
**T^{S1}**, T^{S2}, T^{S3}, **T^{S4}** and **T^{S5}** represent independently of each other: 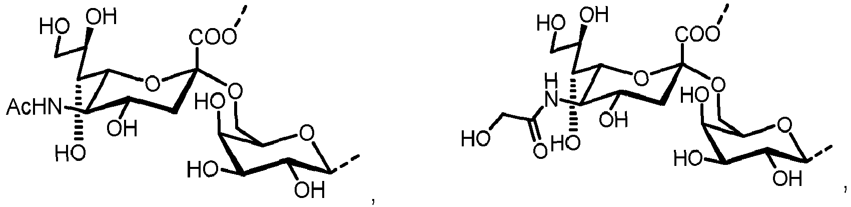 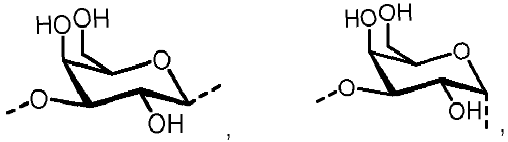 -SO₄- or a bond wherein m_{S1}, m_{S2}, m_{S3}, m_{S4}, and m_{S5} represent independently of each other an integer selected from 0, 1, 2, 3, 4, 5, 6, 7, 8, 9, 10, 11, 12, 13, 14 and 15,
wherein I_{S1}, I_{S2}, I_{S3}, I_{S4}, and I_{S5} represent independently of each other an integer selected from 0, 1, 2, and 3,
n_{S1}, n_{S2}, n_{S3}, n_{S4} and n_{S5} represent an integer selected from 0 and 1 ,
with the proviso that if n_{S3} = 1 then
   n_{S1} = 1 and m_{S2} = m_{S4} = m_{S5} = 0; and
if n_{S3} = 0 then
   i) n_{S2} = m_{S2} = m_{S3} = 0, and
   ii) m_{S1} + m_{S4} + m_{S5} ≥ 0 ;
the method comprises the steps:
   A) providing a solution comprising a compound of formula **(II)**
   B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
      wherein the at least one glycosyltransferase enzyme is selected from *N*-acetylglucosaminyltransferase, mannosyltransferase, glucosyltransferase, galactosyltransferase, fucosyltransferase and sialyltransferase; and
   C) reacting the compound of formula **(III)** with the peptide **P** in the presence of an oligosaccharyltransferase enzyme to produce the compound of formula **(I).**

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein **T^{S1}**, **T^{S2}**, **T^{S3}**, **T^{S4}** and **T^{S5}** represent a bond and wherein I_{S1}, I_{S2}, I_{S3}, I_{S4}, and I_{S5} represent independently of each other an integer selected from 0, 1, 2 and 3.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase, and wherein I_{S1}, I_{S2}, I_{S3}, I_{S4}, and I_{S5} represent independently of each other an integer selected from 0, 1, 2 and 3.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a eukaryotic oligosaccharyltransferase to produce the compound of formula **(I),**
and wherein I_{S1}, I_{S2}, I_{S3}, I_{S4}, and I_{S5} represent independently of each other an integer selected from 0, 1, 2 and 3.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of STT3A protein from *Trypanosoma brucei* to produce the compound of formula **(I),**
and wherein I_{S1}, I_{S2}, I_{S3}, I_{S4}, and I_{S5} represent independently of each other an integer selected from 0, 1, 2 and 3.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a bacterial oligosaccharyltransferase to produce the compound of formula **(I),**
and wherein I_{S1}, I_{S2}, I_{S3}, I_{S4}, and I_{S5} represent independently of each other an integer selected from 0, 1, 2 and 3.

In a further embodiment of the inventive method for producing a glycoprotein of general formula **(I),** wherein **C, NH** and **o** have the meanings as defined herein, **P** represents a therapeutic protein of at least 10 amino acids comprising at least **o**-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the therapeutic protein **P** in the presence of an oligosaccharyltransferase enzyme to produce the compound of formula **(I),**
and wherein I_{S1}, I_{S2}, I_{S3}, I_{S4}, and I_{S5} represent independently of each other an integer selected from 0, 1, 2 and 3.

In a further embodiment of the inventive method for producing a glycoprotein of general formula **(I),** wherein **C, NH** and **o** have the meanings as defined herein, **P** represents an aglycosylated peptide of at least 10 amino acids comprising at least **o**-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the aglycosylated peptide **P** in the presence of an oligosaccharyltransferase enzyme to produce the compound of formula **(I),**
and wherein I_{S1}, I_{S2}, I_{S3}, I_{S4}, and I_{S5} represent independently of each other an integer selected from 0, 1, 2 and 3.

In a further embodiment of the inventive method for producing a glycoprotein of general formula **(I),** wherein **C, NH** and **o** have the meanings as defined herein, **P** represents a peptide of at least 20 amino acids comprising at least **o**-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
and wherein I_{S1}, I_{S2}, I_{S3}, I_{S4}, and I_{S5} represent independently of each other an integer selected from 0, 1, 2 and 3.

In a preferred embodiment of the inventive method for producing a glycoprotein of general formula **(I),** wherein **C,** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
and wherein I_{S1}, I_{S2}, I_{S3}, I_{S4}, and I_{S5} represent independently of each other an integer selected from 0, 1, 2 and 3.

In a preferred embodiment of the inventive method for producing a glycoprotein of general formula **(I),** wherein **C, NH** and **o** have the meanings as defined herein, **P** represents a peptide of at least 20 amino acids comprising at least **o**-times a consensus sequence of N-V-T or N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III),** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
and wherein I_{S1}, I_{S2}, I_{S3}, I_{S4}, and I_{S5} represent independently of each other an integer selected from 0, 1, 2 and 3.

In a further embodiment of the inventive method for producing a glycoprotein of general formula **(I),** wherein **C** represents a carbohydrate of the following structure 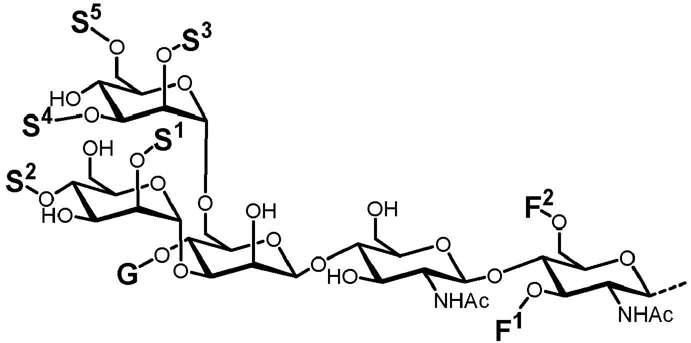 **o**, **P,** and **NH** have the meanings as defined herein;
**F¹** and **F²** represent 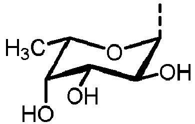 or -H; with the proviso that **F¹** and **F²** cannot be simultaneously 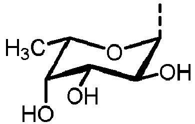
**G** represents 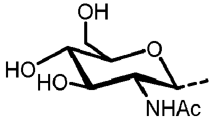 or -H;
**S¹** represents 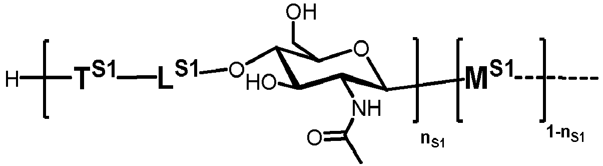
**S²** represents 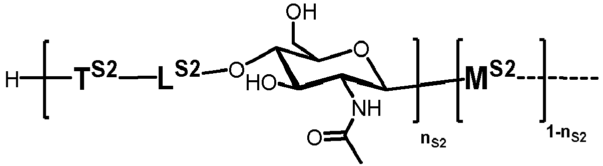
**S³** represents 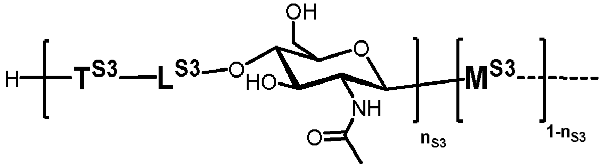
**S⁴** represents 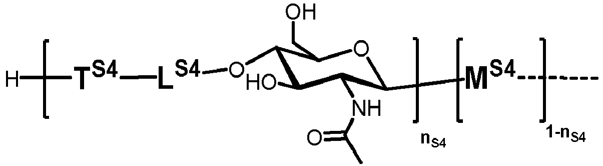
**S⁵** represents 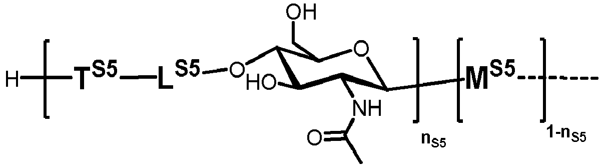
**M^{S1}** represents 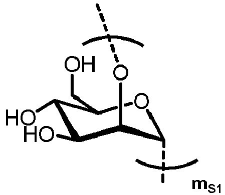
**M^{S2}** represents 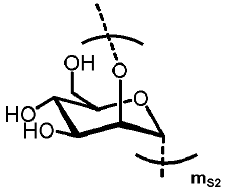
**M^{S3}** represents 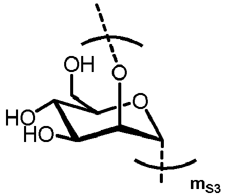
**M^{S4}** represents 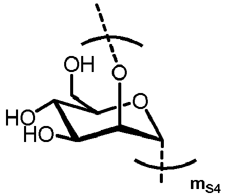
**M^{S5}** represents 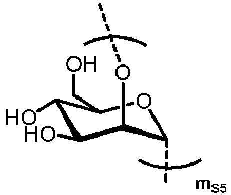
**L^{S1}** represents 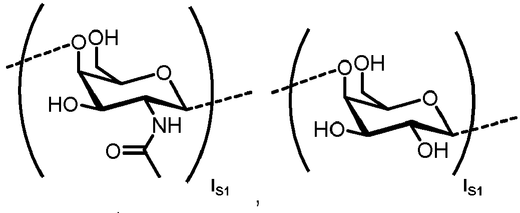 or 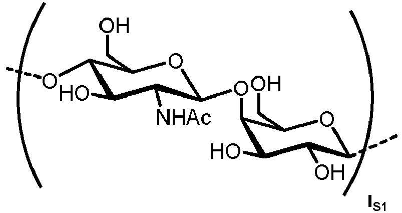
**L^{S2}** represents 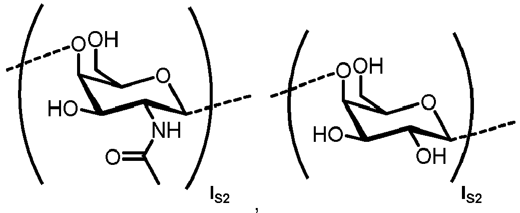 or 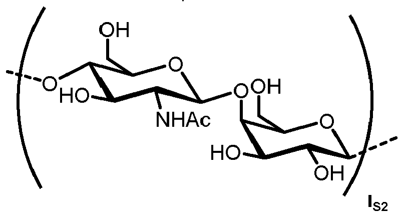
**L^{S3}** represents 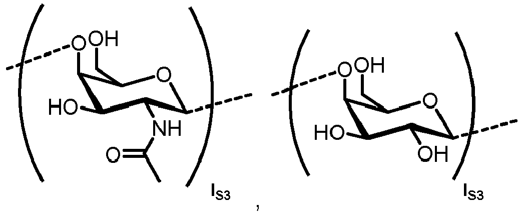 or 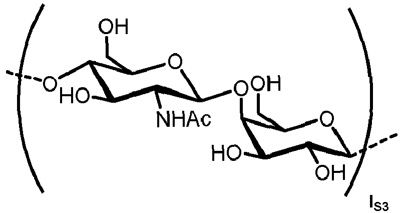
**L^{S4}** represents 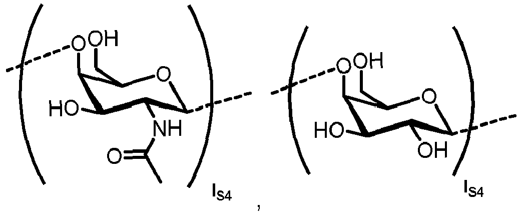 or 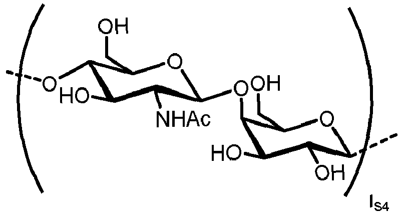
**L^{S5}** represents 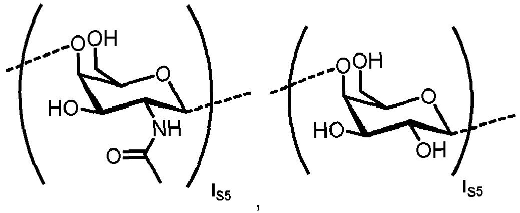 or 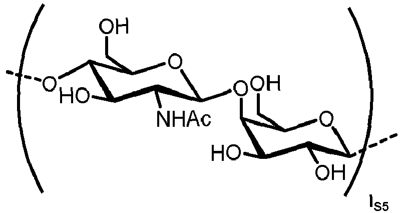
**T^{S1}**, **T^{S2}**, **T^{S3}**, **T^{S4}** and **T^{S5}** represent independently of each other: 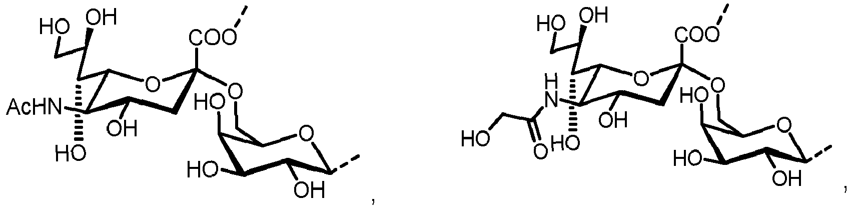 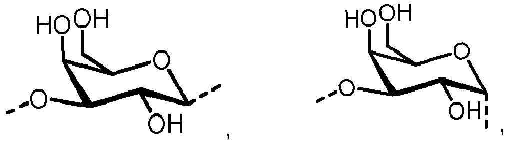 -SO₄- or a bond wherein m_{S1}, m_{S2}, m_{S3}, m_{S4}, and m_{S5} represent independently of each other an integer selected from 0, 1, 2, 3, 4 and 5
wherein I_{S1}, I_{S2}, I_{S3}, I_{S4}, and I_{S5} represent independently of each other an integer selected from 0, 1, 2, 3, 4, 5, 6, 7, 8, 9, 10, 11, 12, 13, 14 and 15,
n_{S1}, n_{S2}, n_{S3}, n_{S4} and n_{S5} represent an integer selected from 0 and 1 ,
with the proviso that if n_{S3} = 1 then
   n_{S1} = 1 and m_{S2} = m_{S4} = m_{S5} = 0; and
if n_{S3} = 0 then
   i) n_{S2} = m_{S2} = m_{S3} = 0, and
   ii) m_{S1} + m_{S4} + m_{S5} ≥ 0 ;
the method comprises the steps:
   A) providing a solution comprising a compound of formula **(II)**
   B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
      wherein the at least one glycosyltransferase enzyme is selected from *N*-acetylglucosaminyltransferase, mannosyltransferase, glucosyltransferase, galactosyltransferase, fucosyltransferase and sialyltransferase; and
   C) reacting the compound of formula **(III)** with the peptide **P** in the presence of an oligosaccharyltransferase enzyme to produce the compound of formula **(I).**

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein I_{S1}, I_{S2}, I_{S3}, I_{S4}, and I_{S5} represent independently of each other an integer selected from 0, 1, 2 and 3 and wherein m_{S1}, m_{S2}, m_{S3}, m_{S4}, and m_{S5} represent independently of each other an integer selected from 0, 1, 2, 3, 4 and 5.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein **T^{S1}**, **T^{S2}**, **T^{S3}**, **T^{S4}** and **T^{S5}** represent a bond and wherein m_{S1}, m_{S2}, m_{S3}, m_{S4}, and m_{S5} represent independently of each other an integer selected from 0, 1, 2, 3, 4 and 5.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase, and wherein m_{S1}, m_{S2}, m_{S3}, m_{S4}, and m_{S5} represent independently of each other an integer selected from 0, 1, 2, 3, 4 and 5.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a eukaryotic oligosaccharyltransferase to produce the compound of formula **(I),**
and wherein m_{S1}, m_{S2}, m_{S3}, m_{S4}, and m_{S5} represent independently of each other an integer selected from 0, 1, 2, 3, 4 and 5.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of STT3A protein from *Trypanosoma brucei* to produce the compound of formula **(I),**
and wherein m_{S1}, m_{S2}, m_{S3}, m_{S4}, and m_{S5} represent independently of each other an integer selected from 0, 1, 2, 3, 4 and 5.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a bacterial oligosaccharyltransferase to produce the compound of formula **(I),**
and wherein m_{S1}, m_{S2}, m_{S3}, m_{S4}, and m_{S5} represent independently of each other an integer selected from 0, 1, 2, 3, 4 and 5.

In a further embodiment of the inventive method for producing a glycoprotein of general formula **(I),** wherein **C, NH** and **o** have the meanings as defined herein, **P** represents a therapeutic protein of at least 10 amino acids comprising at least **o**-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the therapeutic protein **P** in the presence of an oligosaccharyltransferase enzyme to produce the compound of formula **(I),**
and wherein m_{S1}, m_{S2}, m_{S3}, m_{S4}, and m_{S5} represent independently of each other an integer selected from 0, 1, 2, 3, 4 and 5.

In a further embodiment of the inventive method for producing a glycoprotein of general formula **(I),** wherein **C, NH** and **o** have the meanings as defined herein, **P** represents an aglycosylated peptide of at least 10 amino acids comprising at least **o**-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the aglycosylated peptide **P** in the presence of an oligosaccharyltransferase enzyme to produce the compound of formula **(I),**
and wherein m_{S1}, m_{S2}, m_{S3}, m_{S4}, and m_{S5} represent independently of each other an integer selected from 0, 1, 2, 3, 4 and 5.

In a further embodiment of the inventive method for producing a glycoprotein of general formula **(I),** wherein **C, NH** and **o** have the meanings as defined herein, **P** represents a peptide of at least 20 amino acids comprising at least **o**-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
and wherein m_{S1}, m_{S2}, m_{S3}, m_{S4}, and m_{S5} represent independently of each other an integer selected from 0, 1, 2, 3, 4 and 5.

In a preferred embodiment of the inventive method for producing a glycoprotein of general formula **(I),** wherein **C,** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
and wherein m_{S1}, m_{S2}, m_{S3}, m_{S4}, and m_{S5} represent independently of each other an integer selected from 0, 1, 2, 3, 4 and 5.

In a preferred embodiment of the inventive method for producing a glycoprotein of general formula **(I),** wherein **C, NH** and **o** have the meanings as defined herein, **P** represents a peptide of at least 20 amino acids comprising at least **o**-times a consensus sequence of N-V-T or N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III),** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
and wherein m_{S1}, m_{S2}, m_{S3}, m_{S4}, and m_{S5} represent independently of each other an integer selected from 0, 1, 2, 3, 4 and 5.

In a further embodiment of the inventive method for producing a glycoprotein of general formula **(I),** wherein **C** represents a carbohydrate of the following structure 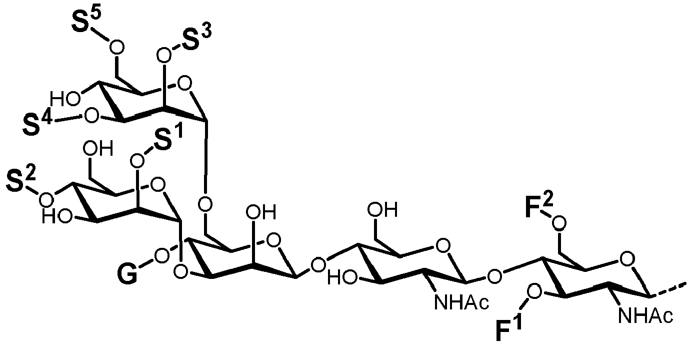
**o**, **P,** and **NH** have the meanings as defined herein;
**F¹**, **F²** and **G** represent -H,
**S¹** represents 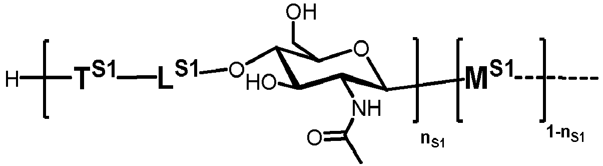
**S²** represents 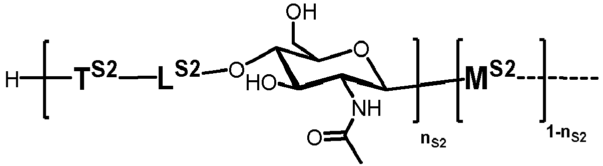
**S³** represents 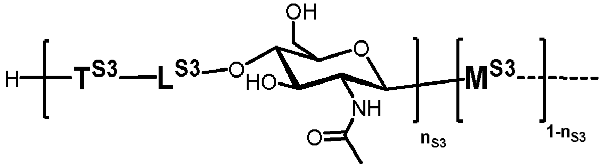
**S⁴** represents 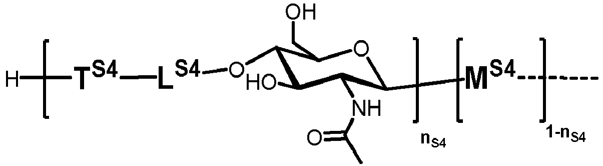
**S⁵** represents 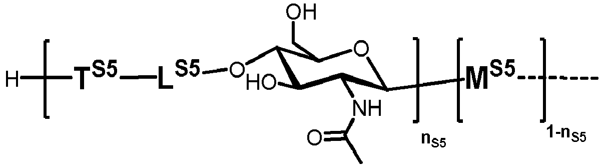
**M^{S1}** represents 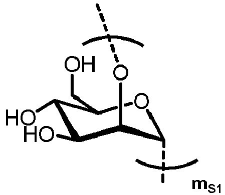
**M^{S2}** represents 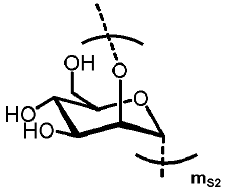
**M^{S3}** represents 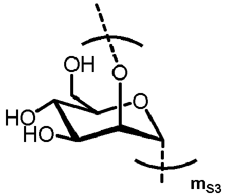
**M^{S4}** represents 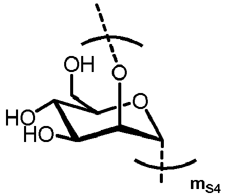
**M^{S5}** represents 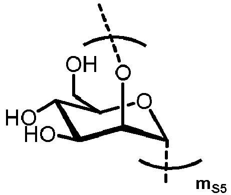
**L^{S1}** represents 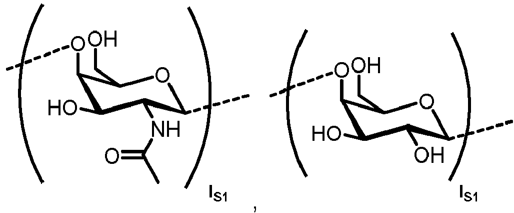 or 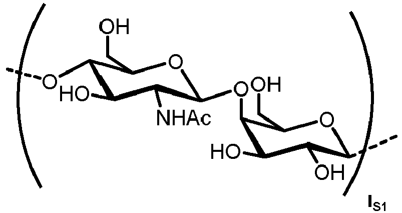
**L^{S2}** represents 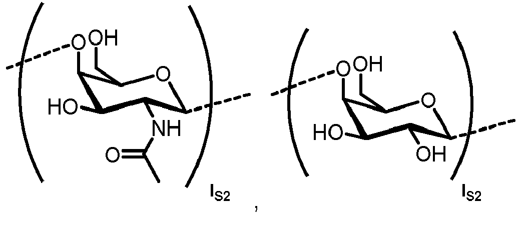 or 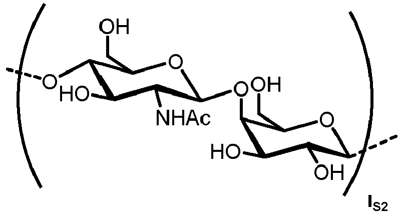
**L^{S3}** represents 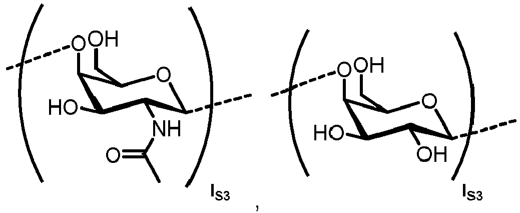 or 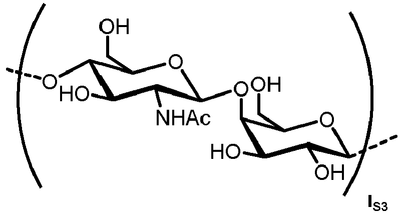
**L^{S4}** represents 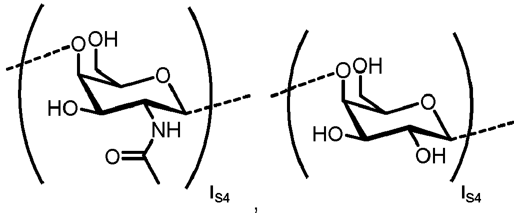 or 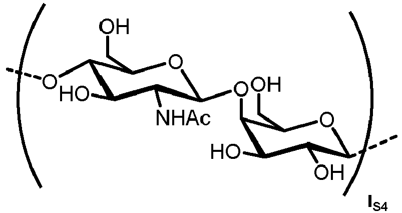
**L^{S5}** represents 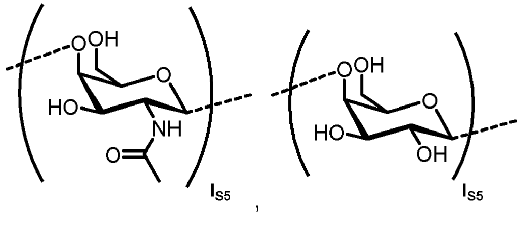 or 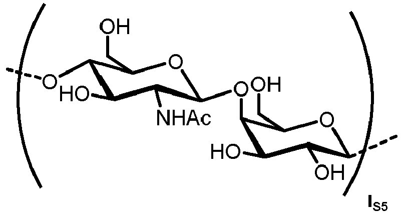
**T^{S1}**, **T^{S2}**, **T^{S3}**, **T^{S4}** and **T^{S5}** represent independently of each other: 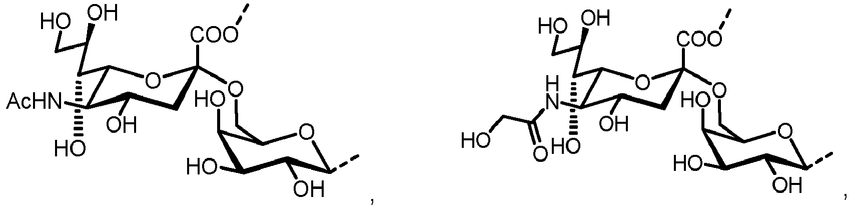 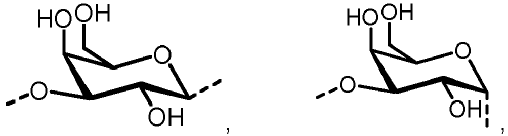 -SO₄- or a bond wherein m_{S1}, m_{S2}, m_{S3}, m_{S4}, m_{S5}, I_{S1}, I_{S2}, I_{S3}, I_{S4}, and I_{S5} represent independently of each other an integer selected from 0, 1, 2, 3, 4, 5, 6, 7, 8, 9, 10, 11, 12, 13, 14 and 15,
n_{S1}, n_{S2}, n_{S3}, n_{S4} and n_{S5} represent an integer selected from 0 and 1 ,
with the proviso that if n_{S3} = 1 then
   n_{S1} = 1 and m_{S2} = m_{S4} = m_{S5} = 0; and
if n_{S3} = 0 then
   i) n_{S2} = m_{S2} = m_{S3} = 0, and
   ii) m_{S1} + m_{S4} + m_{S5} ≥ 0 ;
the method comprises the steps:
   A) providing a solution comprising a compound of formula **(II)**
   B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
      wherein the at least one glycosyltransferase enzyme is selected from *N*-acetylglucosaminyltransferase, mannosyltransferase, glucosyltransferase, galactosyltransferase, fucosyltransferase and sialyltransferase; and
   C) reacting the compound of formula **(III)** with the peptide **P** in the presence of an oligosaccharyltransferase enzyme to produce the compound of formula **(I).**

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein m_{S1}, m_{S2}, m_{S3}, m_{S4}, and m_{S5} represent independently of each other an integer selected from 0, 1, 2, 3, 4 and 5, and **F¹**, **F²** and **G** represent -H.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein I_{S1}, I_{S2}, I_{S3}, I_{S4}, and I_{S5} represent independently of each other an integer selected from 0, 1, 2 and 3 and **F¹**, **F²** and **G** represent -H.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein **T^{S1}**, **T^{S2}**, **T^{S3}**, **T^{S4}** and **T^{S5}** represent a bond and wherein **F¹**, **F²** and **G** represent -H.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase, and wherein **F¹**, **F²** and **G** represent -H.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a eukaryotic oligosaccharyltransferase to produce the compound of formula **(I),**
and wherein **F¹**, **F²** and **G** represent -H.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of STT3A protein from *Trypanosoma brucei* to produce the compound of formula **(I),**
and wherein **F¹**, **F²** and **G** represent -H.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a bacterial oligosaccharyltransferase to produce the compound of formula **(I),**
and wherein **F¹**, **F²** and **G** represent -H.

In a further embodiment of the inventive method for producing a glycoprotein of general formula **(I),** wherein **C, NH** and **o** have the meanings as defined herein, **P** represents a therapeutic protein of at least 10 amino acids comprising at least **o**-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the therapeutic protein **P** in the presence of an oligosaccharyltransferase enzyme to produce the compound of formula **(I),**
and **F¹**, **F²** and **G** represent -H.

In a further embodiment of the inventive method for producing a glycoprotein of general formula **(I),** wherein **C, NH** and **o** have the meanings as defined herein, **P** represents an aglycosylated peptide of at least 10 amino acids comprising at least **o**-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the aglycosylated peptide **P** in the presence of an oligosaccharyltransferase enzyme to produce the compound of formula **(I),**
and **F¹**, **F²** and **G** represent -H.

In a further embodiment of the inventive method for producing a glycoprotein of general formula **(I),** wherein **C, NH** and **o** have the meanings as defined herein, **P** represents a peptide of at least 20 amino acids comprising at least **o**-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
and **F¹**, **F²** and **G** represent -H.

In a preferred embodiment of the inventive method for producing a glycoprotein of general formula **(I),** wherein **C,** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyl+ transferase, fucosyltransferase and sialyltransferase; and
C) reacting the compound of formula **(III)** with the peptide **P** in the presence of an oligosaccharyltransferase enzyme to produce the compound of formula **(I),**
and **F¹**, **F²** and **G** represent -H.

In a preferred embodiment of the inventive method for producing a glycoprotein of general formula **(I),** wherein **C, NH** and **o** have the meanings as defined herein, **P** represents a peptide of at least 20 amino acids comprising at least **o**-times a consensus sequence of N-V-T or N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III),** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
and **F¹**, **F²** and **G** represent -H.

The construction of the carbohydrate in step B) may be performed in one-pot or sequentially in multiple steps. Thus, in a further embodiment of the inventive method for producing a glycoprotein of general formula **(I),** wherein **C** represents a carbohydrate of the following structure 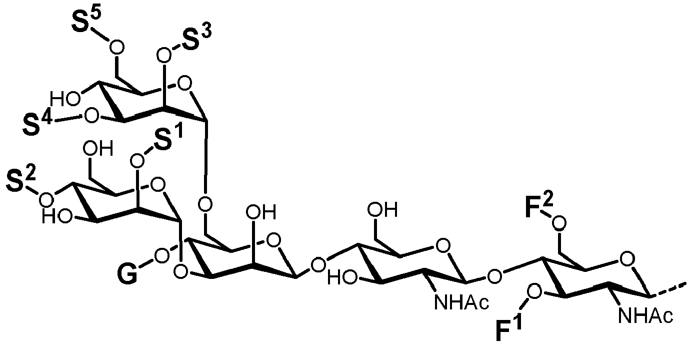
**o, P,** and **NH** have the meanings as defined herein;
**F¹**, **F²** and **G** represent -H,
**S¹** represents 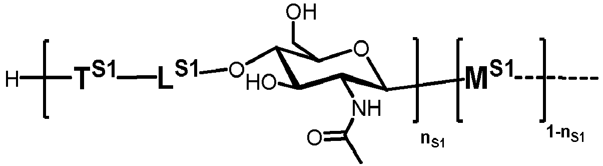
**S²** represents 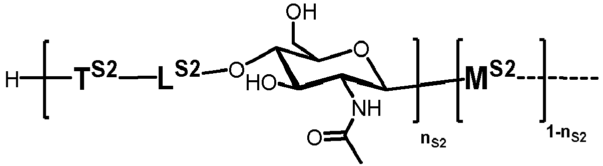
**S³** represents 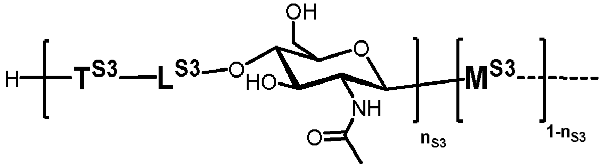
**S⁴** represents 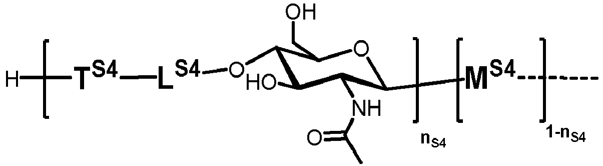
**S⁵** represents 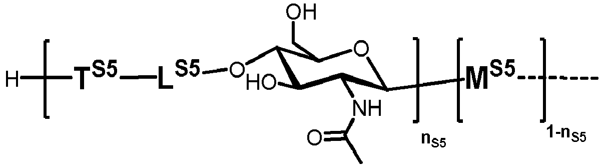
**M^{S1}** represents 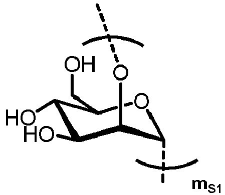
**M^{S2}** represents 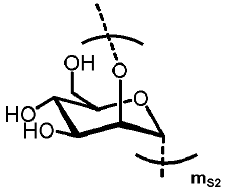
**M^{S3}** represents 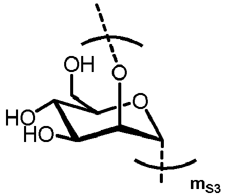
**M^{S4}** represents 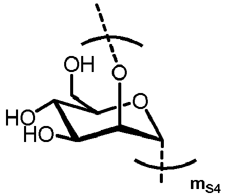
**M^{S5}** represents 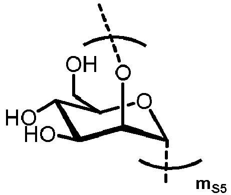
**L^{S1}** represents 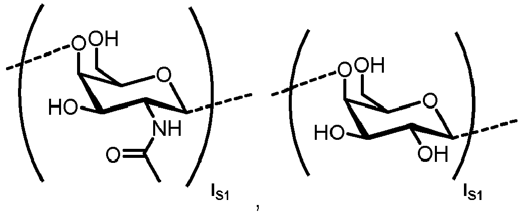 or 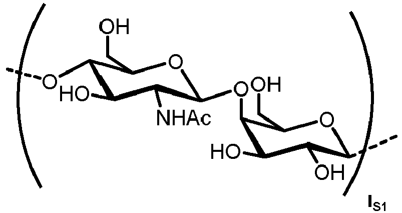
**L^{S2}** represents 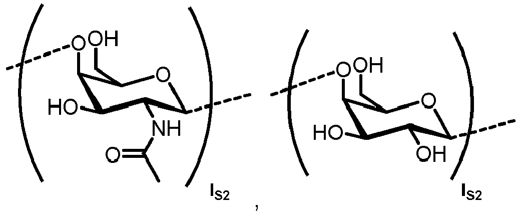 or 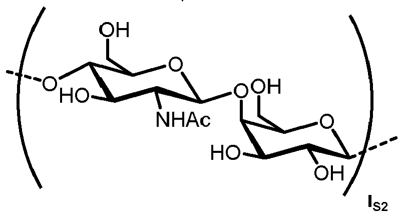
**L^{S3}** represents 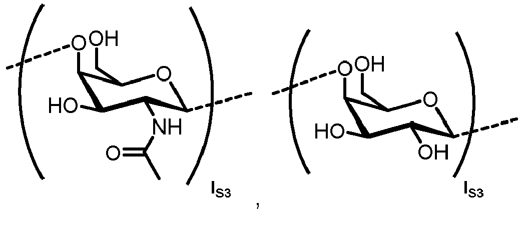 or 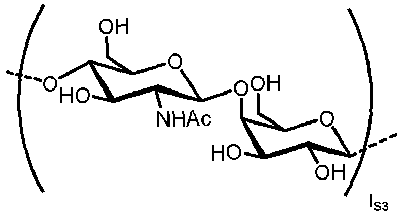
**L^{S4}** represents 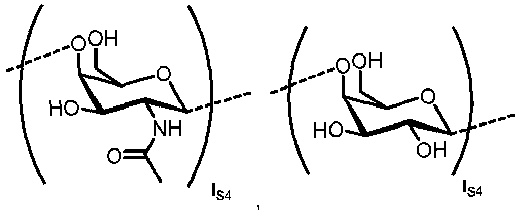 or 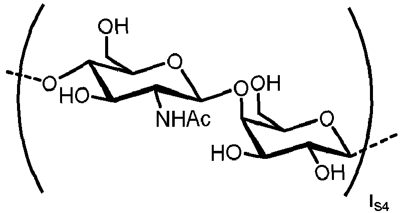
**L^{S5}** represents 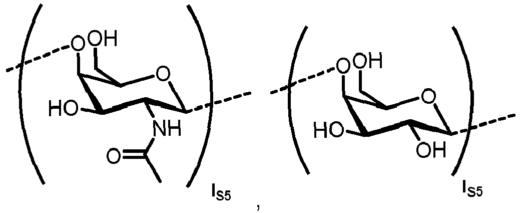 or 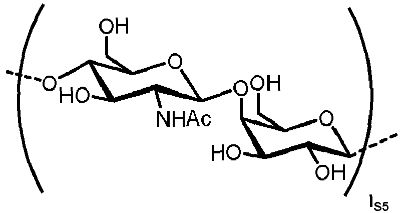
**T^{S1}**, **T^{S2}**, **T^{S3}, T^{S4}** and **T^{S5}** represent independently of each other: 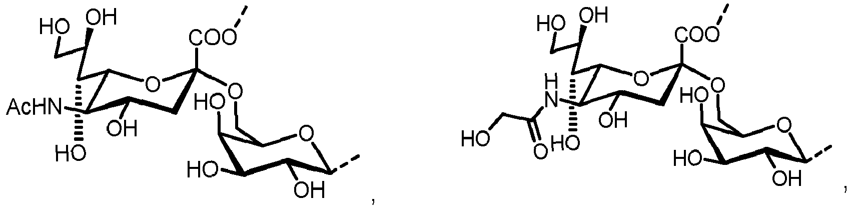 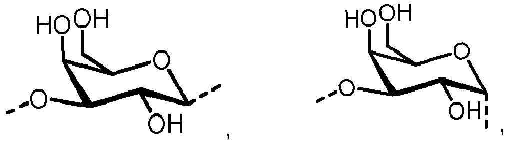 -SO₄- or a bond wherein m_{S1}, m_{S2}, m_{S3}, m_{S4}, m_{S5}, I_{S1}, I_{S2}, I_{S3}, I_{S4}, and I_{S5} represent independently of each other an integer selected from 0, 1, 2, 3, 4, 5, 6, 7, 8, 9, 10, 11, 12, 13, 14 and 15,
n_{S1}, n_{S2}, n_{S3}, n_{S4}, and n_{S5} represent an integer selected from 0 and 1 ,
with the proviso that if n_{S3} = 1 then
   n_{S1} = 1 and m_{S2} = m_{S4} = m_{S5} = 0; and
if n_{S3} = 0 then
   i) n_{S2} = m_{S2} = m_{S3} = 0, and
   ii) m_{S1} + m_{S4} + m_{S5} ≥ 0 ;
the method comprises the steps:
   A) providing a solution comprising a compound of formula **(II)**
   B1) mixing the solution with a nucleotide sugar and a glycosyltransferase enzyme and reacting a resulting mixture
   B2) repeating step B1 until a compound of formula **(III)** is produced, wherein the nucleotide sugar is selected from GDP-mannose, UDP-galactose, UDP-GlcNAc, UDP-GalNAc, GDP-fucose, CMP-NeuAc and CMP-NeuGc,
      wherein the glycosyltransferase enzyme is selected from *N*-acetylglucosaminyltransferase, mannosyltransferase, glucosyltransferase, galactosyltransferase, fucosyltransferase and sialyltransferase; and
   C) reacting the compound of formula **(III)** with the peptide **P** in the presence of an oligosaccharyltransferase enzyme to produce the compound of formula **(I).**

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein **F¹**, **F²** and **G** represent -H.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein m_{S1}, m_{S2}, m_{S3}, m_{S4}, and m_{S5} represent independently of each other an integer selected from 0, 1, 2, 3, 4 and 5.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein I_{S1}, I_{S2}, I_{S3}, I_{S4}, and I_{S5} represent independently of each other an integer selected from 0, 1, 2 and 3.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein **T^{S1}**, **T^{S2}**, **T^{S3}**, **T^{S4}**, and **T^{S5}** represent a bond.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a eukaryotic oligosaccharyltransferase to produce the compound of formula **(I).**

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of STT3A protein from *Trypanosoma brucei* to produce the compound of formula **(I).**

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a bacterial oligosaccharyltransferase to produce the compound of formula **(I).**

In a further embodiment of the inventive method for producing a glycoprotein of general formula **(I),** wherein **C, NH** and **o** have the meanings as defined herein, **P** represents a therapeutic protein of at least 10 amino acids comprising at least **o**-times a consensus sequence of N-X-S/T, wherein X represents any amino acid except proline,
the method comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the therapeutic protein **P** in the presence of an oligosaccharyltransferase enzyme to produce the compound of formula **(I).**

In a further embodiment of the inventive method for producing a glycoprotein of general formula **(I),** wherein **C, NH** and **o** have the meanings as defined herein, **P** represents an aglycosylated peptide of at least 10 amino acids comprising at least **o**-times a consensus sequence of N-X-S/T, wherein X represents any amino acid except proline,
the method comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the aglycosylated peptide **P** in the presence of an oligosaccharyltransferase enzyme to produce the compound of formula **(I).**

In a further embodiment of the inventive method for producing a glycoprotein of general formula **(I),** wherein **C, NH** and **o** have the meanings as defined herein, **P** represents a peptide of at least 20 amino acids comprising at least **o**-times a consensus sequence of N-X-S/T, wherein X represents any amino acid except proline,
the method comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a preferred embodiment of the inventive method for producing a glycoprotein of general formula **(I),** wherein **C,** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a preferred embodiment of the inventive method for producing a glycoprotein of general formula **(I),** wherein **C, NH** and **o** have the meanings as defined herein, **P** represents a peptide of at least 20 amino acids comprising at least **o**-times a consensus sequence of N-V-T or N-Y-T,
the method comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I).**

In a further embodiment, n_{S1}, n_{S2}, n_{S3}, n_{S4}, n_{S5}, m_{S1}, m_{S2}, m_{S3}, m_{S4}, and m_{S5} are defined as follows:
n_{S1}, n_{S2}, n_{S3}, n_{S4} and n_{S5} represent an integer selected from 0 and 1 ,
with the proviso that if n_{S3} = 1 then
   n_{S1} = 1 and m_{S2} = m_{S4} = m_{S5} = 0; and
if n_{S3} = 0 then
   i) n_{S2} = m_{S2} = m_{S3} = 0, and
   ii) m_{S1} + m_{S4} + m_{S5} > 0 ;

Thus, the method for producing a glycoprotein of general formula **(I),** wherein **C, NH, P** and **o** have the meanings as defined herein, comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
n_{S1}, n_{S2}, n_{S3}, n_{S4} and n_{S5} represent an integer selected from 0 and 1 ,
with the proviso that if n_{S3} = 1 then
   n_{S1} = 1 and m_{S2} = m_{S4} = m_{S5} = 0; and
if n_{S3} = 0 then
   i) n_{S2} = m_{S2} = m_{S3} = 0, and
   ii) m_{S1} + m_{S4} + m_{S5} > 0 ;

In a further embodiment of the inventive method for producing a glycoprotein of general formula **(I),** wherein **C** represents a carbohydrate of the following structure 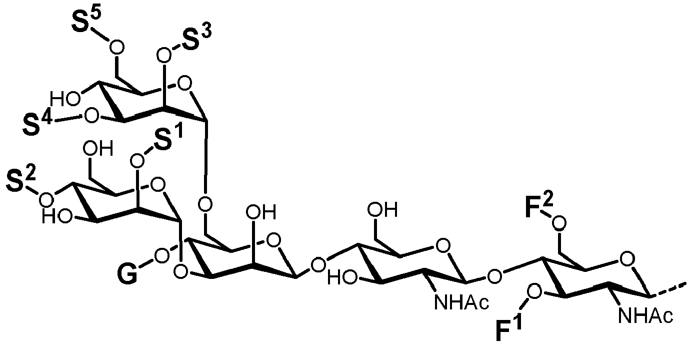
**o, P,** and **NH** have the meanings as defined herein;
**F¹** and **F²** represent 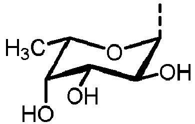 or -H; with the proviso that **F¹** and **F²** cannot be simultaneously 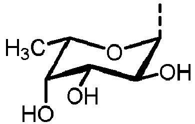
**G** represents 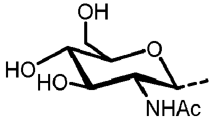 or -H;
**S¹** represents 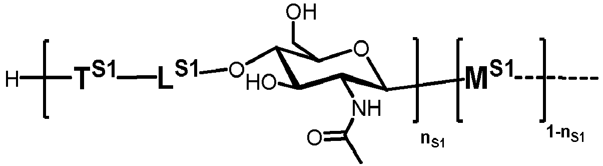
**S²** represents 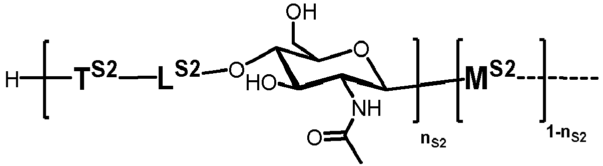
**S³** represents 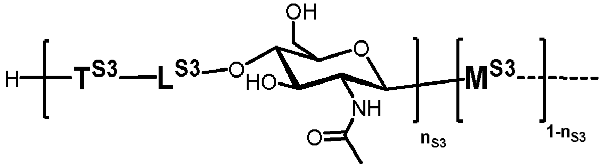
**S⁴** represents 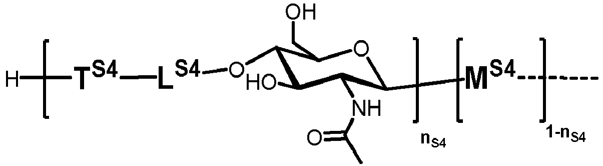
**S⁵** represents 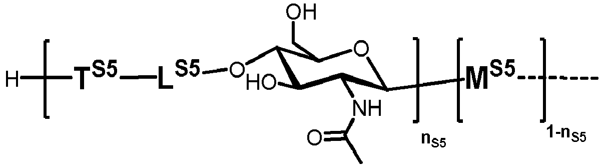
**M^{S1}** represents 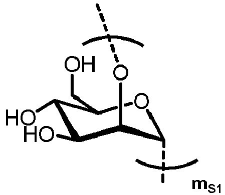
**M^{S2}** represents 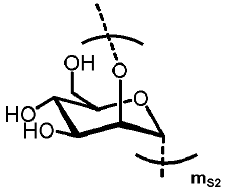
**M^{S3}** represents 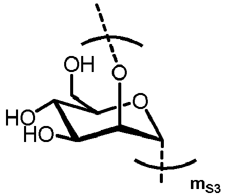
**M^{S4}** represents 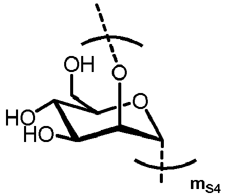
**M^{S5}** represents 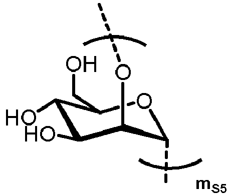
**L^{S1}** represents 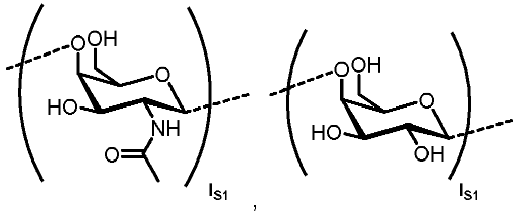 or 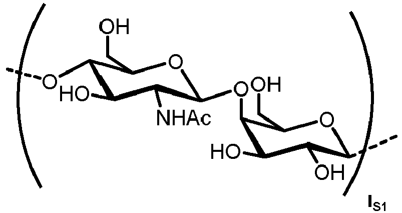
**L^{S2}** represents 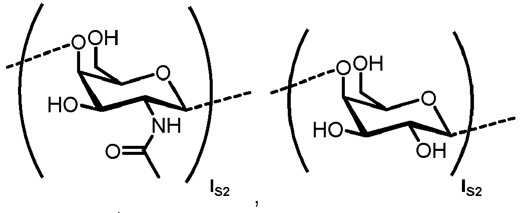 or 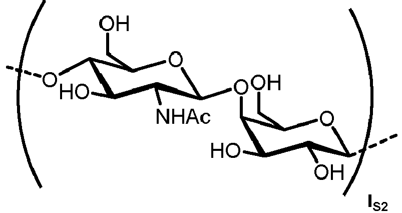
**L^{S3}** represents 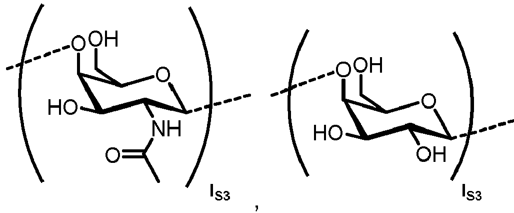 or 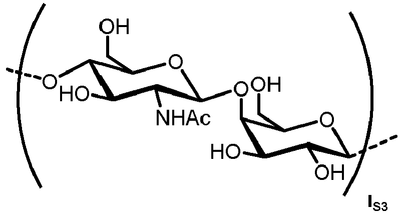
**L^{S4}** represents 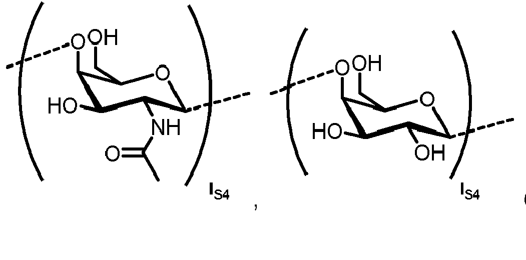 or 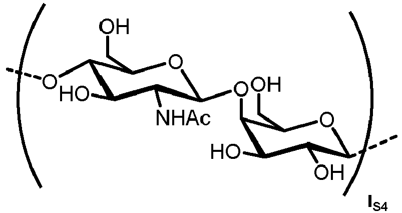
**L^{S5}** represents 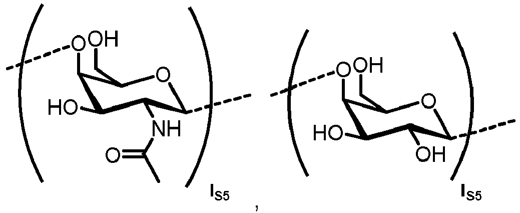 or 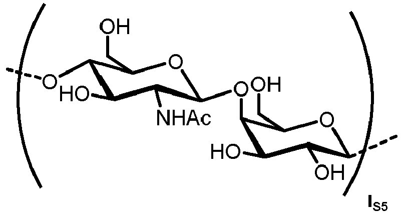
**T^{S1}**, **T^{S2}**, **T^{S3}**, **T^{S4}**, and **T^{S5}** represent independently of each other: 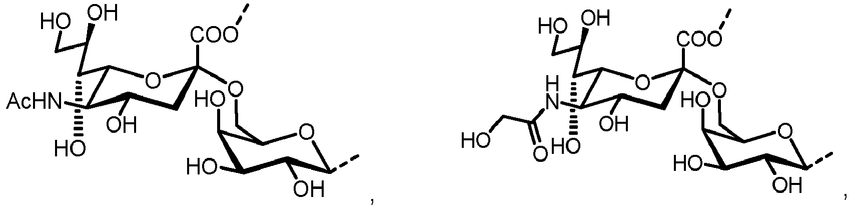 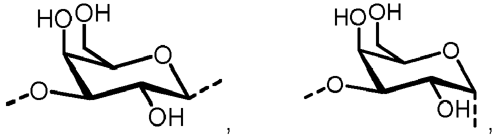 -SO₄- or a bond wherein m_{S1}, m_{S2}, m_{S3}, m_{S4}, m_{S5}, I_{S1}, I_{S2}, I_{S3}, I_{S4}, and I_{S5} represent independently of each other an integer selected from 0, 1, 2, 3, 4, 5, 6, 7, 8, 9, 10, 11, 12, 13, 14 and 15,
n_{S1}, n_{S2}, n_{S3}, n_{S4} and n_{S5} represent an integer selected from 0 and 1 ,
with the proviso that if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 ;
the method comprises the steps:
   A) providing a solution comprising a compound of formula **(II)**
   B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
      wherein the at least one glycosyltransferase enzyme is selected from *N*-acetylglucosaminyltransferase, mannosyltransferase, glucosyltransferase, galactosyltransferase, fucosyltransferase and sialyltransferase; and
   C) reacting the compound of formula **(III)** with the peptide **P** in the presence of an oligosaccharyltransferase enzyme to produce the compound of formula **(I).**

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B1) mixing the solution with a nucleotide sugar and a glycosyltransferase enzyme and reacting a resulting mixture
B2) repeating step B1 until a compound of formula **(III)** is produced, wherein the at least one nucleotide sugar is selected from GDP-mannose, UDP-galactose, UDP-GlcNAc, UDP-GalNAc, GDP-fucose, CMP-NeuAc and CMP-NeuGc, wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
with the proviso that if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 .

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein **F¹**, **F²** and **G** represent -H
with the proviso that if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 .

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from **N-**acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein m_{S1}, m_{S2}, m_{S3}, m_{S4}, and m_{S5} represent independently of each other an integer selected from 0, 1, 2, 3, 4 and 5, and with the proviso that if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 .

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein I_{S1}, I_{S2}, I_{S3}, I_{S4}, and I_{S5} represent independently of each other an integer selected from 0, 1, 2 and 3 and with the proviso that if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 .

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein **T^{S1}**, **T^{S2}**, **T^{S3}**, **T^{S4},** and **T^{S5}** represent a bond and with the proviso that
if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 .

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase, and wherein with the proviso that
if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 .

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
wherein the at least one glycosyltransferase enzyme is a transmembrane domain-deleted enzyme, and with the proviso that
if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 .

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a eukaryotic oligosaccharyltransferase to produce the compound of formula **(I),** and
with the proviso that
if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 .

Thus, in a further embodiment of the inventive method for producing a glycoprotein of general formula **(I),** wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of STT3A protein from *Trypanosoma brucei* to produce the compound of formula **(I),** and
with the proviso that
if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 .

Thus, in a further embodiment of the inventive method for producing a glycoprotein of general formula (I), wherein **C, NH, P** and **o** have the meanings as defined herein, the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of a bacterial oligosaccharyltransferase to produce the compound of formula (I), and
with the proviso that
if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 .

In a further embodiment of the inventive method for producing a glycoprotein of general formula **(I),** wherein **C, NH** and **o** have the meanings as defined herein, **P** represents a therapeutic protein of at least 10 amino acids comprising at least **o**-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the therapeutic protein **P** in the presence of an oligosaccharyltransferase enzyme to produce the compound of formula **(I),**
and with the proviso that
if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 .

In a further embodiment of the inventive method for producing a glycoprotein of general formula (I), wherein **C, NH** and **o** have the meanings as defined herein, **P** represents an aglycosylated peptide of at least 10 amino acids comprising at least **o**-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the aglycosylated peptide **P** in the presence of an oligosaccharyltransferase enzyme to produce the compound of formula **(I),**
and with the proviso that
if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 ..

In a further embodiment of the inventive method for producing a glycoprotein of general formula (I), wherein **C, NH** and **o** have the meanings as defined herein, **P** represents a peptide of at least 20 amino acids comprising at least o-times a consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula (I),
and with the proviso that
if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 ..

In a preferred embodiment of the inventive method for producing a glycoprotein of general formula **(I),** wherein **C,** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III)** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula (I),
and with the proviso that
if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0.

In a preferred embodiment of the inventive method for producing a glycoprotein of general formula (I), wherein **C, NH** and **o** have the meanings as defined herein, **P** represents a peptide of at least 20 amino acids comprising at least o-times a consensus sequence of N-V-T or N-Y-T,
the method comprises the steps:
A) providing a solution comprising a compound of formula **(II)**
B) mixing the solution with at least one nucleotide sugar and at least one glycosyltransferase enzyme and reacting a resulting mixture to produce a compound of formula **(III),** wherein the at least one nucleotide sugar is selected from GDP-mannose, UDP-galactose, UDP-GlcNAc, UDP-GalNAc, GDP-fucose, CMP-NeuAc and CMP-NeuGc,
   wherein the at least one glycosyltransferase enzyme is selected from *N*-acetylglucosaminyltransferase, mannosyltransferase, glucosyltransferase, galactosyltransferase, fucosyltransferase and sialyltransferase; and
C) reacting the compound of formula **(III)** with the peptide **P** in the presence of an oligosaccharyltransferase enzyme to produce the compound of formula **(I),**
and with the proviso that
if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) m_{S1} + m_{S4} + m_{S5} ≥ 0 ..

In a further embodiment of the inventive method for producing a glycoprotein of general formula (I'),

**[C'-NH]ₒP** **(I')**

wherein **C'** represents a carbohydrate of the following structure 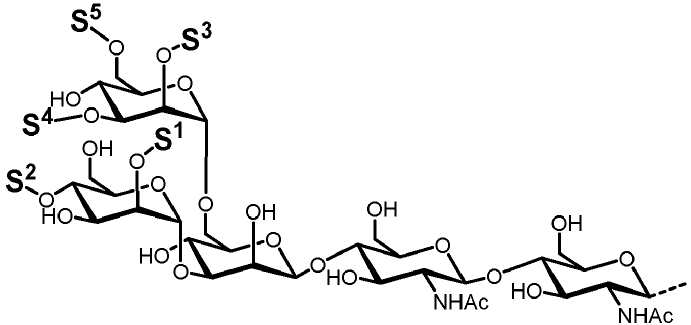 wherein o, **P,** and **NH** have the meanings as defined herein;
**S¹** represents 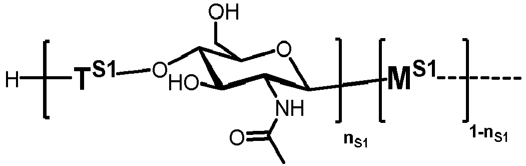
**S²** represents 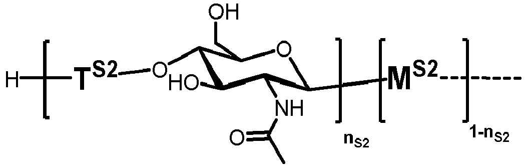
**S³** represents 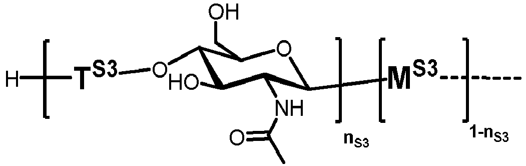
**S⁴** represents 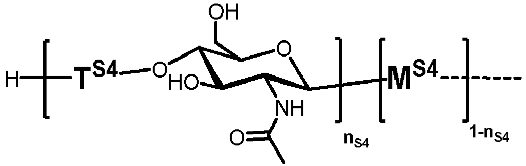
**S⁵** represents 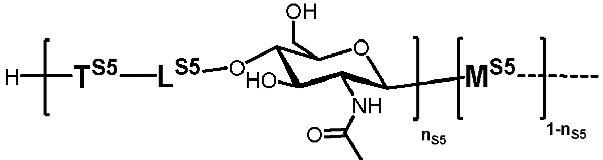
**M^{S1}** represents 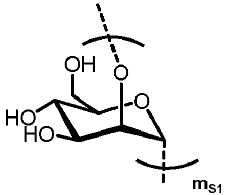
**M^{S2}** represents 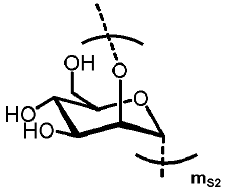
**M^{S3}** represents 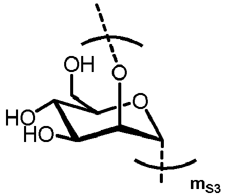
**M^{S4}** represents 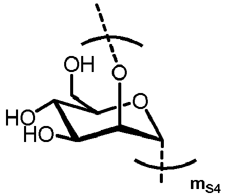
**M^{S5}** represents 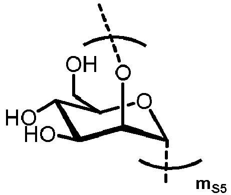
**T^{S1}**, **T^{S2}**, **T^{S3}**, **T^{S4}**, and **T^{S5}** represent independently of each other: 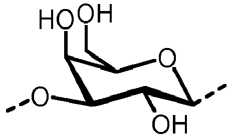 or a bond wherein m_{S1}, m_{S2}, m_{S3}, m_{S4}, and m_{S5} represent independently of each other an integer selected from 0 and 1,
n_{S1}, n_{S2}, n_{S3}, n_{S4} and n_{S5} represent an integer selected from 0 and 1 , with the proviso that if n_{S1} = 1 then
   n_{S3} = 1 and m_{S2} = m_{S4} = m_{S5} = 0; and
if n_{S1} = 0 then
   i) n_{S2} = n_{S3} = m_{S2} = m_{S3} = 0, and
   ii) 0 < m_{S1} + m_{S4} + m_{S5} ≤ 2 ;
the method comprises the steps:
   A) providing a solution comprising a compound of formula **(II')** 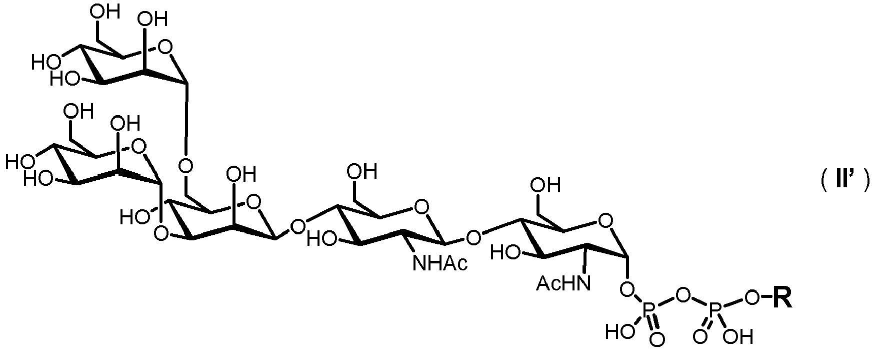 wherein R represents 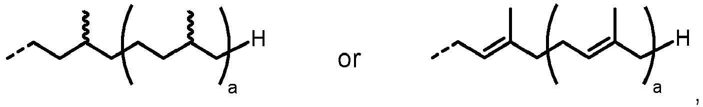 with a being an integer selected from 2, 3, 4, 5, 6, 7, 8, 9, 10, 11, 12, 13, 14 or 15,
   B) mixing the solution with at least one nucleotide sugar and at least one glycosyltransferase enzyme and reacting a resulting mixture to produce a compound of formula (III') 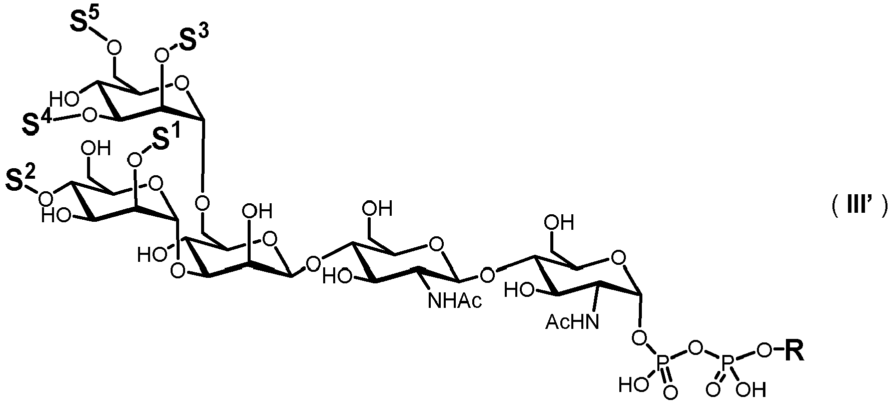 wherein the at least one nucleotide sugar is selected from GDP-mannose, UDP-galactose, and UDP-GlcNAc,
      wherein the at least one glycosyltransferase enzyme is selected from *N*-acetylglucosaminyltransferase, mannosyltransferase, glucosyltransferase, and galactosyltransferase; and
   C) reacting the compound of formula **(III')** with the peptide **P** in the presence of an oligosaccharyltransferase enzyme to produce the compound of formula (I').

Thus, in a further embodiment of the inventive method for producing a glycoprotein of general formula **(I'),** wherein **C', NH, P** and **o** have the meanings as defined herein, the method comprises the steps:
A) providing a solution comprising a compound of formula **(II')**
B1) mixing the solution with a nucleotide sugar and a glycosyltransferase enzyme and reacting a resulting mixture
   B2) repeating step B1 until a compound of formula **(III)** is produced, wherein the at least one nucleotide sugar is selected from GDP-mannose, UDP-galactose, and UDP-GlcNAc,
   wherein the at least one glycosyltransferase enzyme is selected from N-acetylglucosaminyltransferase, mannosyltransferase, glucosyltransferase, and galactosyltransferase; and
C) reacting the compound of formula **(III')** with the peptide **P** in the presence of an oligosaccharyltransferase enzyme to produce the compound of formula **(I').**

Thus, in a further embodiment of the inventive method for producing a glycoprotein of general formula **(I'),** wherein **C', NH, P** and **o** have the meanings as defined herein, the method comprises the steps:
A) providing a solution comprising a compound of formula **(II')**
   B) mixing the solution with at least one nucleotide sugar and at least one glycosyltransferase enzyme and reacting a resulting mixture to produce a compound of formula **(III')** wherein the at least one nucleotide sugar is selected from GDP-mannose, UDP-galactose, and UDP-GlcNAc,
   wherein the at least one glycosyltransferase enzyme is selected from *N*-acetylglucosaminyltransferase, mannosyltransferase, glucosyltransferase, and galactosyltransferase; and
C) reacting the compound of formula **(III')** with the peptide **P** in the presence of an oligosaccharyltransferase enzyme to produce the compound of formula (I'),
wherein **T^{S1}**, **T^{S2}**, **T^{S3}**, **T^{S4}**, and **T^{S5}** represent a bond.

Thus, in a further embodiment of the inventive method for producing a glycoprotein of general formula **(I'),** wherein **C', NH, P** and **o** have the meanings as defined herein, the method comprises the steps:
A) providing a solution comprising a compound of formula **(II')**
B) mixing the solution with at least one nucleotide sugar and at least one glycosyltransferase enzyme and reacting a resulting mixture to produce a compound of formula **(III')** wherein the at least one nucleotide sugar is selected from GDP-mannose, UDP-galactose, and UDP-GlcNAc,
   wherein the at least one glycosyltransferase enzyme is selected from *N*-acetylglucosaminyltransferase, mannosyltransferase, glucosyltransferase, and galactosyltransferase; and
C) reacting the compound of formula **(III')** with the peptide **P** in the presence of an oligosaccharyltransferase enzyme to produce the compound of formula (I'),
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

Thus, in a further embodiment of the inventive method for producing a glycoprotein of general formula (I'), wherein **C', NH, P** and **o** have the meanings as defined herein, the method comprises the steps:
A) providing a solution comprising a compound of formula **(II')**
B) mixing the solution with at least one nucleotide sugar and at least one glycosyltransferase enzyme and reacting a resulting mixture to produce a compound of formula **(III')** wherein the at least one nucleotide sugar is selected from GDP-mannose, UDP-galactose, and UDP-GlcNAc,
   wherein the at least one glycosyltransferase enzyme is selected from *N*-acetylglucosaminyltransferase, mannosyltransferase, glucosyltransferase, and galactosyltransferase; and
C) reacting the compound of formula **(III')** with the peptide **P** in the presence of an oligosaccharyltransferase enzyme to produce the compound of formula **(I'),**
wherein the at least one glycosyltransferase enzyme is a transmembrane domain-deleted enzyme.

Thus, in a further embodiment of the inventive method for producing a glycoprotein of general formula **(I'),** wherein **C', NH, P** and **o** have the meanings as defined herein, the method comprises the steps:
A) providing a solution comprising a compound of formula **(II')**
B) mixing the solution with at least one nucleotide sugar and at least one glycosyltransferase enzyme and reacting a resulting mixture to produce a compound of formula **(III')** wherein the at least one nucleotide sugar is selected from GDP-mannose, UDP-galactose, and UDP-GlcNAc,
   wherein the at least one glycosyltransferase enzyme is selected from *N*-acetylglucosaminyltransferase, mannosyltransferase, glucosyltransferase, and galactosyltransferase; and
C) reacting the compound of formula **(III')** with the peptide **P** in the presence of a eukaryotic oligosaccharyltransferase to produce the compound of formula **(I').**

Thus, in a further embodiment of the inventive method for producing a glycoprotein of general formula **(I'),** wherein **C', NH, P** and **o** have the meanings as defined herein, the method comprises the steps:
A) providing a solution comprising a compound of formula **(II')**
B) mixing the solution with at least one nucleotide sugar and at least one glycosyltransferase enzyme and reacting a resulting mixture to produce a compound of formula **(III')** wherein the at least one nucleotide sugar is selected from GDP-mannose, UDP-galactose, and UDP-GlcNAc,
   wherein the at least one glycosyltransferase enzyme is selected from N-acetylglucosaminyltransferase, mannosyltransferase, glucosyltransferase, and galactosyltransferase; and
C) reacting the compound of formula **(III')** with the peptide **P** in the presence of STT3A protein from *Trypanosoma brucei* to produce the compound of formula **(I').**

Thus, in a further embodiment of the inventive method for producing a glycoprotein of general formula **(I'),** wherein **C', NH, P** and **o** have the meanings as defined herein, the method comprises the steps:
A) providing a solution comprising a compound of formula **(II')**
B) mixing the solution with at least one nucleotide sugar and at least one glycosyltransferase enzyme and reacting a resulting mixture to produce a compound of formula **(III')** wherein the at least one nucleotide sugar is selected from GDP-mannose, UDP-galactose, and UDP-GlcNAc,
   wherein the at least one glycosyltransferase enzyme is selected from *N*-acetylglucosaminyltransferase, mannosyltransferase, glucosyltransferase, and galactosyltransferase; and
C) reacting the compound of formula **(III')** with the peptide **P** in the presence of a bacterial oligosaccharyltransferase to produce the compound of formula **(I').**

In a further embodiment of the inventive method for producing a glycoprotein of general formula **(I'),** wherein **C', NH** and **o** have the meanings as defined herein, **P** represents a therapeutic protein of at least 10 amino acids comprising at least **o**-times a consensus sequence of N-X-S/T, wherein X represents any amino acid except proline,
the method comprises the steps:
A) providing a solution comprising a compound of formula **(II')**
B) mixing the solution with at least one nucleotide sugar and at least one glycosyltransferase enzyme and reacting a resulting mixture to produce a compound of formula **(III')** wherein the at least one nucleotide sugar is selected from GDP-mannose, UDP-galactose, and UDP-GlcNAc,
   wherein the at least one glycosyltransferase enzyme is selected from *N*-acetylglucosaminyltransferase, mannosyltransferase, glucosyltransferase, and galactosyltransferase; and
C) reacting the compound of formula **(III')** with the therapeutic protein **P** in the presence of an oligosaccharyltransferase enzyme to produce the compound of formula **(I').**

In a further embodiment of the inventive method for producing a glycoprotein of general formula **(I'),** wherein **C', NH** and o have the meanings as defined herein, **P** represents an aglycosylated peptide of at least 10 amino acids comprising at least **o-**times a consensus sequence of N-X-S/T, wherein X represents any amino acid except proline,
the method comprises the steps:
A) providing a solution comprising a compound of formula **(II')**
B) mixing the solution with at least one nucleotide sugar and at least one glycosyltransferase enzyme and reacting a resulting mixture to produce a compound of formula **(III')** wherein the at least one nucleotide sugar is selected from GDP-mannose, UDP-galactose, and UDP-GlcNAc,
   wherein the at least one glycosyltransferase enzyme is selected from *N*-acetylglucosaminyltransferase, mannosyltransferase, glucosyltransferase, and galactosyltransferase; and
C) reacting the compound of formula **(III')** with the aglycosylated peptide **P** in the presence of an oligosaccharyltransferase enzyme to produce the compound of formula **(I').**

In a further embodiment of the inventive method for producing a glycoprotein of general formula **(I'),** wherein **C', NH** and **o** have the meanings as defined herein, **P** represents a peptide of at least 20 amino acids comprising at least **o**-times a consensus sequence of N-X-S/T, wherein X represents any amino acid except proline,
the method comprises the steps:
A) providing a solution comprising a compound of formula **(II')**
B) mixing the solution with at least one nucleotide sugar and at least one glycosyltransferase enzyme and reacting a resulting mixture to produce a compound of formula **(III')** wherein the at least one nucleotide sugar is selected from GDP-mannose, UDP-galactose, and UDP-GlcNAc,
   wherein the at least one glycosyltransferase enzyme is selected from *N*-acetylglucosaminyltransferase, mannosyltransferase, glucosyltransferase, and galactosyltransferase; and
C) reacting the compound of formula **(III')** with the peptide **P** in the presence of an oligosaccharyltransferase enzyme to produce the compound of formula (I').

In a preferred embodiment of the inventive method for producing a glycoprotein of general formula **(I'),** wherein **C',** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
A) providing a solution comprising a compound of formula **(II')**
B) mixing the solution with at least one nucleotide sugar and at least one glycosyltransferase enzyme and reacting a resulting mixture to produce a compound of formula **(III')** wherein the at least one nucleotide sugar is selected from GDP-mannose, UDP-galactose, and UDP-GlcNAc,
   wherein the at least one glycosyltransferase enzyme is selected from *N*-acetylglucosaminyltransferase, mannosyltransferase, glucosyltransferase, and galactosyltransferase; and
C) reacting the compound of formula **(III')** with the peptide **P** in the presence of an oligosaccharyltransferase enzyme to produce the compound of formula (I').

In a preferred embodiment of the inventive method for producing a glycoprotein of general formula **(I'),** wherein **C',** and **NH** have the meanings as defined herein and **o** is an integer as defined as follows:

| | |
|---|---|
| o is between 1 and 10 | if **P** consists of 10 to 50 amino acids |
| o is between 1 and 20 | if **P** consists of 51 to 100 amino acids |
| o is between 1 and 40 | if **P** consists of 101 to 200 amino acids |
| o is between 1 and 60 | if **P** consists of 201 to 300 amino acids |
| o is between 1 and 80 | if **P** consists of 301 to 400 amino acids |
| o is between 1 and 100 | if **P** consists of 401 to 500 amino acids, |

**P** represents a peptide of at least 20 amino acids comprising at least one consensus sequence of N-X-S/T, wherein X represents any amino acid except proline,
the method comprises the steps:
   A) providing a solution comprising a compound of formula **(II')**
   B) mixing the solution with at least one nucleotide sugar and at least one glycosyltransferase enzyme and reacting a resulting mixture to produce a compound of formula **(III')** wherein the at least one nucleotide sugar is selected from GDP-mannose, UDP-galactose, and UDP-GlcNAc,
      wherein the at least one glycosyltransferase enzyme is selected from *N*-acetylglucosaminyltransferase, mannosyltransferase, glucosyltransferase, and galactosyltransferase; and
   C) reacting the compound of formula (**II'I**) with the peptide **P** in the presence of an oligosaccharyltransferase enzyme to produce the compound of formula (I').

In a further preferred embodiment, **o** is an integer defined as follows:

| | |
|---|---|
| o is 1 | if **P** consists of 10 to 50 amino acids |
| o is 1 or 2 | if **P** consists of 51 to 100 amino acids |
| o is 1, 2 or 3 | if **P** consists of 101 to 200 amino acids |
| o is 2, 3 or 4 | if **P** consists of 201 to 300 amino acids |
| o is 2, 3, 4 or 5 | if **P** consists of 301 to 400 amino acids |
| o is 2, 3, 4, 5 or 6 | if **P** consists of 401 to 500 amino acids. |

In a preferred embodiment of the inventive method for producing a glycoprotein of general formula **(I'),** wherein **C', NH** and o have the meanings as defined herein, **P** represents a peptide of at least 20 amino acids comprising at least **o**-times a consensus sequence of N-V-T or N-Y-T,
the method comprises the steps:
A) providing a solution comprising a compound of formula **(II')**
B) mixing the solution with at least one nucleotide sugar and at least one glycosyltransferase enzyme and reacting a resulting mixture to produce a compound of formula **(III'),** wherein the at least one nucleotide sugar is selected from GDP-mannose, UDP-galactose, and UDP-GlcNAc,
   wherein the at least one glycosyltransferase enzyme is selected from *N*-acetylglucosaminyltransferase, mannosyltransferase, glucosyltransferase, and galactosyltransferase; and
C) reacting the compound of formula **(III')** with the peptide **P** in the presence of an oligosaccharyltransferase enzyme to produce the compound of formula **(I').**

### Definitions

As used herein, the term **"glycosyltransferase"** refers to a polypeptide having glycosyltransferase activity, i.e. a glycosyltransferase catalyzes the transfer reaction of a monosaccharide from a nucleotide sugar to a free hydroxyl group of an acceptor saccharide. Glycosyltransferases belong to the EC class 2.4. Representatives of the glycosyltransferases used in the inventive methods described herein include but are not limited to *N*-acetylglucosaminyltransferase, mannosyltransferase, glucosyltransferase, galactosyltransferase, fucosyltransferase, sialyltransferase, α-1,3-mannosyl-glycoprotein 2-β-*N*-acetylglucosaminyltransferase, α-1,6-mannosyl-glycoprotein 2-β-*N*-acetylglucosaminyltransferase, β-1,4-mannosyl-glycoprotein 4-β-*N*-acetylglucosaminyltransferase, α-1,3-mannosyl-glycoprotein 4-β-*N*-acetylglucosaminyltransferase, α-1,2-mannosyltransferase, α-1,3-mannosyltransferase, α-1,6-mannosyltransferase, α-1,3/1,6-mannosyltransferase, α-1,3-galactosyltransferase, β-1,4-galactosyltransferase, α-2,3-sialyltransferase, α-2,6-sialyltransferase, α-1,2-fucosyltransferase, α-1,3-fucosyltransferase, α-1,6-fucosyltransferase, α-1,2-glucosyltransferase, and α-1,3-glucosyltransferase.

As used herein, the term **"oligosaccharyltransferase"** refers to a polypeptide having oligosaccharyltransferase activity, i.e. an oligosaccharyltransferase catalyzes the transfer reaction of an oligosaccharide from a lipid moiety to an asparagine γ-amido residue within the N-X-T/S (wherein X represents any amino acid except proline) consensus sequon of a polypeptide chain, thereby forming an α-*N*-glycosidic bond. Oligosaccharyltransferase belongs to the EC class 2.4.1.119.

As used herein, the term **"transmembrane domain-deleted"** refers to a deletional or substitutional recombinant variant of an enzyme, which lacks the transmembrane domain. Deletion or substitution of the transmembrane facilitates the use of the respective enzymes in chemical reactions, for instance, recovery and isolation is simplified due to the reduced cellular or membrane lipid affinity. Further, transmembrane domain deleted variants exhibit a better water solubility which renders the use of detergents dispensable.

As used herein, the term **"nucleotide sugar"** refers to nucleoside diphosphate-monosaccharide compounds which are substrates of glycosyltransferase enzymes and serve as monosaccharide donors in glycosylation reaction. Representatives of the nucleotide sugars used in the inventive methods described herein include but are not limited to are GDP-mannose, UDP-galactose, UDP-GlcNAc, UDP-GalNAc, GDP-fucose, CMP-NeuAc and CMP-NeuGc.

As used herein, **"saccharide"** refers to but not restricted to monosaccharide, disaccharide, trisaccharide, tetrasaccharide, pentasaccharide, hexasaccharide, heptasaccharide, octasaccharide..., oligosaccharide, glycan and polysaccharide. The saccharide comprises preferably monosaccharide units selected from: D-Galactose, L-Fucose, *N*-acetyl-D-glucosamine, α-D-mannopyranose, α-D-galactopyranose, β-D-mannopyranose, β-D-galactopyranose, *N*-acetylneuraminic acid, and *N*-glycolylneuraminic acid.

The saccharides are further optionally modified to carry amide, carbonate, carbamate, carbonyl, thiocarbonyl, carboxy, thiocarboxy, ester, thioester, ether, epoxy, hydroxyalkyl, alkylenyl, phenylene, alkenyl, imino, imide, isourea, thiocarbamate, thiourea and/or urea moieties.

As used herein, the term **"glycopeptide"** refers to a peptide that contains carbohydrate moieties covalently attached to the side chains of the amino acid residues that constitute the peptide. The carbohydrate moieties form side chains and are either O-glycosidic connected to the hydroxy group of a serine or threonine residue or *N*-glycosidic connected to the amido nitrogen of an asparagine residue.

As used herein, the term **"glycoprotein"** refers to a polypeptide that contains carbohydrate moieties covalently attached to the side chains of the amino acid residues that constitute the polypeptide. The carbohydrate moieties form side chains and are either O-glycosidic connected to the hydroxy group of a serine or threonine residue or *N*-glycosidic connected to the amido nitrogen of an asparagine residue.

As used herein, the term **"protein"** refers to a polypeptide that contains or lacks of carbohydrate moieties covalently attached to the side chains of the amino acid residues that constitute the polypeptide including aglycosylated proteins and glycosylated proteins.

As used herein, the term **"peptide"** refers to a peptide that contains or lacks of carbohydrate moieties covalently attached to the side chains of the amino acid residues that constitute the peptide, including aglycosylated peptides and glycosylated peptides.

### High-Mannose

The carbohydrate **C** can be any high-mannose, complex-type or hybrid-type oligosaccharide comprising a GlcNAc₂Man₃ core structure as naturally occurring in mammals and humans.

Thus, a further aspect of the present invention is directed to a method for producing a glycoprotein of general formula (**Ia**)

**[Ca-NH]ₒP** (**Ia**)

wherein **Ca** represents a carbohydrate of the following structure 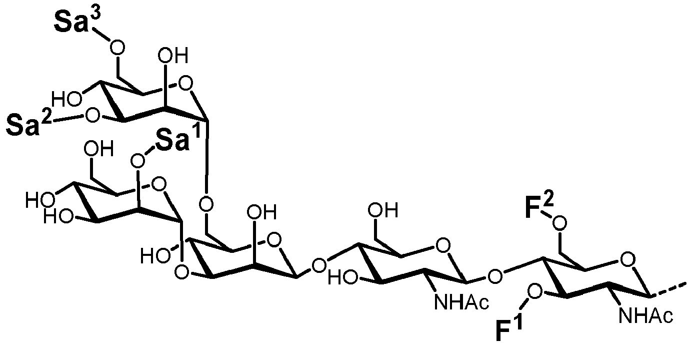
**o** is an integer representing the number of carbohydrates **C** which are bound to a peptide **P,**
**P** represents a peptide of at least 10 amino acids comprising at least **o**-times a consensus sequence of N-X-S/T, wherein X represents any amino acid except proline, and
**NH** represents an asparagine γ-amido group of the consensus sequence;
**F¹** and **F²** represent 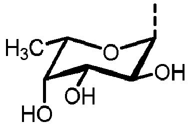 or -H; with the proviso that **F¹** and **F²** cannot be simultaneously 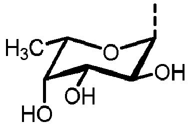
**Sa¹** represents 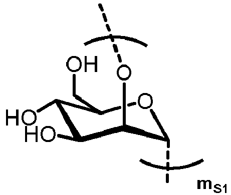
**Sa²** represents 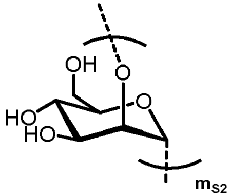
**Sa³** represents 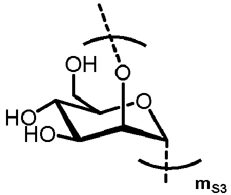 wherein m_{S1}, m_{S2}, and m_{S3} represent independently of each other an integer selected from 0, 1, 2, 3, 4, 5, 6, 7, 8, 9, 10, 11, 12, 13, 14 and 15,
comprising the steps:
   Aa) providing a solution comprising a compound of formula (**IIIa**) 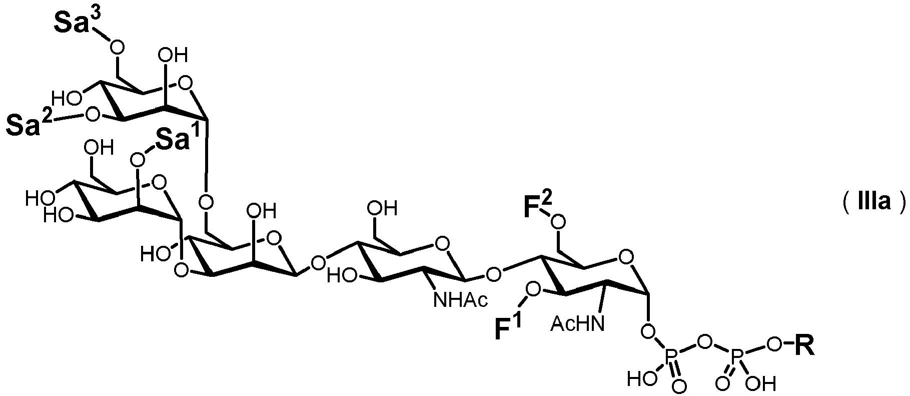 wherein **R** represents 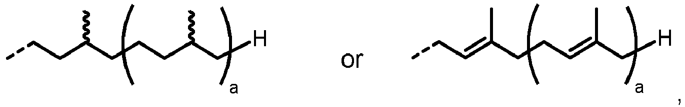 with a being an integer selected from 2, 3, 4, 5, 6, 7, 8, 9, 10, 11, 12, 13, 14 or 15,
   Ba) reacting the compound of formula (**IIIa**) with the peptide **P** in the presence of an oligosaccharyltransferase enzyme to produce the compound of formula (**Ia**).

The inventive methods described herein are directed to the *in vitro* preparation of glycoproteins of formula (**Ia**) which are *N*-glycans having an *N*-glycosidic bond between the terminal GlcNAc and an asparagine γ-amido group of the peptide **P.** The carbohydrate moiety **Ca** comprises a lipid-linked core structure of LL-GlcNac₂Man₃ with further mannose units at the terminal mannoses (high mannose).

The peptide **P** can be any type of a polypeptide having at least 10 amino acids and comprising at least one consensus sequence of N-X-S/T, wherein X represents any amino acid except proline, including proteins, folded peptides, folded proteins, therapeutic peptides, therapeutic proteins, aglycosylated peptides or aglycosylated proteins.

The oligosaccharyltransferase enzyme used in the inventive methods attaches the carbohydrate **Ca** to an asparagine γ-amido group of the consensus sequence of N-X-S/T, wherein X represents any amino acid except proline. Thus, the peptide P comprises at least one consensus sequence of N-X-S/T, wherein X represents any amino acid except proline. If the glycoprotein of formula **(I)** comprises **o** carbohydrate moieties **Ca,** the peptide **P** comprises at least o-times the consensus sequence of N-X-S/T, wherein X represents any amino acid except proline.

The number **o** of carbohydrates **Ca** which are bound to a peptide **P** may depend on the length of the peptide **P** itself. For instance, for shorter peptides consisting of 10 to 20 amino acids **o** may be 1, thus only one carbohydrate is bound to the peptide, whereas for peptides consisting of 20 to 50 amino acids **o** may be 1 or 2, thus one or two carbohydrates are bound to the peptide, etc.

Thus, in one embodiment of the inventive method for producing a glycoprotein of general formula (**Ia**), **o** is an integer as defined as follows:

| | |
|---|---|
| o is between 1 and 10 | if **P** consists of 10 to 50 amino acids |
| o is between 1 and 20 | if **P** consists of 51 to 100 amino acids |
| o is between 1 and 40 | if **P** consists of 101 to 200 amino acids |
| o is between 1 and 60 | if **P** consists of 201 to 300 amino acids |
| o is between 1 and 80 | if **P** consists of 301 to 400 amino acids |
| o is between 1 and 100 | if **P** consists of 401 to 500 amino acids, |

the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**),
wherein **Ca, NH** and **P** have the meanings as defined herein.

In a preferred embodiment, o is an integer selected from:

| | |
|---|---|
| o is 1 | if **P** consists of 10 to 50 amino acids |
| o is 1 or 2 | if **P** consists of 51 to 100 amino acids |
| o is 1, 2 or 3 | if **P** consists of 101 to 200 amino acids |
| o is 2, 3 or 4 | if **P** consists of 201 to 300 amino acids |
| o is 2, 3, 4 or 5 | if **P** consists of 301 to 400 amino acids |
| o is 2, 3, 4, 5 or 6 | if **P** consists of 401 to 500 amino acids, |

the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**),
wherein **Ca, NH** and **P** have the meanings as defined herein.

In a preferred embodiment, **o** is 1. Thus, the inventive method for producing a glycoprotein of general formula (**Ia**), comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**),
wherein **Ca, NH** and **P** have the meanings as defined herein and **o** represents 1.

In one embodiment of the inventive method for producing a glycoprotein of general formula (**Ia**), wherein **Ca, NH** and **o** have the meanings as defined herein, **P** represents a peptide of at least 10 amino acids comprising at least **o**-times a consensus sequence of N-V-T,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**).

The inventors have found that the transfer of the carbohydrate **Ca** to the peptide **P** by an oligosaccharyltransferase enzyme is particularly efficient when the consensus sequence of peptide **P** is N-V-T or N-Y-T.

Thus, in one embodiment of the inventive method for producing a glycoprotein of general formula (**Ia**), wherein **Ca, NH** and o have the meanings as defined herein, **P** represents a peptide of at least 10 amino acids comprising at least **o**-times a consensus sequence of N-V-T or N-Y-T,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**).

In one embodiment of the inventive method for producing a glycoprotein of general formula (**Ia**), wherein **Ca, NH** and **o** have the meanings as defined herein, **P** represents a peptide of at least 10 amino acids comprising at least **o**-times a consensus sequence of N-V-T,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**).

In one embodiment of the inventive method for producing a glycoprotein of general formula (**Ia**), wherein **Ca, NH** and o have the meanings as defined herein, **P** represents a peptide of at least 10 amino acids comprising at least o-times a consensus sequence of N-Y-T,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**).

In a further embodiment of the inventive method for producing a glycoprotein of general formula (**Ia**), wherein **Ca, NH** and o have the meanings as defined herein, **P** represents a peptide of at least 20 amino acids comprising at least **o-**times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**).

In a preferred embodiment of the inventive method for producing a glycoprotein of general formula (**Ia**), wherein **Ca,** and **NH** have the meanings as defined herein and **o** represents 1, **P** represents a peptide of at least 20 amino acids comprising at least a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**).

In a preferred embodiment of the inventive method for producing a glycoprotein of general formula (**Ia**), wherein **Ca, NH** and **o** have the meanings as defined herein, **P** represents a peptide of at least 20 amino acids comprising at least **o**-times a consensus sequence of N-V-T or N-Y-T,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**).

In a further embodiment of the inventive method for producing a glycoprotein of general formula (**Ia**), wherein **Ca, NH** and o have the meanings as defined herein, P represents a folded peptide of at least 10 amino acids comprising at least o-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the folded peptide **P** in the presence of an oligosaccharyltransferase enzyme to produce the compound of formula (**Ia**).

In a further embodiment of the inventive method for producing a glycoprotein of general formula (**Ia**), wherein **Ca, NH** and o have the meanings as defined herein, **P** represents a folded peptide of at least 20 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the folded peptide **P** in the presence of an oligosaccharyltransferase enzyme to produce the compound of formula (**Ia**).

In a preferred embodiment of the inventive method for producing a glycoprotein of general formula (**Ia**), wherein **Ca,** and **NH** have the meanings as defined herein and **o** represents 1, **P** represents a folded peptide of at least 20 amino acids comprising at least a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the folded peptide **P** in the presence of an oligosaccharyltransferase enzyme to produce the compound of formula (**Ia**).

In a preferred embodiment of the inventive method for producing a glycoprotein of general formula (**Ia**), wherein **Ca, NH** and **o** have the meanings as defined herein, **P** represents a folded peptide of at least 20 amino acids comprising at least **o**-times a consensus sequence of N-V-T or N-Y-T,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**).

In a preferred embodiment, **P** is an aglycosylated peptide or protein. Aglycosylated peptides or proteins can be recombinantly produced in cell-based bacterial and cell-free production systems. An aglycosylated peptide or aglycosylated protein, as used herein, is a peptide or protein which is not functionalized with a carbohydrate at an amino acid residue.

Thus, in a further embodiment of the inventive method for producing a glycoprotein of general formula (**Ia**), wherein **Ca, NH** and o have the meanings as defined herein, **P** represents an aglycosylated peptide or an aglycosylated protein of at least 10 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with **P** in the presence of an oligosaccharyltransferase enzyme to produce the compound of formula (**Ia**).

In a further embodiment of the inventive method for producing a glycoprotein of general formula (**Ia**), wherein **Ca, NH** and o have the meanings as defined herein, **P** represents an aglycosylated peptide or an aglycosylated protein of at least 20 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**).

In a preferred embodiment of the inventive method for producing a glycoprotein of general formula (**Ia**), wherein **Ca,** and **NH** have the meanings as defined herein and **o** represents 1, **P** represents an aglycosylated peptide or an aglycosylated protein of at least 20 amino acids comprising at least a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**).

In a preferred embodiment of the inventive method for producing a glycoprotein of general formula (**Ia**), wherein **Ca, NH** and **o** have the meanings as defined herein, **P** represents an aglycosylated peptide or an aglycosylated protein of at least 20 amino acids comprising at least o-times a consensus sequence of N-V-T or N-Y-T,
the method comprises the steps:
Aa) providing a solution comprising a compound of formula (**IIIa**),
Ba) reacting the compound of formula (**IIIa**) with **P** in the presence of an oligosaccharyltransferase enzyme to produce the compound of formula (**Ia**).

In a preferred embodiment, **P** is a therapeutic protein. Thus, in a further embodiment of the inventive method for producing a glycoprotein of general formula (**Ia**), wherein **Ca, NH** and **o** have the meanings as defined herein, **P** represents a therapeutic protein of at least 10 amino acids comprising at least o-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the therapeutic protein **P** in the presence of an oligosaccharyltransferase enzyme to produce the compound of formula (**Ia**).

In a further embodiment of the inventive method for producing a glycoprotein of general formula (**Ia**), wherein **Ca, NH** and **o** have the meanings as defined herein, **P** represents an aglycosylated therapeutic protein of at least 10 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with **P** in the presence of an oligosaccharyltransferase enzyme to produce the compound of formula (**Ia**).

In a further embodiment of the inventive method for producing a glycoprotein of general formula (**Ia**), wherein **Ca, NH** and o have the meanings as defined herein, **P** represents a therapeutic protein of at least 20 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the therapeutic protein **P** in the presence of an oligosaccharyltransferase enzyme to produce the compound of formula (**Ia**).

In a preferred embodiment of the inventive method for producing a glycoprotein of general formula (**Ia**), wherein **Ca,** and **NH** have the meanings as defined herein and **o** represents 1, **P** represents a therapeutic protein of at least 20 amino acids comprising at least a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the therapeutic protein **P** in the presence of an oligosaccharyltransferase enzyme to produce the compound of formula (**Ia**).

In a preferred embodiment of the inventive method for producing a glycoprotein of general formula (**Ia**), wherein **Ca, NH** and o have the meanings as defined herein, **P** represents a therapeutic protein of at least 20 amino acids comprising at least **o-**times a consensus sequence of N-V-T or N-Y-T,
the method comprises the steps:
Aa) providing a solution comprising a compound of formula (**IIIa**),
Ba) reacting the compound of formula (**IIIa**) with the therapeutic protein **P** in the presence of an oligosaccharyltransferase enzyme to produce the compound of formula (**Ia**).

In a preferred embodiment, the oligosaccharyltransferase enzyme is a eukaryotic oligosaccharyltransferase. In a more preferred embodiment, the oligosaccharyltransferase enzyme is STT3A protein from *Trypanosoma brucei.* In contrast to oligosaccharyltransferases from higher eukaryotes, which are only present in two catalytically active isoforms STT3, the single-subunit OST of *Trypanosoma brucei* recognizes also human-like glycans and is thus more efficient for transferring glycans to a polypeptide.

In a further embodiment, the oligosaccharyltransferase enzyme is a bacterial oligosaccharyltransferase. In a further embodiment, the oligosaccharyltransferase enzyme is selected from one of the following organisms: *Campylobacter jejuni* strain RM1221, *Campylobacter lari strain* RM2100 / D67 / ATC BAA-1060, *Campylobacter coli, Campylobacter upsaliensis, Desulfovibrio desulfuricans* strain DSM 7057, *Megalodesulfovibrio gigas* DSM 1382, *Desulfovibrio vulgaris, Trypanosoma brucei, Saccharomyces cerevisiae* strain ATCC 204508 / S288c, *Mus musculus, Schizosaccharomyces pombe* strain 972 / ATCC 24843, *Canis lupus familiaris, Arabidopsis thaliana, Caenorhabditis elegans, Bos taurus, Pongo pygmaeus abelii, Oryza stiva japonica, Dictyostelium discoideum, Brachypodium distachyon, Brachypodium retusum, Rattus norvegicus, Brachypodium sylvaticum, Brachypodium pinnatum, Brachypodium rupestre,* and *Campylobacter jejuni* strain ATCC 700819 / NCTC 11168.

In a further embodiment, the oligosaccharyltransferase enzyme consists of an amino acid sequence as set forth in SEQ ID Nos 3 to 31.

Thus, in a further embodiment of the inventive method for producing a glycoprotein of general formula (**Ia**), wherein **Ca, NH, P** and **o** have the meanings as defined herein, the method comprises the steps:
Aa) providing a solution comprising a compound of formula (**IIIa**),
Ba) reacting the compound of formula (**IIIa**) with the peptide **P** in the presence of a eukaryotic oligosaccharyltransferase to produce the compound of formula (**Ia**).

In a further embodiment of the inventive method for producing a glycoprotein of general formula (**Ia**), wherein **Ca, NH** and **o** have the meanings as defined herein, **P** represents a therapeutic protein of at least 10 amino acids comprising at least **o-**times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the therapeutic protein **P** in the presence of a eukaryotic oligosaccharyltransferase to produce the compound of formula (**Ia**).

In a further embodiment of the inventive method for producing a glycoprotein of general formula (**Ia**), wherein **Ca, NH** and **o** have the meanings as defined herein, **P** represents an aglycosylated peptide of at least 10 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the aglycosylated peptide **P** in the presence of a eukaryotic oligosaccharyltransferase to produce the compound of formula (**Ia**).

In a further embodiment of the inventive method for producing a glycoprotein of general formula (**Ia**), wherein **Ca, NH** and **o** have the meanings as defined herein, **P** represents a peptide of at least 20 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the peptide **P** in the presence of a eukaryotic oligosaccharyltransferase to produce the compound of formula (**Ia**).

In a preferred embodiment of the inventive method for producing a glycoprotein of general formula (**Ia**), wherein **Ca,** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the peptide **P** in the presence of a eukaryotic oligosaccharyltransferase to produce the compound of formula (**Ia**).

In a preferred embodiment of the inventive method for producing a glycoprotein of general formula (**Ia**), wherein **Ca, NH** and **o** have the meanings as defined herein, P represents a peptide of at least 20 amino acids comprising at least o-times a consensus sequence of N-V-T or N-Y-T,
the method comprises the steps:
Aa) providing a solution comprising a compound of formula (**IIIa**),
Ba) reacting the compound of formula (**IIIa**) with the peptide **P** in the presence of a eukaryotic oligosaccharyltransferase to produce the compound of formula (**Ia**).

Thus, in a further embodiment of the inventive method for producing a glycoprotein of general formula (**Ia**), wherein **Ca, NH, P** and **o** have the meanings as defined herein, the method comprises the steps:
Aa) providing a solution comprising a compound of formula (**IIIa**),
Ba) reacting the compound of formula (**IIIa**) with the peptide **P** in the presence of a bacterial oligosaccharyltransferase to produce the compound of formula (**Ia**).

In a further embodiment of the inventive method for producing a glycoprotein of general formula (**Ia**), wherein **Ca, NH** and **o** have the meanings as defined herein, **P** represents a therapeutic protein of at least 10 amino acids comprising at least **o-**times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the therapeutic protein **P** in the presence of a bacterial oligosaccharyltransferase to produce the compound of formula (**Ia**).

In a further embodiment of the inventive method for producing a glycoprotein of general formula (**Ia**), wherein **Ca, NH** and **o** have the meanings as defined herein, **P** represents an aglycosylated peptide of at least 10 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the aglycosylated peptide **P** in the presence of a bacterial oligosaccharyltransferase to produce the compound of formula (**Ia**).

In a further embodiment of the inventive method for producing a glycoprotein of general formula (**Ia**), wherein **Ca, NH** and **o** have the meanings as defined herein, **P** represents a peptide of at least 20 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the peptide **P** in the presence of a bacterial oligosaccharyltransferase to produce the compound of formula (**Ia**).

In a preferred embodiment of the inventive method for producing a glycoprotein of general formula (**Ia**), wherein **Ca,** and **NH** have the meanings as defined herein and o represents 1, **P** represents a peptide of at least 20 amino acids comprising at least one consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the peptide **P** in the presence of a bacterial oligosaccharyltransferase to produce the compound of formula (**Ia**).

In a preferred embodiment of the inventive method for producing a glycoprotein of general formula (**Ia**), wherein **Ca, NH** and **o** have the meanings as defined herein, **P** represents a peptide of at least 20 amino acids comprising at least o-times a consensus sequence of N-V-T or N-Y-T,
the method comprises the steps:
Aa) providing a solution comprising a compound of formula (**IIIa**),
Ba) reacting the compound of formula (**IIIa**) with the peptide **P** in the presence of a bacterial oligosaccharyltransferase to produce the compound of formula (**Ia**).

Thus, in a further embodiment of the inventive method for producing a glycoprotein of general formula (**Ia**), wherein **Ca, NH, P** and **o** have the meanings as defined herein, the method comprises the steps:
Aa) providing a solution comprising a compound of formula (**IIIa**),
Ba) reacting the compound of formula (**IIIa**) with the peptide **P** in the presence of STT3A protein from *Trypanosoma brucei* to produce the compound of formula (**Ia**).

In a further embodiment of the inventive method for producing a glycoprotein of general formula (**Ia**), wherein **Ca, NH** and **o** have the meanings as defined herein, **P** represents a therapeutic protein of at least 10 amino acids comprising at least **o-**times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the therapeutic protein **P** in the presence of STT3A protein from *Trypanosoma brucei* to produce the compound of formula (**Ia**).

In a further embodiment of the inventive method for producing a glycoprotein of general formula (**Ia**), wherein **Ca, NH** and **o** have the meanings as defined herein, **P** represents an aglycosylated peptide of at least 10 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the aglycosylated peptide **P** in the presence of STT3A protein from *Trypanosoma brucei* to produce the compound of formula (**Ia**).

In a further embodiment of the inventive method for producing a glycoprotein of general formula (**Ia**), wherein **Ca, NH** and **o** have the meanings as defined herein, **P** represents a peptide of at least 20 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the peptide **P** in the presence of STT3A protein from *Trypanosoma brucei* to produce the compound of formula (**Ia**).

In a preferred embodiment of the inventive method for producing a glycoprotein of general formula (**Ia**), wherein **Ca,** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the peptide **P** in the presence of STT3A protein from *Trypanosoma brucei* to produce the compound of formula (**Ia**).

In a preferred embodiment of the inventive method for producing a glycoprotein of general formula (**Ia**), wherein **Ca, NH** and **o** have the meanings as defined herein, **P** represents a peptide of at least 20 amino acids comprising at least o-times a consensus sequence of N-V-T or N-Y-T,
the method comprises the steps:
Aa) providing a solution comprising a compound of formula (**IIIa**),
Ba) reacting the compound of formula (**IIIa**) with the peptide **P** in the presence of STT3A protein from *Trypanosoma brucei* to produce the compound of formula (**Ia**).

In a further embodiment of the inventive method for producing a glycoprotein of general formula (**Ia**), wherein **Ca, NH, o,** and **P** have the meanings as defined herein, the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**),
wherein m_{S1}, m_{S2}, and m_{S3} represent independently of each other an integer selected from 0, 1, 2, 3, 4 and 5.

Thus, in a further embodiment of the inventive method for producing a glycoprotein of general formula (**Ia**), wherein **Ca, NH, P** and **o** have the meanings as defined herein, the method comprises the steps:
Aa) providing a solution comprising a compound of formula (**IIIa**),
Ba) reacting the compound of formula (**IIIa**) with the peptide **P** in the presence of a eukaryotic oligosaccharyltransferase to produce the compound of formula (**Ia**),
and wherein m_{S1}, m_{S2}, and m_{S3} represent independently of each other an integer selected from 0, 1, 2, 3, 4 and 5.

Thus, in a further embodiment of the inventive method for producing a glycoprotein of general formula (**Ia**), wherein **Ca, NH, P** and o have the meanings as defined herein, the method comprises the steps:
Aa) providing a solution comprising a compound of formula (**IIIa**),
Ba) reacting the compound of formula (**IIIa**) with the peptide **P** in the presence of a bacterial oligosaccharyltransferase to produce the compound of formula (**Ia**),
and wherein m_{S1}, m_{S2}, and m_{S3} represent independently of each other an integer selected from 0, 1, 2, 3, 4 and 5.

Thus, in a further embodiment of the inventive method for producing a glycoprotein of general formula (**Ia**), wherein **Ca, NH, P** and **o** have the meanings as defined herein, the method comprises the steps:
Aa) providing a solution comprising a compound of formula (**IIIa**),
Ba) reacting the compound of formula (**IIIa**) with the peptide **P** in the presence of STT3A protein from *Trypanosoma brucei* to produce the compound of formula (**Ia**),
and wherein m_{S1}, m_{S2}, and m_{S3} represent independently of each other an integer selected from 0, 1, 2, 3, 4 and 5.

In a further embodiment of the inventive method for producing a glycoprotein of general formula (**Ia**), wherein **Ca, NH** and **o** have the meanings as defined herein, **P** represents a therapeutic protein of at least 10 amino acids comprising at least **o**-times a consensus sequence of N-X-S/T, wherein X represents any amino acid except proline,
the method comprises the steps:
Aa) providing a solution comprising a compound of formula (**IIIa**), Ba) reacting the compound of formula (**IIIa**) with the therapeutic protein **P** in the presence of an oligosaccharyltransferase enzyme to produce the compound of formula (**Ia**),
and wherein m_{S1}, m_{S2}, and m_{S3} represent independently of each other an integer selected from 0, 1, 2, 3, 4 and 5.

In a further embodiment of the inventive method for producing a glycoprotein of general formula (**Ia**), wherein **Ca, NH** and **o** have the meanings as defined herein, **P** represents an aglycosylated peptide of at least 10 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the aglycosylated peptide **P** in the presence of an oligosaccharyltransferase enzyme to produce the compound of formula (**Ia**),
and wherein m_{S1}, m_{S2}, and m_{S3} represent independently of each other an integer selected from 0, 1, 2, 3, 4 and 5.

In a further embodiment of the inventive method for producing a glycoprotein of general formula (**Ia**), wherein **Ca, NH** and **o** have the meanings as defined herein, **P** represents a peptide of at least 20 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**),
and wherein m_{S1}, m_{S2}, and m_{S3} represent independently of each other an integer selected from 0, 1, 2, 3, 4 and 5.

In a preferred embodiment of the inventive method for producing a glycoprotein of general formula (**Ia**), wherein **Ca,** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**),
and wherein m_{S1}, m_{S2}, and m_{S3} represent independently of each other an integer selected from 0, 1, 2, 3, 4 and 5.

In a preferred embodiment of the inventive method for producing a glycoprotein of general formula (**Ia**), wherein **Ca, NH** and **o** have the meanings as defined herein, **P** represents a peptide of at least 20 amino acids comprising at least o-times a consensus sequence of N-V-T or N-Y-T,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**),
and wherein m_{S1}, m_{S2}, and m_{S3} represent independently of each other an integer selected from 0, 1, 2, 3, 4 and 5.

In a further embodiment of the inventive method for producing a glycoprotein of general formula (**Ia**), wherein **Ca, NH, P** and **o** have the meanings as defined herein, the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**),
wherein **F¹**, **F²** and **G** represent -H

Thus, in a further embodiment of the inventive method for producing a glycoprotein of general formula (**Ia**), wherein **Ca, NH, P** and **o** have the meanings as defined herein, the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**),
wherein m_{S1}, m_{S2}, and m_{S3} represent independently of each other an integer selected from 0, 1, 2, 3, 4 and 5, and **F¹**, **F²** and **G** represent -H.

Thus, in a further embodiment of the inventive method for producing a glycoprotein of general formula (**Ia**), wherein **Ca, NH, P** and **o** have the meanings as defined herein, the method comprises the steps:
Aa) providing a solution comprising a compound of formula (**IIIa**),
Ba) reacting the compound of formula (**IIIa**) with the peptide **P** in the presence of a eukaryotic oligosaccharyltransferase to produce the compound of formula (**Ia**),
and wherein **F¹**, **F²** and **G** represent -H.

Thus, in a further embodiment of the inventive method for producing a glycoprotein of general formula (**Ia**), wherein **Ca, NH, P** and **o** have the meanings as defined herein, the method comprises the steps:
Aa) providing a solution comprising a compound of formula (**IIIa**),
Ba) reacting the compound of formula (**IIIa**) with the peptide **P** in the presence of a bacterial oligosaccharyltransferase to produce the compound of formula (**Ia**),
and wherein **F¹**, **F²** and **G** represent -H.

Thus, in a further embodiment of the inventive method for producing a glycoprotein of general formula (**Ia**), wherein **Ca, NH, P** and **o** have the meanings as defined herein, the method comprises the steps:
Aa) providing a solution comprising a compound of formula (**IIIa**),
Ba) reacting the compound of formula (**IIIa**) with the peptide **P** in the presence of STT3A protein from *Trypanosoma brucei* to produce the compound of formula (**Ia**),
and wherein **F¹**, **F²** and **G** represent -H.

In a further embodiment of the inventive method for producing a glycoprotein of general formula (**Ia**), wherein **Ca, NH** and **o** have the meanings as defined herein, **P** represents a therapeutic protein of at least 10 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the therapeutic protein **P** in the presence of an oligosaccharyltransferase enzyme to produce the compound of formula (**Ia**),
and **F¹**, **F²** and **G** represent -H.

In a further embodiment of the inventive method for producing a glycoprotein of general formula (**Ia**), wherein **Ca, NH** and **o** have the meanings as defined herein, **P** represents an aglycosylated peptide of at least 10 amino acids comprising at least **o**-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the aglycosylated peptide **P** in the presence of an oligosaccharyltransferase enzyme to produce the compound of formula (**Ia**),
and **F¹**, **F²** and **G** represent -H.

In a further embodiment of the inventive method for producing a glycoprotein of general formula (**Ia**), wherein **Ca, NH** and **o** have the meanings as defined herein, **P** represents a peptide of at least 20 amino acids comprising at least o-times a consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**),
and **F¹**, **F²** and **G** represent -H.

In a preferred embodiment of the inventive method for producing a glycoprotein of general formula (**Ia**), wherein **Ca,** and **NH** have the meanings as defined herein and **o** represents 1, **P** represents a peptide of at least 20 amino acids comprising at least one consensus sequence of N-X-S/T, wherein X represents any amino acid except proline,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**),
and **F¹**, **F²** and **G** represent -H.

In a preferred embodiment of the inventive method for producing a glycoprotein of general formula (**Ia**), wherein **Ca, NH** and **o** have the meanings as defined herein, **P** represents a peptide of at least 20 amino acids comprising at least o-times a consensus sequence of N-V-T or N-Y-T,
the method comprises the steps:
Aa) providing a solution comprising a compound of formula (**IIIa**),
Ba) reacting the compound of formula (**IIIa**) with the peptide **P** in the presence of an oligosaccharyltransferase enzyme to produce the compound of formula (**Ia**),
and **F¹**, **F²** and **G** represent -H.

### Complex-type Glycans

A further aspect of the present invention is directed to the enzymatic *in vitro* formation of lipid-linked complex-type glycans of general formula (**IIIb**).

Extension of the lipid-linked core structure LL-GlcNAc₂Man₃ is achieved by a set of different glycosylation reactions, which are performed *in vitro* and cell-free by using a set of recombinantly expressed glycosyltransferase enzymes, particularly *N*-acetyl-glucosaminyltransferases, glucosyltransferases, galactosyltransferases, fucosyl-transferases and sialyltransferases, and the corresponding nucleotide sugars, which act as glycosyl donors. Suitable nucleotide sugars are UDP-galactose, UDP-GlcNAc, UDP-GalNAc, GDP-fucose, CMP-NeuAc and CMP-NeuGc.

Thus, according to the invention, a method for producing a compound of general formula (**IIIb**) 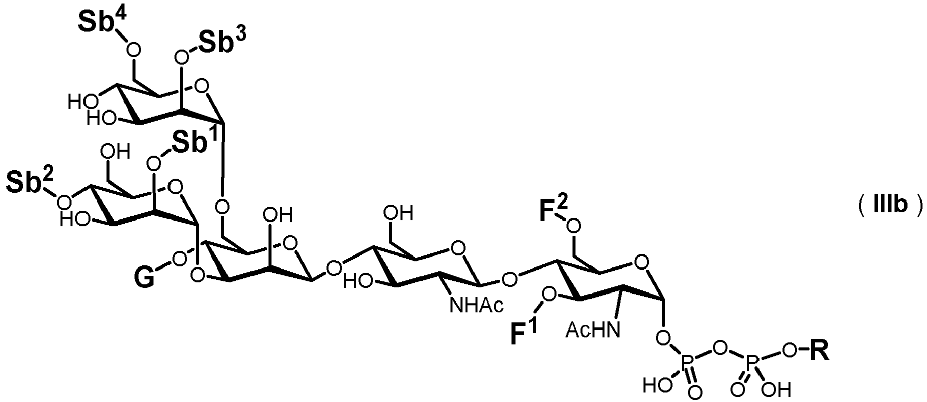 wherein
**F¹** and **F²** represent 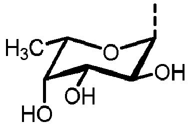 or -H; with the proviso that **F¹** and **F²** cannot be simultaneously 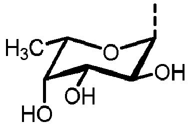
**G** represents 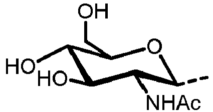 or -H;
**Sb¹** represents 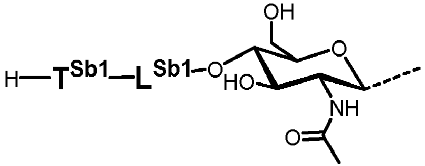
**Sb²** represents 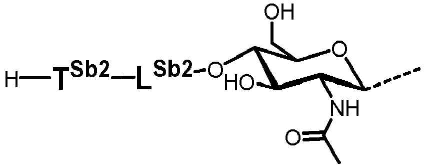 or -H
**Sb³** represents 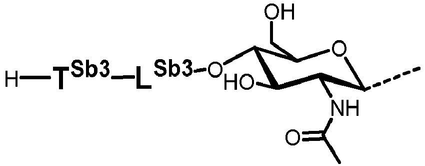
**Sb⁴** represents 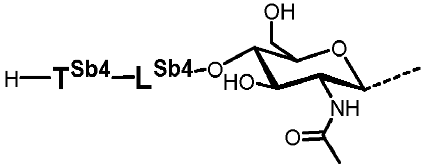 or -H
**L^{Sb1}** represents 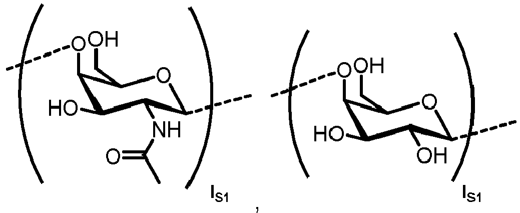 or 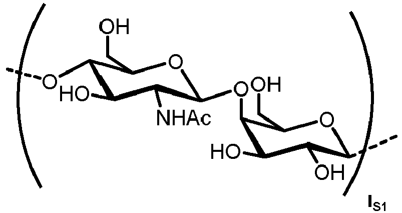
**L^{Sb2}** represents 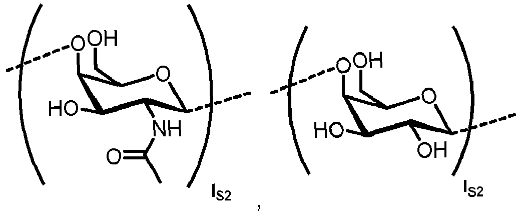 or 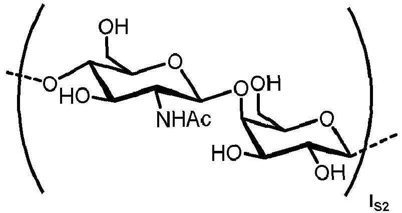
**L^{Sb3}** represents 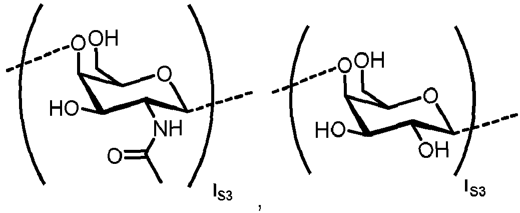 or 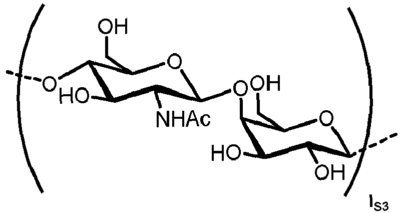
**L^{Sb4}** represents 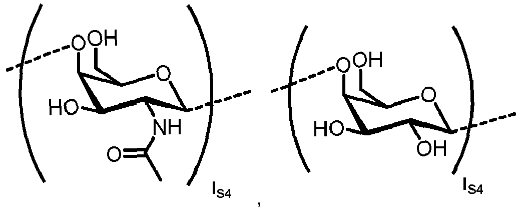 or 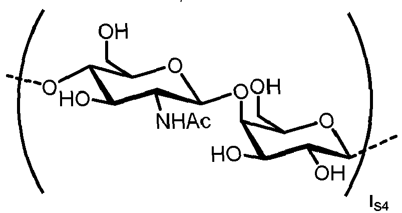
**T^{Sb1}**, **T^{Sb2}**, **T^{Sb3}** and **T^{Sb4}** represent independently of each other: 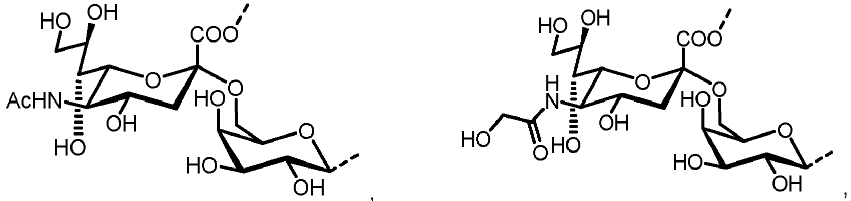 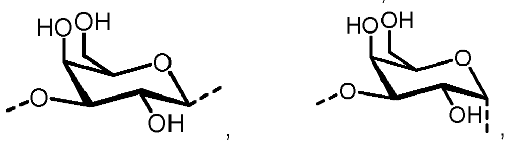 -SO₄- or a bond wherein I_{S1}, I_{S2}, I_{S3}, and I_{S4} represent independently of each other an integer selected from 0, 1, 2, 3, 4, 5, 6, 7, 8, 9, 10, 11, 12, 13, 14 and 15,
comprising the steps:
   Ab) providing a solution comprising a compound of formula (**IIb**) 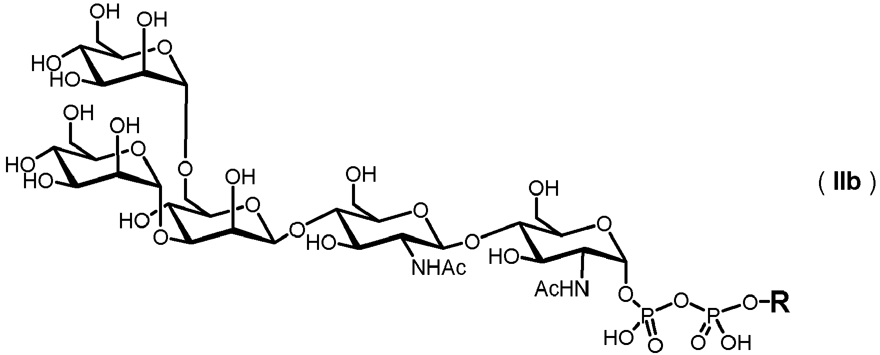 wherein R represents 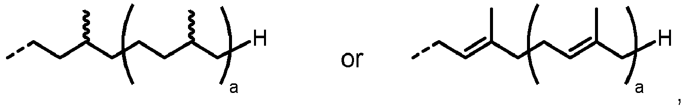 with a being an integer selected from 2, 3, 4, 5, 6, 7, 8, 9, 10, 11, 12, 13, 14 or 15,
   Bb) mixing the solution with at least one nucleotide sugar and at least one glycosyltransferase enzyme and reacting a resulting mixture to produce the compound of formula (**IIIb**), wherein the at least one nucleotide sugar is selected from UDP-galactose, UDP-GlcNAc, UDP-GalNAc, GDP-fucose, CMP-NeuAc and CMP-NeuGc, and wherein the at least one glycosyltransferase enzyme is selected from *N*-acetylglucosaminyltransferase, glucosyltransferase, galactosyltransferase, fucosyltransferase and sialyltransferase.

Preferably, the the at least one glycosyltransferase enzyme is a transmembrane domain-deleted enzyme.

Thus, in an embodiment of the method for producing a compound of general formula (**IIIb**), wherein **F¹**, **F²**, **G, Sb¹**, **Sb²**, **Sb³**, and **Sb⁴** have the meanings a defined herein, the method comprises the steps:
Ab) providing a solution comprising a compound of formula (**IIb**)
Bb) mixing the solution with at least one nucleotide sugar and at least one transmembrane domain-deleted glycosyltransferase enzyme and reacting a resulting mixture to produce the compound of formula (IIIb), wherein the at least one nucleotide sugar is selected from UDP-galactose, UDP-GlcNAc, UDP-GalNAc, GDP-fucose, CMP-NeuAc and CMP-NeuGc, and wherein the at least one transmembrane domain-deleted glycosyltransferase enzyme is selected from *N*-acetylglucosaminyltransferase, glucosyltransferase, galactosyltransferase, fucosyltransferase and sialyltransferase.

Preferably, the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

Thus, in an embodiment of the method for producing a compound of general formula (**IIIb**), wherein **F¹**, **F²**, **G, Sb¹**, **Sb²**, **Sb³**, and **Sb⁴** have the meanings a defined herein, the method comprises the steps:
Ab) providing a solution comprising a compound of formula (**IIb**)
Bb) mixing the solution with at least one nucleotide sugar and at least one glycosyltransferase enzyme and reacting a resulting mixture to produce the compound of formula (IIIb), wherein the at least one nucleotide sugar is selected from UDP-galactose, UDP-GlcNAc, UDP-GalNAc, GDP-fucose, CMP-NeuAc and CMP-NeuGc, and wherein the at least one glycosyltransferase enzyme is selected from *N*-acetylglucosaminyltransferase, glucosyltransferase, galactosyltransferase, fucosyltransferase and sialyltransferase,
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyl-transferase.

Preferably, **T^{Sb1}**, **T^{Sb2}**, **T^{Sb3}**, and **T^{Sb4}** represent a bond.

Thus, in an embodiment of the method for producing a compound of general formula (**IIIb**), wherein **F¹**, **F²**, G, **Sb¹**, **Sb²**, **Sb³**, and **Sb⁴** have the meanings a defined herein, the method comprises the steps:
Ab) providing a solution comprising a compound of formula (**IIb**)
Bb) mixing the solution with at least one nucleotide sugar and at least one glycosyltransferase enzyme and reacting a resulting mixture to produce the compound of formula (IIIb), wherein the at least one nucleotide sugar is selected from UDP-galactose, UDP-GlcNAc, UDP-GalNAc, GDP-fucose, CMP-NeuAc and CMP-NeuGc, and wherein the at least one glycosyltransferase enzyme is selected from *N*-acetylglucosaminyltransferase, glucosyltransferase, galactosyltransferase, fucosyltransferase and sialyltransferase,
wherein **T^{Sb1}**, **T^{Sb2}**, **T^{Sb3}**, and **T^{Sb4}** represent a bond

In a further embodiment of the method for producing a compound of general formula (**IIIb**), wherein **F¹**, **F²**, G, **Sb¹**, **Sb²**, **Sb³**, and **Sb⁴** have the meanings a defined herein, the method comprises the steps:
Ab) providing a solution comprising a compound of formula (**IIb**)
Bb) mixing the solution with at least one nucleotide sugar and at least one transmembrane domain-deleted glycosyltransferase enzyme and reacting a resulting mixture to produce the compound of formula (IIIb), wherein the at least one nucleotide sugar is selected from UDP-galactose, UDP-GlcNAc, UDP-GalNAc, GDP-fucose, CMP-NeuAc and CMP-NeuGc, and wherein the at least one transmembrane domain-deleted glycosyltransferase enzyme is selected from *N*-acetylglucosaminyltransferase, glucosyltransferase, galactosyltransferase, fucosyltransferase and sialyltransferase,
wherein **T^{Sb1}**, **T^{Sb2}**, **T^{Sb3}**, and **T^{Sb4}** represent a bond.

In a further embodiment of the method for producing a compound of general formula (**IIIb**), wherein **F¹**, **F²**, G, **Sb¹**, **Sb²**, **Sb³**, and **Sb⁴** have the meanings a defined herein, the method comprises the steps:
Ab) providing a solution comprising a compound of formula (**IIb**)
Bb) mixing the solution with at least one nucleotide sugar and at least one glycosyltransferase enzyme and reacting a resulting mixture to produce the compound of formula (IIIb), wherein the at least one nucleotide sugar is selected from UDP-galactose, UDP-GlcNAc, UDP-GalNAc, GDP-fucose, CMP-NeuAc and CMP-NeuGc, and wherein the at least one glycosyltransferase enzyme is selected from *N*-acetylglucosaminyltransferase, glucosyltransferase, galactosyltransferase, fucosyltransferase and sialyltransferase,
wherein **T^{Sb1}**, **T^{Sb2}**, **T^{Sb3}**, and **T^{Sb4}** represent a bond and
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyl-transferase.

Preferably, **F¹**, **F²** and **G** represent -H.

Thus, in an embodiment of the method for producing a compound of general formula (**IIIb**), wherein **Sb¹**, **Sb²**, **Sb³**, and **Sb⁴** have the meanings a defined herein,
the method comprises the steps:
Ab) providing a solution comprising a compound of formula (**IIb**)
Bb) mixing the solution with at least one nucleotide sugar and at least one glycosyltransferase enzyme and reacting a resulting mixture to produce the compound of formula (IIIb), wherein the at least one nucleotide sugar is selected from UDP-galactose, UDP-GlcNAc, UDP-GalNAc, GDP-fucose, CMP-NeuAc and CMP-NeuGc, and wherein the at least one glycosyltransferase enzyme is selected from *N*-acetylglucosaminyltransferase, glucosyltransferase, galactosyltransferase, fucosyltransferase and sialyltransferase,
wherein **F¹**, **F²** and **G** represent -H.

In a further embodiment of the method for producing a compound of general formula (**IIIb**), wherein **Sb¹**, **Sb²**, **Sb³**, and **Sb⁴** have the meanings a defined herein,
the method comprises the steps:
Ab) providing a solution comprising a compound of formula (**IIb**)
Bb) mixing the solution with at least one nucleotide sugar and at least one glycosyltransferase enzyme and reacting a resulting mixture to produce the compound of formula (IIIb), wherein the at least one nucleotide sugar is selected from UDP-galactose, UDP-GlcNAc, UDP-GalNAc, GDP-fucose, CMP-NeuAc and CMP-NeuGc, and wherein the at least one glycosyltransferase enzyme is selected from *N*-acetylglucosaminyltransferase, glucosyltransferase, galactosyltransferase, fucosyltransferase and sialyltransferase,
wherein **F¹**, **F²** and **G** represent -H and **T^{Sb1}**, **T^{Sb2}**, **T^{Sb3}**, and **T^{Sb4}** represent a bond.

In a further embodiment of the method for producing a compound of general formula (**IIIb**), wherein **Sb¹**, **Sb²**, **Sb³**, and **Sb⁴** have the meanings a defined herein,
the method comprises the steps:
Ab) providing a solution comprising a compound of formula (**IIb**)
Bb) mixing the solution with at least one nucleotide sugar and at least one transmembrane domain-deleted glycosyltransferase enzyme and reacting a resulting mixture to produce the compound of formula (IIIb), wherein the at least one nucleotide sugar is selected from UDP-galactose, UDP-GlcNAc, UDP-GalNAc, GDP-fucose, CMP-NeuAc and CMP-NeuGc, and wherein the at least one transmembrane domain-deleted glycosyltransferase enzyme is selected from *N*-acetylglucosaminyltransferase, glucosyltransferase, galactosyltransferase, fucosyltransferase and sialyltransferase,
wherein **T^{Sb1}**, **T^{Sb2}**, **T^{Sb3}**, and **T^{Sb4}** represent a bond, **F¹**, **F²** and **G** represent -H.

In a further embodiment of the method for producing a compound of general formula (**IIIb**), wherein **Sb¹**, **Sb²**, **Sb³**, and **Sb⁴** have the meanings a defined herein,
the method comprises the steps:
Ab) providing a solution comprising a compound of formula (**IIb**)
Bb) mixing the solution with at least one nucleotide sugar and at least one glycosyltransferase enzyme and reacting a resulting mixture to produce the compound of formula (IIIb), wherein the at least one nucleotide sugar is selected from UDP-galactose, UDP-GlcNAc, UDP-GalNAc, GDP-fucose, CMP-NeuAc and CMP-NeuGc, and wherein the at least one glycosyltransferase enzyme is selected from *N*-acetylglucosaminyltransferase, glucosyltransferase, galactosyltransferase, fucosyltransferase and sialyltransferase,
wherein **T^{Sb1}**, **T^{Sb2}**, **T^{Sb3}**, and **T^{Sb4}** represent a bond, **F¹**, **F²** and **G** represent -H, and wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyl-transferase.

The construction of the carbohydrate in step Bb) may be performed in one-pot or sequentially in multiple steps. Thus, in a further embodiment of the inventive method for producing a glycoprotein of general formula (**IIIa**), wherein **F¹**, **F²**, **G, Sb¹**, **Sb²**, **Sb³**, and **Sb⁴** have the meanings a defined herein,
the method comprises the steps:
Ab) providing a solution comprising a compound of formula (**IIb**)
Bb1) mixing the solution with a nucleotide sugar and a glycosyltransferase enzyme and reacting a resulting mixture
Bb2) repeating step Bb1) to produce the compound of formula (**IIIb**), wherein the nucleotide sugar is selected from UDP-galactose, UDP-GlcNAc, UDP-GalNAc, GDP-fucose, CMP-NeuAc and CMP-NeuGc, and wherein the glycosyltransferase enzyme is selected from *N*-acetylglucosaminyltransferase, glucosyltransferase, galactosyltransferase, fucosyltransferase and sialyltransferase.

In a further embodiment of the method for producing a compound of general formula (**IIIb**), wherein **F¹**, **F²**, G, **Sb¹**, **Sb²**, **Sb³**, and **Sb⁴** have the meanings a defined herein, the method comprises the steps:
Ab) providing a solution comprising a compound of formula (**IIb**)
Bb1) mixing the solution with a nucleotide sugar and a glycosyltransferase enzyme and reacting a resulting mixture
Bb2) repeating step Bb1) to produce the compound of formula (**IIIb**), wherein the nucleotide sugar is selected from UDP-galactose, UDP-GlcNAc, UDP-GalNAc, GDP-fucose, CMP-NeuAc and CMP-NeuGc, and wherein the glycosyltransferase enzyme is selected from *N*-acetylglucosaminyltransferase, glucosyltransferase, galactosyltransferase, fucosyltransferase and sialyltransferase,
wherein **T^{Sb1}**, **T^{Sb2}**, **T^{Sb3}**, and **T^{Sb4}** represent a bond.

In a further embodiment of the method for producing a compound of general formula (**IIIb**), wherein **Sb¹**, **Sb²**, **Sb³**, and **Sb⁴** have the meanings a defined herein,
the method comprises the steps:
Ab) providing a solution comprising a compound of formula (**IIb**)
Bb1) mixing the solution with a nucleotide sugar and a glycosyltransferase enzyme and reacting a resulting mixture
Bb2) repeating step Bb1) to produce the compound of formula (**IIIb**), wherein the nucleotide sugar is selected from UDP-galactose, UDP-GlcNAc, UDP-GalNAc, GDP-fucose, CMP-NeuAc and CMP-NeuGc, and wherein the glycosyltransferase enzyme is selected from *N*-acetylglucosaminyltransferase, glucosyltransferase, galactosyltransferase, fucosyltransferase and sialyltransferase,
wherein **F¹**, **F²** and **G** represent -H.

In a further embodiment of the method for producing a compound of general formula (**IIIb**), wherein **F¹**, **F²**, **G, Sb¹**, **Sb²**, **Sb³**, and **Sb⁴** have the meanings a defined herein, the method comprises the steps:
Ab) providing a solution comprising a compound of formula (**IIb**)
Bb1) mixing the solution with a nucleotide sugar and a transmembrane domain-deleted glycosyltransferase enzyme and reacting a resulting mixture
Bb2) repeating step Bb1) to produce the compound of formula (**IIIb**), wherein the nucleotide sugar is selected from UDP-galactose, UDP-GlcNAc, UDP-GalNAc, GDP-fucose, CMP-NeuAc and CMP-NeuGc, and wherein the transmembrane domain-deleted glycosyltransferase enzyme is selected from *N*-acetylglucosaminyltransferase, glucosyltransferase, galactosyltransferase, fucosyltransferase and sialyltransferase.

In a further embodiment of the method for producing a compound of general formula (**IIIb**), wherein **F¹**, **F²**, **G, Sb¹**, **Sb²**, **Sb³**, and **Sb⁴** have the meanings a defined herein, the method comprises the steps:
Ab) providing a solution comprising a compound of formula (**IIb**)
Bb1) mixing the solution with a nucleotide sugar and a transmembrane domain-deleted glycosyltransferase enzyme and reacting a resulting mixture
Bb2) repeating step Bb1) to produce the compound of formula (**IIIb**), wherein the nucleotide sugar is selected from UDP-galactose, UDP-GlcNAc, UDP-GalNAc, GDP-fucose, CMP-NeuAc and CMP-NeuGc, and wherein the transmembrane domain-deleted glycosyltransferase enzyme is selected from *N*-acetylglucosaminyltransferase, glucosyltransferase, galactosyltransferase, fucosyltransferase and sialyltransferase,
wherein **T^{Sb1}**, **T^{Sb2}**, **T^{Sb3}**, and **T^{Sb4}** represent a bond and.

In a further embodiment of the method for producing a compound of general formula (**IIIb**), wherein **F¹**, **F²**, **G, Sb¹**, **Sb²**, **Sb³**, and **Sb⁴** have the meanings a defined herein, the method comprises the steps:
Ab) providing a solution comprising a compound of formula (**IIb**)
Bb1) mixing the solution with a nucleotide sugar and a glycosyltransferase enzyme and reacting a resulting mixture
Bb2) repeating step Bb1) to produce the compound of formula (**IIIb**), wherein the nucleotide sugar is selected from UDP-galactose, UDP-GlcNAc, UDP-GalNAc, GDP-fucose, CMP-NeuAc and CMP-NeuGc, and wherein the glycosyltransferase enzyme is selected from *N*-acetylglucosaminyltransferase, glucosyltransferase, galactosyltransferase, fucosyltransferase and sialyltransferase,
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

In a further embodiment of the method for producing a compound of general formula (**IIIb**), wherein **F¹**, **F²**, **G, Sb¹**, **Sb²**, **Sb³**, and **Sb⁴** have the meanings a defined herein, the method comprises the steps:
Ab) providing a solution comprising a compound of formula (**IIb**)
Bb1) mixing the solution with a nucleotide sugar and a glycosyltransferase enzyme and reacting a resulting mixture
Bb2) repeating step Bb1) to produce the compound of formula (**IIIb**), wherein the nucleotide sugar is selected from UDP-galactose, UDP-GlcNAc, UDP-GalNAc, GDP-fucose, CMP-NeuAc and CMP-NeuGc, and wherein the glycosyltransferase enzyme is selected from *N*-acetylglucosaminyltransferase, glucosyltransferase, galactosyltransferase, fucosyltransferase and sialyltransferase,
wherein **T^{Sb1}**, **T^{Sb2}, T^{Sb3}**, and **T^{Sb4}** represent a bond and
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

In a further embodiment of the method for producing a compound of general formula (**IIIb**), wherein **Sb¹**, **Sb²**, **Sb³**, and **Sb⁴** have the meanings a defined herein,
the method comprises the steps:
Ab) providing a solution comprising a compound of formula (**IIb**)
Bb1) mixing the solution with a nucleotide sugar and a glycosyltransferase enzyme and reacting a resulting mixture
Bb2) repeating step Bb1) to produce the compound of formula (**IIIb**), wherein the nucleotide sugar is selected from UDP-galactose, UDP-GlcNAc, UDP-GaINAc, GDP-fucose, CMP-NeuAc and CMP-NeuGc, and wherein the glycosyltransferase enzyme is selected from *N*-acetylglucosaminyltransferase, glucosyltransferase, galactosyltransferase, fucosyltransferase and sialyltransferase,
wherein **F¹**, **F²** and **G** represent -H;
**T^{Sb1}, T^{Sb2}, T^{Sb3},** and **T^{Sb4}** represent independently of each other: 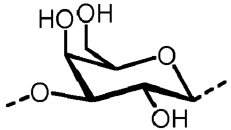 or a bond, and I_{S1}, I_{S2}, I_{S3}, and I_{S4} are 0.

### Complex-type N-Glycans

A further aspect of the present invention is directed to the enzymatic *in vitro* formation of lipid-linked complex-type glycans of general formula (**Ic**).

Extension of the peptide-linked core structure P-GlcNAc₂Man₃ is achieved by a set of different glycosylation reactions, which are performed *in vitro* and cell-free by using a set of recombinantly expressed glycosyltransferase enzymes, particularly *N*-acetyl-glucosaminyltransferases, glucosyltransferases, galactosyltransferases, fucosyltransferases and sialyltransferases, and the corresponding nucleotide sugars, which act as glycosyl donors. Suitable nucleotide sugars are UDP-galactose, UDP-GlcNAc, UDP-GaINAc, GDP-fucose, CMP-NeuAc and CMP-NeuGc.

Thus, a method for producing a compound of general formula (**Ic**)

**[C-NH]ₒP** **(Ic)**

wherein **C** represents a carbohydrate of the following structure 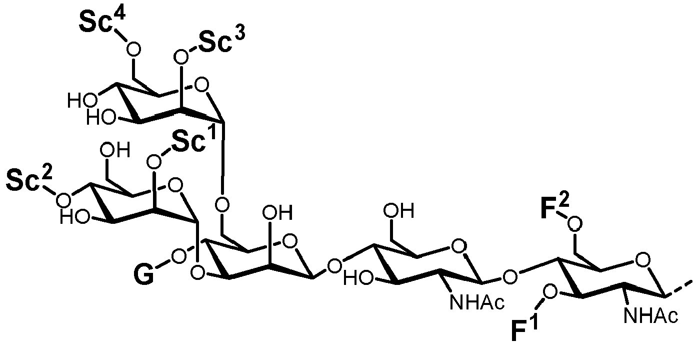
**o** is an integer representing the number of carbohydrates **C** which are bound to a peptide **P,**
**P** represents a peptide of at least 10 amino acids comprising at least o-times a consensus sequence of N-X-S/T, wherein X represents any amino acid except proline, and
**NH** represents an asparagine γ-amido group of the consensus sequence wherein
**F¹** and **F²** represent 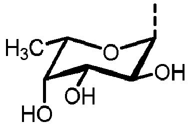 or -H; with the proviso that **F¹** and **F²** cannot be simultaneously 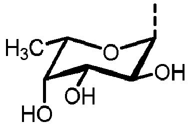
G represents 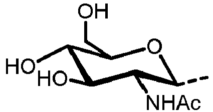 or -H;
**Sc¹** represents 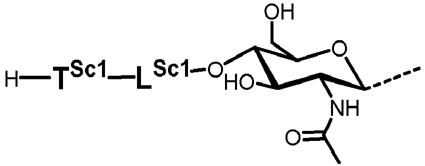
**Sc²** represents 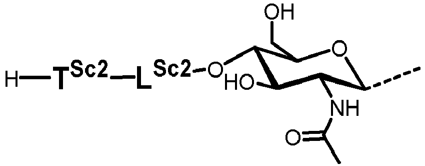 or -H
**Sc³** represents 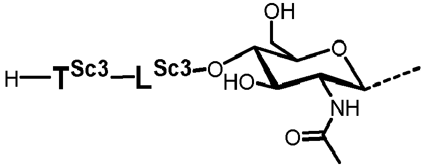
**Sc⁴** represents 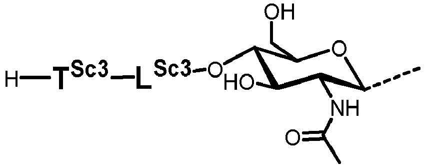 or -H
**Sc¹** represents 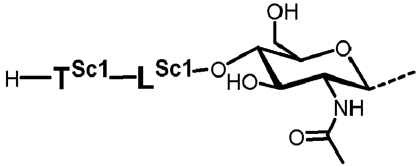
**Sc²** represents 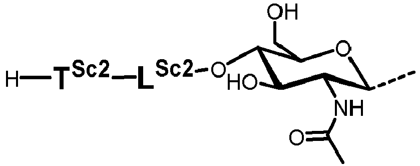 or -H
**Sc³** represents 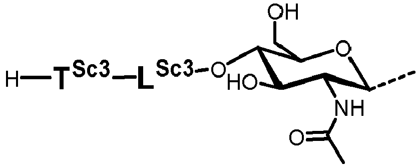
**Sc⁴** represents 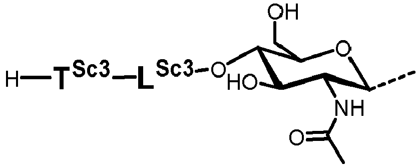 or -H
**L^{Sc1}** represents 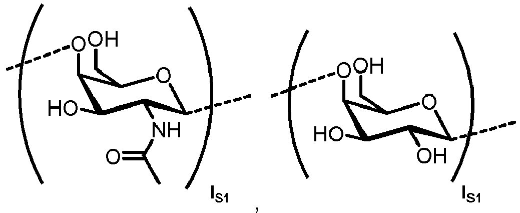 or 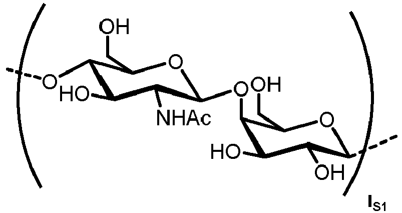
**L^{Sc2}** represents 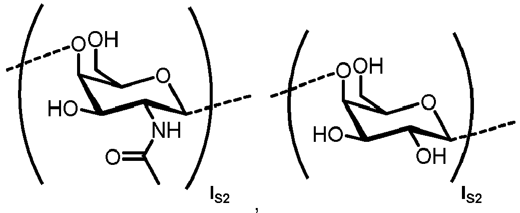 or 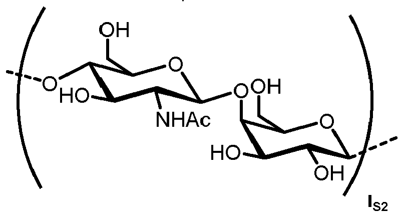
**L^{Sc3}** represents 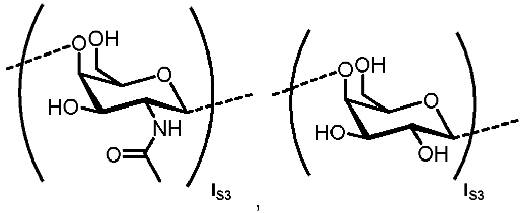 or 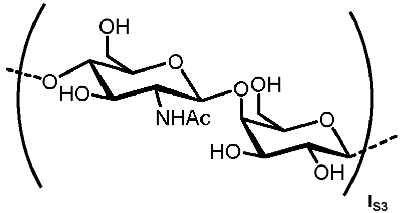
**L^{Sc4}** represents 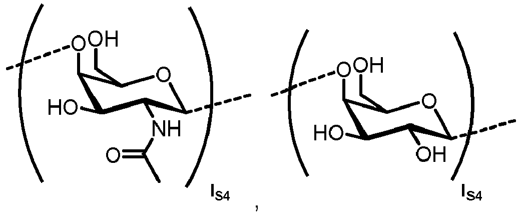 or 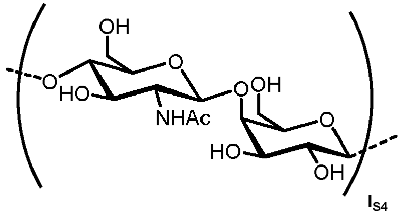
**T^{Sc1}**, **T^{Sc2}, T^{Sc3},** and **T^{Sc4}** represent independently of each other: 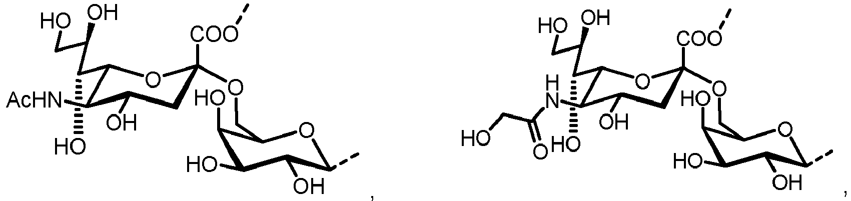 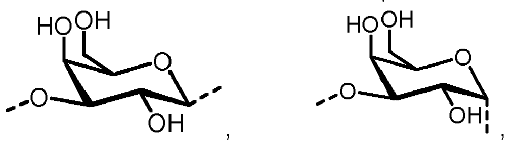 -SO₄- or a bond wherein I_{S1}, I_{S2}, I_{S3}, and I_{S4} represent independently of each other an integer selected from 0, 1, 2, 3, 4, 5, 6, 7, 8, 9, 10, 11, 12, 13, 14 and 15,
comprises the steps:
   Ac) providing a solution comprising a compound of formula (**IIc**) 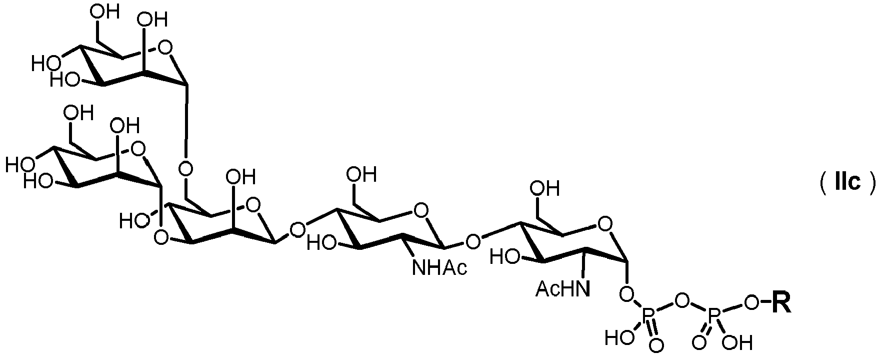 wherein **R** represents 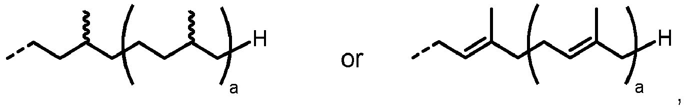 with a being an integer selected from 2, 3, 4, 5, 6, 7, 8, 9, 10, 11, 12, 13, 14 or 15,
   Bc) reacting the compound of formula (**IIc**) with the peptide **P** in the presence of an oligosaccharyltransferase enzyme to produce the compound of formula (**IVc)**

      **[Cc-NH]ₒP** **(IVc)**

      wherein **Cc** represents a carbohydrate of the following structure 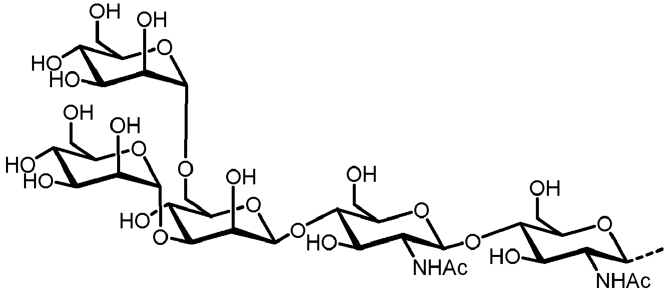
   Cc) mixing the compound of formula (**IVc**) with at least one nucleotide sugar and at least one glycosyltransferase enzyme and reacting a resulting mixture to produce the compound of formula (**Ic**), wherein the at least one nucleotide sugar is selected from GDP-mannose, UDP-galactose, UDP-GlcNAc, UDP-GaINAc, GDP-fucose, CMP-NeuAc and CMP-NeuGc, and wherein the at least one glycosyltransferase enzyme is selected from N-acetylglucosaminyltransferase, mannosyltransferase, glucosyltransferase, galactosyltransferase, fucosyltransferase and sialyltransferase.

Preferably, the the at least one glycosyltransferase enzyme is a transmembrane domain-deleted enzyme.

Thus, in an embodiment of the method for producing a compound of general formula (**Ic**), wherein **C, NH, P** and **o** have the meanings a defined herein,
the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**),
Cc) mixing the compound of formula (**IVc**) with at least one nucleotide sugar and at least one transmembrane domain-deleted glycosyltransferase enzyme and reacting a resulting mixture to produce the compound of formula (**Ic**), wherein the at least one nucleotide sugar is selected from GDP-mannose, UDP-galactose, UDP-GlcNAc, UDP-GaINAc, GDP-fucose, CMP-NeuAc and CMP-NeuGc, and wherein the at least one transmembrane domain-deleted glycosyltransferase enzyme is selected from *N*-acetylglucosaminyltransferase, mannosyltransferase, glucosyltransferase, galactosyltransferase, fucosyltransferase and sialyltransferase.

Preferably, the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

Thus, in an embodiment of the method for producing a compound of general formula (**Ic**), wherein **C, NH, P** and **o** have the meanings a defined herein,
the method comprises the steps:
Ac) providing a solution comprising a compound of formula (**IIc**)
Bc) reacting the compound of formula (**IIc**) with the peptide **P** in the presence of an oligosaccharyltransferase enzyme to produce the compound of formula (**IVc**)
Cc) mixing the compound of formula (**IVc**) with at least one nucleotide sugar and at least one glycosyltransferase enzyme and reacting a resulting mixture to produce the compound of formula (**Ic**), wherein the at least one nucleotide sugar is selected from GDP-mannose, UDP-galactose, UDP-GlcNAc, UDP-GaINAc, GDP-fucose, CMP-NeuAc and CMP-NeuGc, and wherein the at least one glycosyltransferase enzyme is selected from *N*-acetylglucosaminyltransferase, mannosyltransferase, glucosyltransferase, galactosyltransferase, fucosyltransferase and sialyltransferase,
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

Preferably, **T^{Sc1}** and **T^{Sc2}** represent a bond.

Thus, in an embodiment of the method for producing a compound of general formula (**Ic**), wherein **C, NH, P** and **o** have the meanings a defined herein, the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**),
Cc) mixing the compound of formula (**IVc**) with at least one nucleotide sugar and at least one glycosyltransferase enzyme and reacting a resulting mixture to produce the compound of formula (**Ic**), wherein the at least one nucleotide sugar is selected from GDP-mannose, UDP-galactose, UDP-GlcNAc, UDP-GaINAc, and GDP-fucose, and wherein the at least one glycosyltransferase enzyme is selected from *N*-acetylglucosaminyltransferase, mannosyltransferase, glucosyltransferase, galactosyltransferase, fucosyltransferase and sialyltransferase,
wherein **T^{Sc1}**, **T^{Sc2}**, **T^{Sc3}**, and **T^{Sc4}** represent a bond.

In a further embodiment of the method for producing a compound of general formula (**Ic**), wherein **C, NH, P** and **o** have the meanings a defined herein, the method comprises the steps:
Ac) providing a solution comprising a compound of formula (**IIc**)
Bc) reacting the compound of formula (**IIc**) with the peptide **P** in the presence of an oligosaccharyltransferase enzyme to produce the compound of formula (**IVc**)
Cc) mixing the compound of formula (**IVc**) with at least one nucleotide sugar and at least one transmembrane domain-deleted glycosyltransferase enzyme and reacting a resulting mixture to produce the compound of formula (**Ic**), wherein the at least one nucleotide sugar is selected from GDP-mannose, UDP-galactose, UDP-GlcNAc, UDP-GaINAc, and GDP-fucose, and wherein the at least one transmembrane domain-deleted glycosyltransferase enzyme is selected from *N*-acetylglucosaminyltransferase, mannosyltransferase, glucosyltransferase, galactosyltransferase, fucosyltransferase and sialyltransferase,
wherein **T^{Sc1}**, **T^{Sc2}**, **T^{Sc3}**, and **T^{Sc4}** represent a bond.

In a further embodiment of the method for producing a compound of general formula (**Ic**), wherein **C, NH, P** and **o** have the meanings a defined herein, the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**),
Cc) mixing the compound of formula (**IVc**) with at least one nucleotide sugar and at least one glycosyltransferase enzyme and reacting a resulting mixture to produce the compound of formula (**Ic**), wherein the at least one nucleotide sugar is selected from GDP-mannose, UDP-galactose, UDP-GlcNAc, UDP-GaINAc, and GDP-fucose, and wherein the at least one glycosyltransferase enzyme is selected from *N*-acetylglucosaminyltransferase, mannosyltransferase, glucosyltransferase, galactosyltransferase, fucosyltransferase and sialyltransferase,
wherein **T^{Sc1}**, **T^{Sc2}**, **T^{Sc3}**, and **T^{Sc4}** represent a bond and
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

Preferably, **F¹**, **F²** and **G** represent -H.

Thus, in a further embodiment of the method for producing a compound of general formula (**Ic**), wherein **C, NH, P** and **o** have the meanings a defined herein, the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**),
Cc) mixing the compound of formula (**IVc**) with at least one nucleotide sugar and at least one glycosyltransferase enzyme and reacting a resulting mixture to produce the compound of formula (**Ic**), wherein the at least one nucleotide sugar is selected from GDP-mannose, UDP-galactose, UDP-GlcNAc, UDP-GaINAc, CMP-NeuAc and CMP-NeuGc, and wherein the at least one glycosyltransferase enzyme is selected from *N*-acetylglucosaminyltransferase, mannosyltransferase, glucosyltransferase, galactosyltransferase, fucosyltransferase and sialyltransferase,
wherein **F¹**, **F²** and **G** represent -H.

In a further embodiment of the method for producing a compound of general formula (**Ic**), wherein **C, NH, P** and **o** have the meanings a defined herein, the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**),
Cc) mixing the compound of formula (**IVc**) with at least one nucleotide sugar and at least one glycosyltransferase enzyme and reacting a resulting mixture to produce the compound of formula (**Ic**), wherein the at least one nucleotide sugar is selected from GDP-mannose, UDP-galactose, UDP-GlcNAc, and wherein the at least one glycosyltransferase enzyme is selected from *N*-acetylglucosaminyltransferase, mannosyltransferase, glucosyltransferase, galactosyltransferase, fucosyltransferase and sialyltransferase,
wherein **F¹**, **F²** and **G** represent -H and **T^{Sc1}**, **T^{Sc2}**, **T^{Sc3}**, and **T^{Sc4}** represent a bond.

In a further embodiment of the method for producing a compound of general formula (**Ic**), wherein **C, NH, P** and **o** have the meanings a defined herein, the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**),
Cc) mixing the compound of formula (**IVc**) with at least one nucleotide sugar and at least one transmembrane domain-deleted glycosyltransferase enzyme and reacting a resulting mixture to produce the compound of formula (**Ic**), wherein the at least one nucleotide sugar is selected from GDP-mannose, UDP-galactose, UDP-GlcNAc, and wherein the at least one transmembrane domain-deleted glycosyltransferase enzyme is selected from *N*-acetylglucosaminyltransferase, mannosyltransferase, glucosyltransferase, galactosyltransferase, fucosyltransferase and sialyltransferase,
wherein **F¹**, **F²** and **G** represent -H and **T^{Sc1}**, **T^{Sc2}**, **T^{Sc3}**, and **T^{Sc4}** represent a bond.

In a further embodiment of the method for producing a compound of general formula (**Ic**), wherein **C, NH, P** and **o** have the meanings a defined herein, the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**),
Cc) mixing the compound of formula (**IVc**) with at least one nucleotide sugar and at least one glycosyltransferase enzyme and reacting a resulting mixture to produce the compound of formula (**Ic**), wherein the at least one nucleotide sugar is selected from GDP-mannose, UDP-galactose, UDP-GlcNAc, and wherein the at least one glycosyltransferase enzyme is selected from *N*-acetylglucosaminyltransferase, mannosyltransferase, glucosyltransferase, galactosyltransferase, fucosyltransferase and sialyltransferase,
wherein **F¹**, **F²** and **G** represent -H and **T^{Sc1}**, **T^{Sc2}**, **T^{Sc3}**, and **T^{Sc4}** represent a bond, and
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

The construction of the carbohydrate in step Cc) may be performed in one-pot or sequentially in multiple steps. Thus, in a further embodiment of the inventive method for producing a glycoprotein of general formula (**Ic**), wherein **C, NH, P** and **o** have the meanings a defined herein, the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**)
Cc1) mixing the compound of formula (**IVc**) with a nucleotide sugar and a glycosyltransferase enzyme and reacting a resulting mixture,
Cc2) repeating step Cc1) to produce the compound of formula (**Ic**), wherein the nucleotide sugar is selected from GDP-mannose, UDP-galactose, UDP-GlcNAc, GDP-fucose, CMP-NeuAc and CMP-NeuGc, and wherein the glycosyltransferase enzyme is selected from *N*-acetylglucosaminyltransferase, mannosyltransferase, glucosyltransferase, galactosyltransferase, fucosyltransferase and sialyltransferase.

In a further embodiment of the method for producing a glycoprotein of general formula (**Ic**), wherein **C, NH, P** and **o** have the meanings a defined herein, the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**)
Cc1) mixing the compound of formula (**IVc**) with a nucleotide sugar and a glycosyltransferase enzyme and reacting a resulting mixture,
Cc2) repeating step Cc1) to produce the compound of formula (**Ic**), wherein the nucleotide sugar is selected from GDP-mannose, UDP-galactose, UDP-GlcNAc, GDP-fucose, and wherein the glycosyltransferase enzyme is selected from *N*-acetylglucosaminyltransferase, mannosyltransferase, glucosyltransferase, galactosyltransferase, fucosyltransferase and sialyltransferase,
wherein **T^{Sc1}**, **T^{Sc2}**, **T^{Sc3}**, and **T^{Sc4}** represent a bond.

In a further embodiment of the method for producing a glycoprotein of general formula (**Ic**), wherein **C, NH, P** and **o** have the meanings a defined herein, the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**)
Cc1) mixing the compound of formula (**IVc**) with a nucleotide sugar and a glycosyltransferase enzyme and reacting a resulting mixture,
Cc2) repeating step Cc1) to produce the compound of formula (**Ic**), wherein the nucleotide sugar is selected from GDP-mannose, UDP-galactose, UDP-GlcNAc, CMP-NeuAc and CMP-NeuGc, and wherein the glycosyltransferase enzyme is selected from *N*-acetylglucosaminyltransferase, mannosyltransferase, glucosyltransferase, galactosyltransferase, fucosyltransferase and sialyltransferase,
wherein **F¹**, **F²** and **G** represent -H.

In a further embodiment of the inventive method for producing a glycoprotein of general formula (**Ic**), wherein **C, NH, P** and **o** have the meanings a defined herein, the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**)
Cc1) mixing the compound of formula (**IVc**) with a nucleotide sugar and a transmembrane domain-deleted glycosyltransferase enzyme and reacting a resulting mixture,
Cc2) repeating step Cc1) to produce the compound of formula (**Ic**), wherein the nucleotide sugar is selected from GDP-mannose, UDP-galactose, UDP-GlcNAc, GDP-fucose, CMP-NeuAc and CMP-NeuGc, and wherein the transmembrane domain-deleted glycosyltransferase enzyme is selected from *N*-acetylglucosaminyltransferase, mannosyltransferase, glucosyltransferase, galactosyltransferase, fucosyltransferase and sialyltransferase.

In a further embodiment of the method for producing a glycoprotein of general formula (**Ic**), wherein **C, NH, P** and **o** have the meanings a defined herein, the method comprises the steps:
Ac) providing a solution comprising a compound of formula (**IIc**)
Bc) reacting the compound of formula (**IIc**) with the peptide **P** in the presence of an oligosaccharyltransferase enzyme to produce the compound of formula (**IVc**)
Cc1) mixing the compound of formula (**IVc**) with a nucleotide sugar and a transmembrane domain-deleted glycosyltransferase enzyme and reacting a resulting mixture,
Cc2) repeating step Cc1) to produce the compound of formula (**Ic**), wherein the nucleotide sugar is selected from GDP-mannose, UDP-galactose, UDP-GlcNAc, GDP-fucose, and wherein the transmembrane domain-deleted glycosyltransferase enzyme is selected from *N*-acetylglucosaminyltransferase, mannosyltransferase, glucosyltransferase, galactosyltransferase, fucosyltransferase and sialyltransferase,
wherein **T^{Sc1}**, **T^{Sc2}**, **T^{Sc3}**, and **T^{Sc4}** represent a bond.

In a further embodiment of the inventive method for producing a glycoprotein of general formula (**Ic**), wherein **C, NH, P** and **o** have the meanings a defined herein, the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**)
Cc1) mixing the compound of formula (**IVc**) with a nucleotide sugar and a glycosyltransferase enzyme and reacting a resulting mixture,
Cc2) repeating step Cc1) to produce the compound of formula (**Ic**), wherein the nucleotide sugar is selected from GDP-mannose, UDP-galactose, UDP-GlcNAc, GDP-fucose, CMP-NeuAc and CMP-NeuGc, and wherein the glycosyltransferase enzyme is selected from *N*-acetylglucosaminyltransferase, mannosyltransferase, glucosyltransferase, galactosyltransferase, fucosyltransferase and sialyltransferase,
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

In a further embodiment of the inventive method for producing a glycoprotein of general formula (**Ic**), wherein **C, P** and **o** have the meanings a defined herein, the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**)
Cc1) mixing the compound of formula (**IVc**) with a nucleotide sugar and a glycosyltransferase enzyme and reacting a resulting mixture,
Cc2) repeating step Cc1) to produce the compound of formula (**Ic**), wherein the nucleotide sugar is selected from GDP-mannose, UDP-galactose, UDP-GlcNAc, GDP-fucose, CMP-NeuAc and CMP-NeuGc, and wherein the glycosyltransferase enzyme is selected from *N*-acetylglucosaminyltransferase, mannosyltransferase, glucosyltransferase, galactosyltransferase, fucosyltransferase and sialyltransferase,
wherein **T^{Sc1}**, **T^{Sc2}**, **T^{Sc3}**, and **T^{Sc4}** represent a bond and
wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosyltransferase.

In a further embodiment of the inventive method for producing a glycoprotein of general formula (**Ic**), wherein **C, P** and **o** have the meanings a defined herein, the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**)
Cc1) mixing the compound of formula (**IVc**) with a nucleotide sugar and a glycosyltransferase enzyme and reacting a resulting mixture,
Cc2) repeating step Cc1) to produce the compound of formula (**Ic**), wherein the nucleotide sugar is selected from GDP-mannose, UDP-galactose, and UDP-GlcNAc, and wherein the glycosyltransferase enzyme is selected from *N*-acetylglucosaminyltransferase, mannosyltransferase, glucosyltransferase, galactosyltransferase, fucosyltransferase and sialyltransferase
wherein **F¹**, **F²** and **G** represent -H;
**T^{Sc1} , T^{Sc2}, T^{Sc3},** and **T^{Sc4}** represent independently of each other: 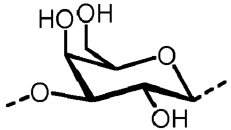 or a bond, and I_{S1}, I_{S2}, I_{S3}, and I_{S4} are 0.

The peptide **P** can be any type of a polypeptide having at least 10 amino acids and comprising at least one consensus sequence of N-X-S/T, wherein X represents any amino acid except proline, including proteins, folded peptides, folded proteins, therapeutic peptides, therapeutic proteins, aglycosylated peptides or aglycosylated proteins.

The oligosaccharyltransferase enzyme used in the inventive methods attaches the carbohydrates **C** or **Cc** to an asparagine γ-amido group of the consensus sequence of N-X-S/T, wherein X represents any amino acid except proline. Thus, the peptide P comprises at least one consensus sequence of N-X-S/T, wherein X represents any amino acid except proline. If the glycoprotein of formula (**Ic**) comprises **o** carbohydrate moieties **C,** the peptide **P** comprises at least o-times the consensus sequence of N-X-S/T, wherein X represents any amino acid except proline.

The number **o** of carbohydrates **C** which are bound to a peptide **P** may depend on the length of the peptide **P** itself. For instance, for shorter peptides consisting of 10 to 20 amino acids **o** may be 1, thus only one carbohydrate is bound to the peptide, whereas for peptides consisting of 20 to 50 amino acids **o** may be 1 or 2, thus one or two carbohydrates are bound to the peptide, etc.

Thus, in one embodiment of the inventive method for producing a glycoprotein of general formula (**Ic**), **o** is an integer as defined as follows:

| | |
|---|---|
| o is between 1 and 10 | if **P** consists of 10 to 50 amino acids |
| o is between 1 and 20 | if **P** consists of 51 to 100 amino acids |
| o is between 1 and 40 | if **P** consists of 101 to 200 amino acids |
| o is between 1 and 60 | if **P** consists of 201 to 300 amino acids |
| o is between 1 and 80 | if **P** consists of 301 to 400 amino acids |
| o is between 1 and 100 | if **P** consists of 401 to 500 amino acids, |

the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**),
Cc) mixing the compound of formula (**IVc**) with at least one nucleotide sugar and at least one glycosyltransferase enzyme and reacting a resulting mixture to produce the compound of formula (**Ic**), wherein the at least one nucleotide sugar is selected from GDP-mannose, UDP-galactose, UDP-GlcNAc, UDP-GaINAc, GDP-fucose, CMP-NeuAc and CMP-NeuGc, and wherein the at least one glycosyltransferase enzyme is selected from *N*-acetylglucosaminyltransferase, mannosyltransferase, glucosyltransferase, galactosyltransferase, fucosyltransferase and sialyltransferase
wherein wherein **C, NH,** and **P** have the meanings a defined herein.

In a preferred embodiment, **o** is an integer selected from:

| | |
|---|---|
| o is 1 | if **P** consists of 10 to 50 amino acids |
| o is 1 or 2 | if **P** consists of 51 to 100 amino acids |
| o is 1, 2 or 3 | if **P** consists of 101 to 200 amino acids |
| o is 2, 3 or 4 | if **P** consists of 201 to 300 amino acids |
| o is 2, 3, 4 or 5 | if **P** consists of 301 to 400 amino acids |
| o is 2, 3, 4, 5 or 6 | if **P** consists of 401 to 500 amino acids, |

the method comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**),
Cc) mixing the compound of formula (**IVc**) with at least one nucleotide sugar and at least one glycosyltransferase enzyme and reacting a resulting mixture to produce the compound of formula (**Ic**), wherein the at least one nucleotide sugar is selected from GDP-mannose, UDP-galactose, UDP-GlcNAc, UDP-GaINAc, GDP-fucose, CMP-NeuAc and CMP-NeuGc, and wherein the at least one glycosyltransferase enzyme is selected from *N*-acetylglucosaminyltransferase, mannosyltransferase, glucosyltransferase, galactosyltransferase, fucosyltransferase and sialyltransferase
wherein wherein **C, NH,** and **P** have the meanings a defined herein.

In a preferred embodiment, **o** is 1. Thus, the inventive method for producing a glycoprotein of general formula (**Ic**), comprises the steps:
Ac) providing a solution comprising a compound of formula (**IIc**),
Bc) reacting the compound of formula (**IIc**) with the peptide **P** in the presence of an oligosaccharyltransferase enzyme to produce the compound of formula (**IVc**),
Cc) mixing the compound of formula (**IVc)** with at least one nucleotide sugar and at least one glycosyltransferase enzyme and reacting a resulting mixture to produce the compound of formula (**Ic**), wherein the at least one nucleotide sugar is selected from GDP-mannose, UDP-galactose, UDP-GlcNAc, UDP-GaINAc, GDP-fucose, CMP-NeuAc and CMP-NeuGc, and wherein the at least one glycosyltransferase enzyme is selected from *N*-acetylglucosaminyltransferase, mannosyltransferase, glucosyltransferase, galactosyltransferase, fucosyltransferase and sialyltransferase
wherein wherein **C, NH,** and **P** have the meanings a defined herein and **o** represents 1.

In a preferred embodiment, the oligosaccharyltransferase enzyme is a eukaryotic oligosaccharyltransferase. In a more preferred embodiment, the oligosaccharyltransferase is a STT3A protein from *Trypanosoma brucei.* In contrast to oligosaccharyltransferases from higher eukaryotes, which are only present in two catalytically active isoforms STT3, the single-subunit OST of *Trypanosoma brucei* recognizes also human-like glycans and is thus more efficient for transferring glycans to a polypeptide.

Thus, in a further embodiment of the inventive method for producing a glycoprotein of general formula (**Ic**), wherein **C, NH, P** and **o** have the meanings as defined herein, the method comprises the steps:
Ac) providing a solution comprising a compound of formula (**IIc**),
Bc) reacting the compound of formula (**IIc**) with the peptide **P** in the presence of a eukaryotic oligosaccharyltransferase enzyme to produce the compound of formula (**IVc**),
Cc) mixing the compound of formula (**IVc)** with at least one nucleotide sugar and at least one glycosyltransferase enzyme and reacting a resulting mixture to produce the compound of formula (**Ic**), wherein the at least one nucleotide sugar is selected from GDP-mannose, UDP-galactose, UDP-GlcNAc, UDP-GaINAc, GDP-fucose, CMP-NeuAc and CMP-NeuGc, and wherein the at least one glycosyltransferase enzyme is selected from *N*-acetylglucosaminyltransferase, mannosyltransferase, glucosyltransferase, galactosyltransferase, fucosyltransferase and sialyltransferase.

In a further embodiment of the inventive method for producing a glycoprotein of general formula (**Ic**), wherein **C, NH, P** and **o** have the meanings as defined herein, the method comprises the steps:
Ac) providing a solution comprising a compound of formula (**IIc**),
Bc) reacting the compound of formula (**IIc**) with the peptide **P** in the presence of a bacterial oligosaccharyltransferase enzyme to produce the compound of formula (**IVc**),
Cc) mixing the compound of formula (**IVc**) with at least one nucleotide sugar and at least one glycosyltransferase enzyme and reacting a resulting mixture to produce the compound of formula (**Ic**), wherein the at least one nucleotide sugar is selected from GDP-mannose, UDP-galactose, UDP-GlcNAc, UDP-GaINAc, GDP-fucose, CMP-NeuAc and CMP-NeuGc, and wherein the at least one glycosyltransferase enzyme is selected from *N*-acetylglucosaminyltransferase, mannosyltransferase, glucosyltransferase, galactosyltransferase, fucosyltransferase and sialyltransferase.

In a further embodiment of the inventive method for producing a glycoprotein of general formula (**Ic**), wherein **C, NH, P** and **o** have the meanings as defined herein, the method comprises the steps:
Ac) providing a solution comprising a compound of formula (**IIc**),
Bc) reacting the compound of formula (**IIc**) with the peptide **P** in the presence of STT3A protein from *Trypanosoma brucei* to produce the compound of formula (**IVc**),
Cc) mixing the compound of formula (**IVc)** with at least one nucleotide sugar and at least one glycosyltransferase enzyme and reacting a resulting mixture to produce the compound of formula (**Ic**), wherein the at least one nucleotide sugar is selected from GDP-mannose, UDP-galactose, UDP-GlcNAc, UDP-GaINAc, GDP-fucose, CMP-NeuAc and CMP-NeuGc, and wherein the at least one glycosyltransferase enzyme is selected from *N*-acetylglucosaminyltransferase, mannosyltransferase, glucosyltransferase, galactosyltransferase, fucosyltransferase and sialyltransferase.

### Description of the Figures

**Figure 1****:** shows the general structure of the three types of *N*-glycans: high mannose, complex-type and hybrid-type.

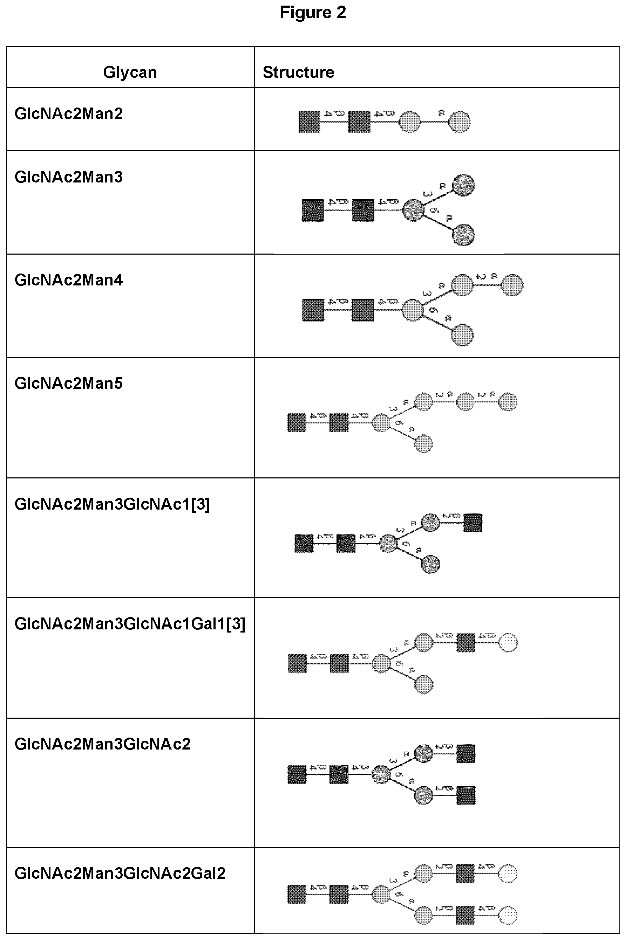

**Figure 2****:** shows exemplarily the structure of glycans prepared with the inventive methods

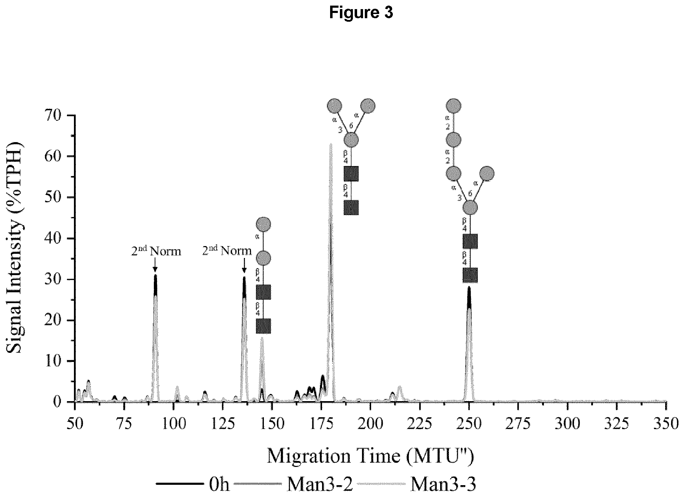

**Figure 3****:** shows a xCGE-LIF electropherogram of LL-GlcNAc₂Man₃ synthesis. The reaction was conducted in triplicates (Man3-1/2/3) for 8 h in a reaction batch initially, containing 50 mM MOPS (pH 6.8), 0.1% IGEPAL, 10 mM MgCl₂ (buffer B), 1 mM DTT, 0.1 mM phytanyl-PP-chitobiose, 2 mM GDP-mannose, 0.1 mg/mL purified ALG1ΔTM and 35% (v/v) ALG2 yeast membrane fraction. Samples were prepared for CGE-LIF measurements by mild acidic hydrolysis, APTS labeling followed by HILIC purification. Internal LIZ and 2^{nd} NormMix standards were used for analysis using glyXtool^{CE}. *N*-glycans are depicted according to the SNFG-nomenclature *(*Glycobiology 2015, 25, 1323-1324).

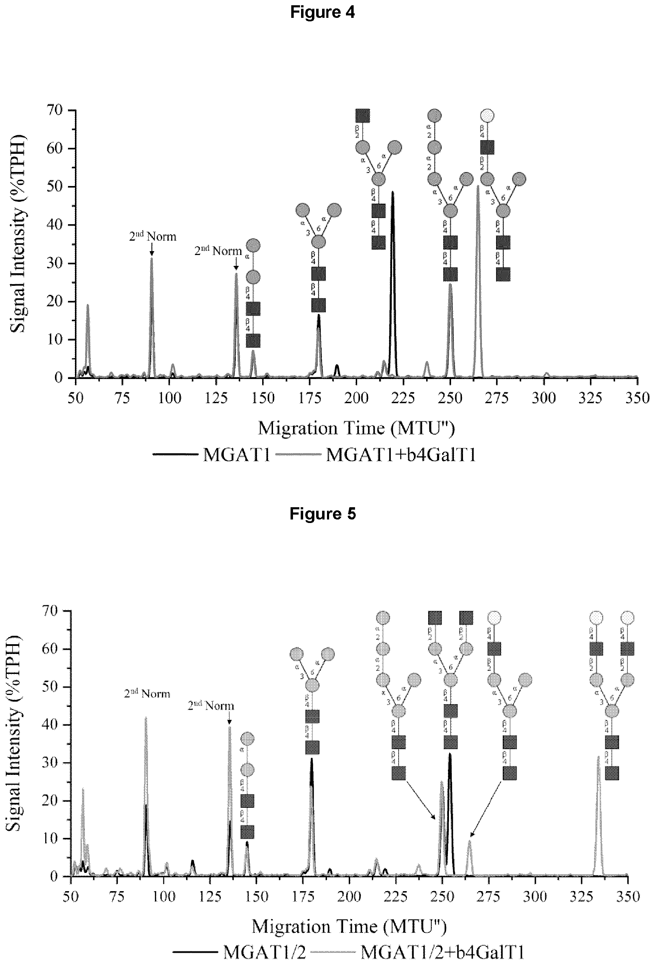

**Figure 4****:** shows a xCGE-LIF electropherogram showing the synthesis of LL-GlcNAc₂Man₃GlcNAc[3] with MGAT1 (red) and LL-GlcNAc₂Man₃GlcNAcGal[3] with MGAT1 + β4GaIT1 (blue). Internal LIZ and 2nd NormMix standards were used for analysis using glyXtoolCE. *N*-glycans are depicted according to the SNFG-nomenclature *(*Glycobiology 2015, 25, 1323-1324).
**Figure 5****:** shows a xCGE-LIF electropherogram showing the synthesis of LL-GlcNAc₂Man₃GlcNAc₂ with MGAT1+2 (red) and LL-GlcNAc₂Man₃GlcNAc₂Gal₂ with MGAT1+2 and β4GaIT1 (blue). Internal LIZ and 2nd NormMix standards were used for analysis using glyXtoolCE. *N*-glycans are depicted according to the SNFG-nomenclature *(*Glycobiology 2015, 25, 1323-1324).

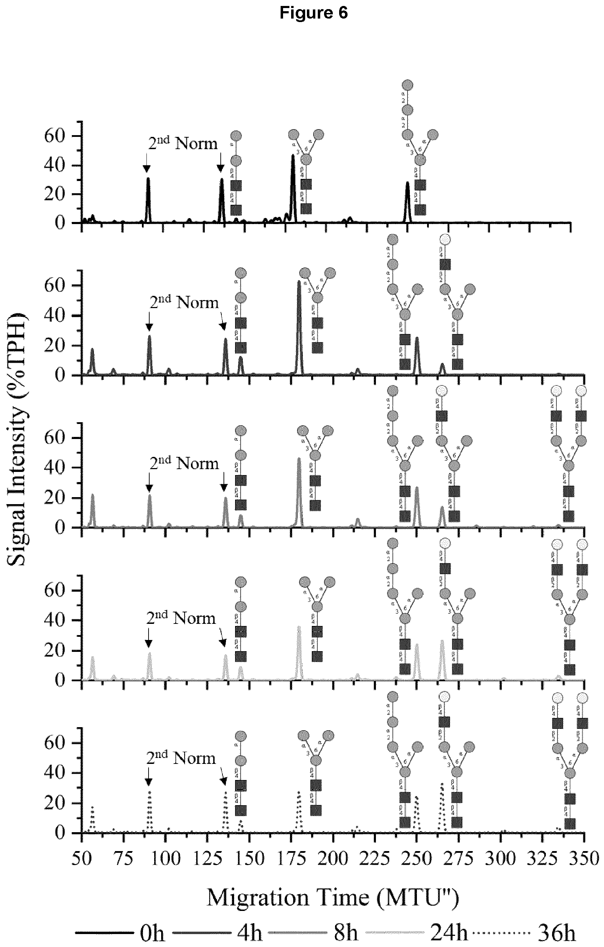

**Figure 6****:** shows a xCGE-LIF electropherogram showing the one-pot synthesis of LL-GlcNAc₂Man₃GlcNAc₂Gal₂ at 0 h (black), 4 h (red), 8 h (blue),24 h (orange) and 36 h (violet). Signal intensity was plotted against the migration time. Internal LIZ and 2nd NormMix standards were used for analysis using glyXtoolCE. *N*-glycans are depicted according to the SNFG-nomenclature *(*Glycobiology 2015, 25, 1323-1324).

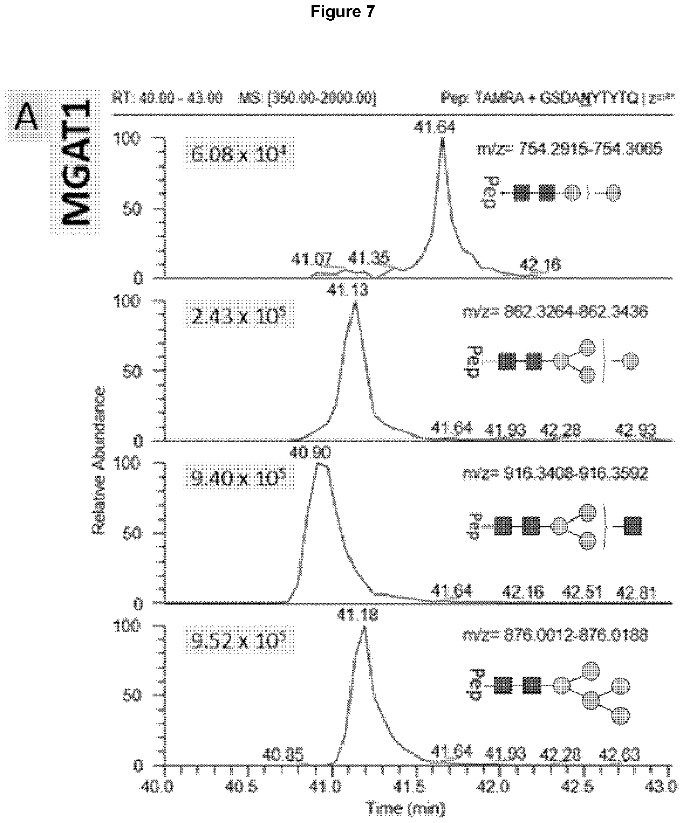

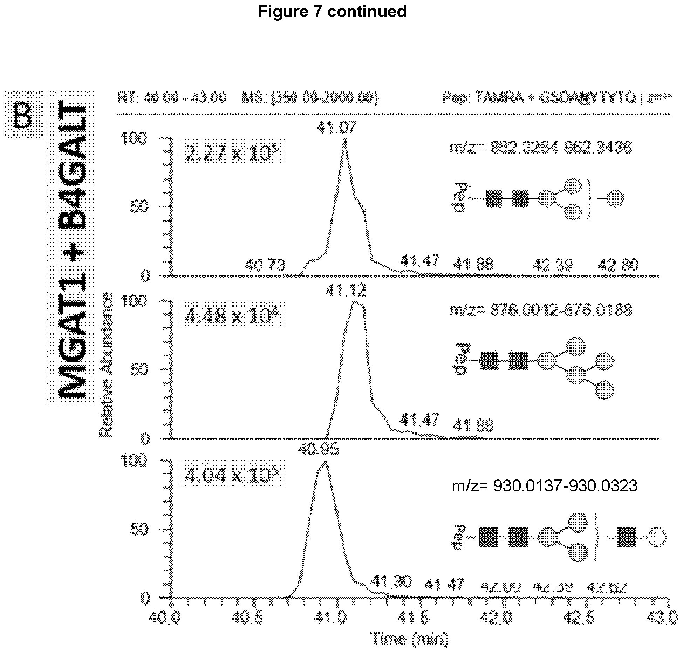

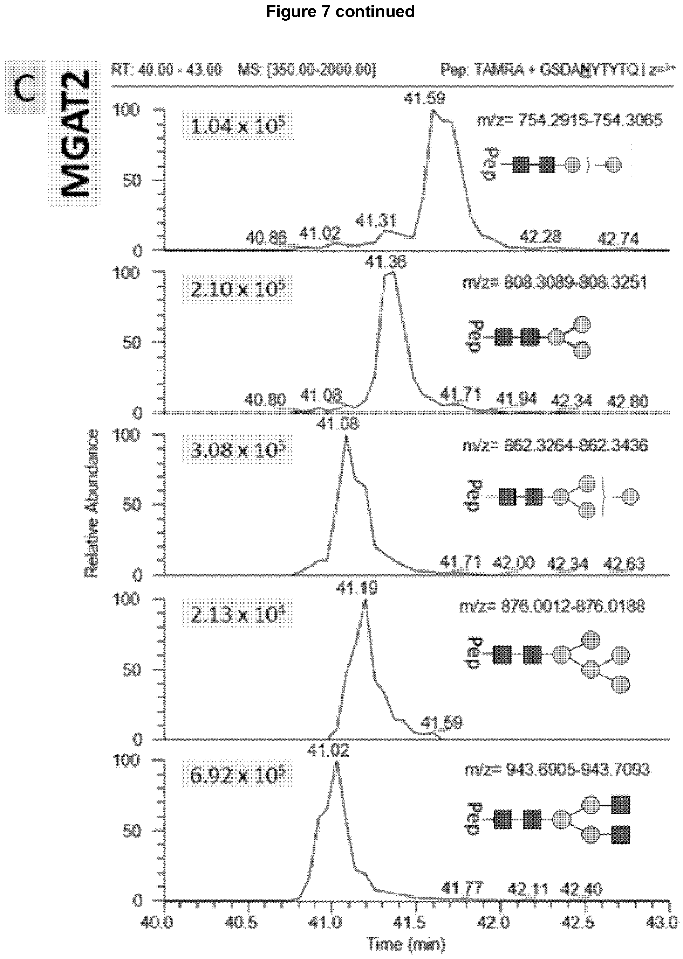

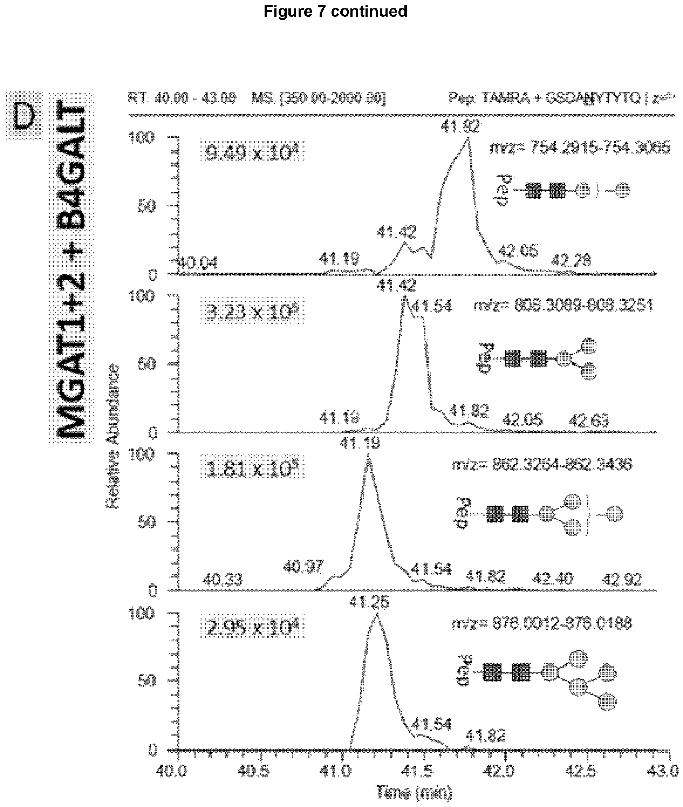

**Figure 7****:** shows the relative quantification of *in vitro N*-glycosylated synthetic TAMRA peptides for four different enzyme reactions (**A:** MGAT1, **B:** MGAT1 +B4GALT, **C:** MGAT1 +2, **D:** MGAT1 +2 +B4GALT). Extracted ion chromatograms (EIC MS¹) of the precursor ions of TAMRA + GSDANYTYTQ + *N*-glycan are depicted for each enzymatic reaction and show the retention and relative abundance of the different *N*-glycoforms. For each EIC the peak intensity in arbitrary units (e.g. 9.25 x 10⁵) is given for the respective glycopeptide precursor ions. Only EIC-MS¹ precursor ions with MS² spectra are selected.

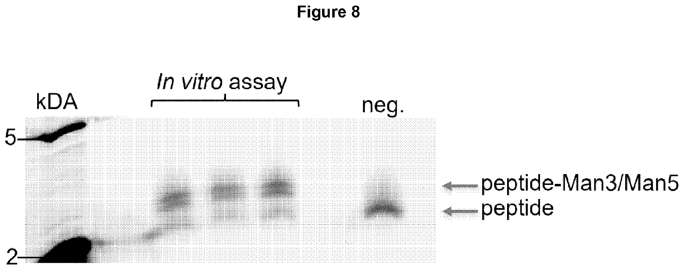

**Figure 8****:** shows Tris-Tricine PAGE of high-mannose LL-GlcNAc2Man3/Man5, with 21-mer peptide. The negative control included heat inactivated OST as a control of aglycosylated peptide and to verify OST activity. The assay was conducted in triplicates.

The following examples are included to demonstrate preferred embodiments of the invention. It should be appreciated by those skilled in the art that the techniques disclosed in the examples, which follow represent techniques discovered by the inventor to function well in the practice of the invention, and thus can be considered to constitute preferred modes for its practice. However, those skilled in the art should, in light of the present disclosure, appreciate that many changes can be made in the specific embodiments, which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Examples

### Abbreviations and Acronyms

- % (v/v): volume percent
- ACN: Acetonitrile
- ALG: Asparagine-linked glycosylation
- ALG1: β-1,4-mannosyltransferase
- ALG1ΔTM: Transmembrane deleted β-1,4-mannosyltransferase
- ALG2: α-1,3/1,6-mannosyltransferase
- APTS: (3-Aminopropyl)triethoxysilane
- β4GalT1ΔTM: transmembrane domaine deleted β-1,4-galactosyltransferase 1
- CGE-LIF: capillary gel electrophoresis with laser-induced fluorescence detection
- CHS: 3β-Hydroxy-5-cholestene 3-hemisuccinate
- DDM: n-Dodecyl-β-D-maltopyranoside
- DTT: Dithiothreitol
- *E. coli*: *Escherichia coli*
- ER: Endoplasmic reticulum
- Gal: Galactose
- GDP: Guanosine diphosphate
- GlcNAc: *N*-Acetylglucosamine
- GT: Glycosyltransferase
- HiDi: Hi-Di^{™} Formamide
- HILIC: Hydrophilic interaction chromatography
- IMAC: Immobilized metal affinity chromatography
- IPTG: Isopropyl β-D-1-thiogalactopyranoside
- LC-MS: Liquid Chromatography coupled mass spectrometry
- LLO: Lipid-linked oligosaccharide
- Man: Mannose
- MGAT1ΔTM: transmembrane domaine deleted α-1,3-mannosyl-glycoprotein 2-β-*N*-acetylglucosaminyltransferase
- MGAT2ΔTM: transmembrane domaine deleted α-1,6-mannosyl-glycoprotein 2-β-*N*-acetylglucosaminyltransferase
- MgCl₂: Magnesium chloride
- MnCl₂: Manganese chloride
- MWCO: Molecular weight cut off
- OD600: Optical density at 600 nm
- OST: Oligosaccharyltransferase
- PTM: Post-translational modification
- rpm: Rounds per minute
- *S*. *cerevisiae*: *Saccharomyces cerevisiae*
- ssOST: Single-subunit oligosaccharyltransferase
- TAMRA: 5-Carboxytetramethylrhodamine
- TFA: Trifluoroacetic acid
- UDP-Gal: Uridindiphosphate-galactose
- UDP-GIcNAc: Uridindiphosphate-*N*-Acetylglucosamine
- YFP: Yellow fluorescent protein

### Chemicals & Reagents

| **Chemical** | **Source of origin** |
|---|---|
| 2nd NormMix | glyXera GmbH |
| β4GalT1ΔTM in pET-28a(+) | BioCat |
| Acetonitril | VWR Chemicals |
| Agar-Agar powder | Carl Roth GmbH |
| APTS | Sigma-Aldrich |
| Baculovirus Insect Cell Medium | Novagen r |
| BioGel^{®} P-10 Media | Bio-Rad |
| BL21 (DE3) Competent *E.coli* | New England Biolabs GmbH |
| CHS | Sigma-Aldrich |
| DDM | Thermo Fisher Scientific^{™} |
| DTT | Sigma-Aldrich |
| Ethanol | Carl Roth GmbH |
| GDP-Man | Sigma-Aldrich |
| GeneScanTM 500 LIZ^{®} Size Standard | Applied Biosystems^{®} |
| Glycerol | Carl Roth GmbH |
| HCl | VWR Chemicals |
| HEPES | Carl Roth GmbH |
| Hi-Di^{™} Formamide | Thermo Fisher Scientific^{™} |
| IGEPAL^{®} CA-630 | Sigma-Aldrich |
| Imidazol | Sigma-Aldrich |
| IPTG | Sigma-Aldrich |
| Isopropanol | Sigma-Aldrich |
| K₂HPO₄ | Carl Roth GmbH |
| Kanamycin sulfate | Carl Roth GmbH |
| KH₂PO₄ | Carl Roth GmbH |
| Lemo21(DE3) Competent *E.coli* | New England Biolabs GmbH |
| L-rhamnose | New England Biolabs GmbH |
| Magnesium chloride | Sigma-Aldrich |
| Manganese chloride | Merck |
| Methanol | Fisher Scientific |
| MGAT1ΔTM in pET-28a(+) | BioCat |
| MGAT2ΔTM in pET-28b(+) | BioCat |
| MOPS | Carl Roth GmbH |
| NaOH | Carl Roth GmbH |
| Phytanyl-PP-Chitobiose | Chiroblock |
| Pierce BCA Protein Assay Kit | Thermo Fisher Scientific^{™} |
| Reducing Agent | glyXera GmbH |
| S. *cerevisiae* ORF ALG2 (YGL065C) | Horizon |
| Shuffle T7 Express LysY Competent *E. coli* | New England Biolabs GmbH |
| Sodium chloride | Carl Roth GmbH |
| TEA | AppliChem Panreac |
| TFA | Sigma-Aldrich |
| Tryptone | Carl Roth GmbH |
| UDP-Gal | Sigma-Aldrich |
| UDP-GlcNac | Sigma-Aldrich |
| Yeast extract | Carl Roth GmbH |

### Methods

### 1. Gene expression and enzyme purification

### 1.1 Gene expression of ALG1ΔTM, MGAT1ΔTM, β4GalT1ΔTM and MGAT2ΔTM in E. coli

The plasmid for ALG1ΔTM was transferred into and expressed by *E. coli* BL21 (DE3) according to Rexer et al. (2018) Biotechnol Bioeng 115, 192-205.

Gene sequences of MGAT1ΔTM and β4GaIT1ΔTM were inserted into expression vector pET-28a(+) and gene sequence of MGAT2ΔTM was inserted into expression vector pET-28b(+). Both vectors harbor an *N*-terminal 6-fold histidine tag (His₆-tag) for purification. Vectors were cloned either in Shuffle^{®} T7 Express *lysY,* for MGAT2ΔTM, or BL21(DE3), for MGAT1ΔTM and β4GaIT1ΔTM, competent *E*. *coli* cells. Positive transformants, selected by antibiotics, were used for protein expression. In general, cultures were cultivated in TB medium under agitation at 30°C. Overnight cultures were inoculated to an optical density at 600 nm (OD₆₀₀) of 0.1. Gene expression was induced at OD₆₀₀ = 0.6 by the addition of 0.4 mM IPTG and cultures were cooled down to 16 °C. Cells were harvested after overnight incubation by centrifugation (7,192 x g, 20 min, 4 °C) and cell pellets were stored at - 20 °C until further usage.

### 1.2 Gene expression of ALG2 in Saccharomyces cerevisiae and membrane fraction preparation

ALG2 expression was conducted according to Rexer et al. (2020) J Biotechnol 322, 54-65 using the yeast strain Yeast ORF ALG2 (YGL065C), which was purchased from the Dharmacon^{™} Yeast ORF Collection (Cambridge, United Kingdom). For gene expression, 200 mL synthetic drop-out medium without uracil was inoculated with an overnight culture to an OD₆₀₀ = 0.3. Cultivation was performed at 30 °C under agitation at 120 rpm until OD₆₀₀ reached 1.2. Gene expression was induced by adding 100 mL YP medium supplemented with 2% galactose (final concentration). Cells were harvested after 23 h of cultivation (120 rpm, 30°C) by centrifugation (6,000 x g, 20 min), washed once with ice cold water and cell pellets were stored at -20°C. Cell pellets were resuspended in buffer A (30 mM Tris (pH 7.5), 3 mM MgCl₂, 0.1% IGEPAL) at 1000 bar for 3 cycles. Cell debris was removed by centrifugation for 20 min at 8,000 x g (4 °C), followed by ultracentrifugation (100,000 x g for 45 min at 4 °C). Membrane fractions were solubilized in buffer A with 50% (v/v) glycerol.

### 1.3 Gene expression of T. brucei STT3A in Sf9 cells

STT3A expression and purification were performed according to Ramirez et al. (2017) using flashBAC DNA (Oxford Expression Systems) and Sf9 insect cells (Merck, Darmstadt, Germany) (J Biotechnol 2020, 322, 54-65; Glycobiology 2017, 27, 525-535). A synthetic gene of *Trypanosomas brucei* coding for STT3A with a 10-fold histidine and YFP tag was purchased from Thermo Fisher Scientific.

### 1.4 Enzyme purification

Enzyme solutions were prepared by cell disruption using a high-pressure homogenizer (3 cycles, 1,000 bar, 4 °C) followed by centrifugation (7,192 x g, 20 min, 4 °C). Supernatants were then applied to an equilibrated immobilized metal affinity chromatography (IMAC) column using Ni Sepharose HP columns from GE Healthcare (Chicago, USA). Purified enzyme solutions were desalted using Amicon^{®} Ultra 0.5mL Filters from Merck (Darmstadt, Germany) with a molecular weight cut-off of 10 kDa according to the manufacturer's instruction. Desalted enzymes were stored in 50% (v/v) glycerol at -20 °C.

### 2 Glycan analysis

### 2.1 CGE-LIF

The synthesis of LLOs was analyzed by multiplexed capillary gel electrophoresis with laser-induced fluorescence detection (CGE-LIF) (J Proteome Res 2010, 9, 6655-6664; Electrophoresis 2008, 29, 4203-4214; Hennig et al "N-Glycosylation Fingerprinting of Viral Glycoproteins by xCGE-LIF" in Carbohydrate based vaccines, Methods and Protocols, 1331, 123-143). Glycans were released from the lipid tail by mild acidic hydrolysis using 50 mM HCI for 30 min at 90 °C and neutralized with 100 mM NaOH. Afterwards freeze-dried glycans were labeled with APTS and excess APTS was removed by hydrophilic interaction chromatography with solid phase extraction (HILIC-SPE) (J Proteome Res 2010, 9, 6655-6664). For analysis 1 µL sample, 9.6 µL HiDi^{™}, 0.7 µL LIZ^{™} base pair standard and 0.7 µL 2^{nd} NormMix were mixed and injected to a 4-capillary DNA sequencer (3130 Genetic Analyzer, Life Technologies, California, USA) with a POP7^{™} polymer matrix (50 cm). Data analysis was carried out using glyXtool^{CE} (glyXera GmbH, Magdeburg, Germany).

### 2.2 Mass spectrometry

Prior to the analysis by mass spectrometry, enzymes and larger molecules were separated from the reaction products by filtration using a 10 kDa molecular weight cut-off filters (Amicon^{®} Ultra 0.5mL Filters, Merck, Darmstadt, Germany). Samples were desalted by manual C18 chromatography. Therefore, samples were reconstituted in up to 1 mL 0.1% trifluoroacetic acid (TFA) to ensure that pH is lower than 3. HyperSEP C18 columns (ThermoFisher Scientific) were conditioned with 3 mL of conditioning buffer (90% methanol, 10% dH₂O, 0.1% TFA). Prior to sample loading the column was equilibrated with 0.1% TFA in water. Samples were desalted by using 0.1% TFA in water and glycopeptides were eluted with 50% acetonitrile (ACN) in water containing 0.1% TFA. For detergent removal HiPPR^{™} detergent removal spin columns (ThermoFisher Scientific) were used according to the manufacturer's instruction. Sample measurement and glycoproteomic analysis was conducted as described in previous studies (Hoffmann et al, 2018 Proteomics 18, e1800282; J Biotechnol 2020, 322, 54-65). Briefly, TAMRA glycopeptides were analyzed by reverse-phase liquid chromatography coupled online to RP-LC-ESI-OT-OT MS/MS (LTQ Orbitrap Elite hybrid mass spectrometer, Thermo Fisher Scientific) using higher-energy collision dissociation fragmentation (HCD) at a normalized collision energy of 20 (NCE 20, HCD.low). Glycopeptide mass spectra were evaluated using Xcalibur Qual Browser 2.2 (Thermo Fisher Scientific) as well as Byonic (Protein Metrics, San Carlos, CA). Relative quantification of TAMRA glycopeptides was performed using Byologic (Protein Metrics).

### 3 Synthesis of lipid-linked oligosaccharides

In general, all reactions were performed in a volume of 100 µL, at 30 °C and under agitation at 300 rpm. Typically, 10 µL aliquots were taken from reaction batches for CGE-LIF analysis.

### Example 1: Synthesis of LL-GlcNAc₂Man₃

The one-pot multi-enzyme run was carried out for 8 h. The reaction batch contained: 50 mM MOPS (pH 6.8), 0.1% IGEPAL, 10 mM MgCl₂ (buffer B), 1 mM DTT, 0.1 mM phytanyl-PP-chitobiose, 2 mM GDP-mannose, 0.1 mg/mL purified ALG1ΔTM and 35% (v/v) ALG2 yeast membrane fraction. After 8 h the reaction batch was quenched for 5 min at 90 °C to ensure enzyme inactivity.

Results of the xCGE-LIF measurement after a reaction time of 8 h are depicted in Figure 3. The electropherogram shows a large fraction of the product, LL-GlcNAc₂Man₃ (178 MTU"), but also small amounts of LL-GlcNAc₂Man₂ at 140 MTU" and LL-GIcNAC₂Man₅ at 248 MTU". Other by-products were not observed in this reaction.

### Example 2: Sequential synthesis of LL-GlcNAc₂Man₃GlcNAc₁Gal₁[3]

For the *in vitro* synthesis of LL-GlcNAc₂Man₃GlcNAc₁GaI₁[3] two sequential runs were conducted. The first reaction batch contained 25 mM HEPES (pH7), 0.1% IGEPAL and 10 mM MnCl₂ (buffer C) with 6 mM UDP-GlcNAc, 50% (v/v) LL-GlcNAc₂Man₃ and 15% (v/v) MGAT1ΔTM. After 24 h at 30 °C, reactions were quenched for 5 min at 90 °C.

Figure 4 (red) shows that after 24 h a large proportion of LL-GlcNAc₂Man₃ was converted to LL-GlcNAc₂Man₃GlcNAc₁[3] (217 MTU"). Small amounts of intermediates such as LL-GlcNAc₂Man₂ (140 MTU") and LL-GIcNAC₂Man₅ (248 MTU") that originate from the LL-GlcNAc₂Man₃ stock solution were also identified.

The second reaction batch was conducted in buffer C, 6 mM UDP-Gal, 50% (v/v) LL-GlcNAc₂Man₃GlcNAc₁[3] and 25% (v/v) β4GaIT1ΔTM. The reaction was performed for 24 h and was quenched at 90 °C for 5 min.

Results of the reaction run after 24 h are shown in blue in Figure 4. LL-GlcNAc₂Man₃GlcNAc₁[3] from the previous reaction (Figure 4, red) was fully converted to LL-GlcNAc₂Man₃GlcNAc₁GaI₁[3] and appeared at approximately 260 MTU" in the electropherogram. Other products from preceding reaction runs are LL-GlcNAc₂Man₂ (140 MTU") (not shown), LL-GlcNAc₂Man₃ (175 MTU") and LL-GlcNAc₂Man₅ (248 MTU").

### Example 3: Stepwise synthesis of LL-GlcNAc₂Man₃GlcNAc₂Gal₂

*Sequential synthesis:* To synthesize LL-GlcNAc₂Man₃GlcNAc₂Gal₂, two reaction runs were carried out sequentially. The first reaction batch contained buffer C with 6 mM UDP-GlcNAc, 50% (v/v) Man3 and 12.5% (v/v) MGAT1ΔTM and MGAT2ΔTM, respectively. After 24 h reactions were quenched at 90 °C for 5 min and centrifuged. Figure 5 (red) shows that after a reaction of 24 hours, a large fraction of LL-GlcNAc₂Man₃ was converted to the lipid-linked complex-type structure LL-GlcNAc₂Man₃GlcNAc₂. Furthermore, intermediates of the reaction, LL-GlcNAc₂Man₂, LL-GlcNAc₂Man₃ and LL-GIcNAC₂Man₅ were also detected by CGE-LIF analysis.

The second reaction batch contained: buffer C, 6 mM UDP-Gal, 25% (v/v) β4GaIT1ΔTM and 50% (v/v) LL-GIcNAc₂Man₃GIcNAc₂[3/6]. The reaction was quenched at 90 °C for 5 min after 24 h of incubation. Here (Figure 5, blue), LL-GlcNAc₂Man₃GlcNAc₂[3/6] was fully converted to either LL-GlcNAc₂Man₃GlcNAc₂Gal₂[3/6] (331 MTU") or LL-GlcNAc₂Man₃GlcNAcGal[3] (262 MTU"). The result demonstrated that the synthesis of LL-GlcNAc₂Man₃GlcNAc₂Gal₂ was successful and a large proportion (~35%) of the final product was obtained.

*One-pot synthesis:* The one-pot synthesis of LL-GlcNAc₂Man₃GlcNAc₂ was performed in buffer C with 4 mM UDP-GlcNAc, 4 mM UDP-Gal, 35% (v/v) Man₃ and 15% (v/v) MGAT1ΔTM, MGAT2ΔTM and β4GalT1ΔTM, respectively. The reaction was performed for 36 h at 30 °C under agitation (350 rpm). After 0, 4, 8, 24 and 36 h aliquots of 10 µL were taken and the reaction was quenched for 5 min at 90 °C.

Samples were analyzed by xCGE-LIF analysis after 4 h, 8 h, 24 h and 36 h. Results of the reaction are depicted in Figure 6. After 4 h, LL-GlcNAc₂Man₃ was partly converted to LL-GlcNAc₂Man₃GlcNAc₁GaI₁[3]. Within the next 4 h the reaction was driven towards the site product LL-GlcNAc₂Man₃GlcNAc₁GaI₁[3] (262 MTU") and the final product LL-GlcNAc₂Man₃GlcNAc₂Gal₂ (331 MTU"). The peak intensity of LL-GlcNAc₂Man₃GlcNAc₂Gal₂[3/6] after 24 h is slightly higher than after 8 h. After 36 hours, the reaction showed no differences in comparison to the 24 hours sample. At the end of the run, the reaction mixture mainly contained LL-GlcNAc₂Man₃GlcNAc₁GaI₁[3] (262 MTU"). Nevertheless, it was shown that the synthesis of LL-GlcNAc₂Man₃GlcNAc₂Gal₂ was successful, even only in a small amount (~5%).

### Example 4: Transfer of LLOs to synthetic peptides by T. brucei STT3A

To analyze the transfer of LLOs, TAMRA-labeled synthetic peptides with the consensus sequence Asn-Xaa-Thr/Ser (wherein Xaa represents any amino acid except proline) and the following amino acid sequence, G-S-D-A-**N-Y-T**-Y-T-Q, were purchased from Biomatik (Cambrigde, Canada). The reaction was conducted in a volume of 100 µL and contained: 20 mM HEPES (pH 7.5), 10 mM MnCI2, 150 mM NaCl, 0.035% (w/v) DDM, 0.007% (w/v), 50% (v/v) LLO, 20 µM synthetic peptides, 36.3% (v/v) *T. brucei* STT3A and EDTA-free protease inhibitor (Roche, Basel, Switzerland).

Four one-pot *in-vitro* glycosylation reactions were conducted, to investigate the ability of recombinant *T. brucei* STT3A to transfer the complex-type structures LL-GlcNAc₂Man₃GlcNAc₁[3] and LL-GlcNAc₂Man₃GlcNAc₁Gal₁[3], LL-GlcNAc₂Man₃GlcNAc₂ and LL-GlcNAc₂Man₃GlcNAc₂Gal₂, from the lipid anchor to the asparagine residue of synthetic peptides following the *N*-glycosylation consensus motif. The transfer was performed using the products of the sequential reactions (see Figures 4 and 5) and was analyzed by LC-MS/MS.

In all runs, the amount of not *N*-glycosylated or deamidated peptides was approximately 90%. Only glycosylated peptides were considered to determine the relative proportion of transferred glycans (Table 1).

Non-glycosylated or deamidated peptides were not included. Around 64% of all glycosylated peptides contained a GlcNAc₂Man₅ *N*-glycan structure. LL-GIcNAC₂Man₅ was present in equal amounts in all reactions due to the naturally occurring ALG11 in the yeast membrane fraction of recombinant ALG2 (see Figures 4 and 5). As LL-GIcNAC₂Man₅ was only present in small amounts, the high proportion of glycosylated Man₅-peptides highlight the high affinity of *T. brucei* ssOST to LL-GlcNAc₂Man₅. Overall, the transfer of LL-GlcNAc₂Man₃GlcNAc₂Gal₂ was not detected by LC-MS/MS analysis.

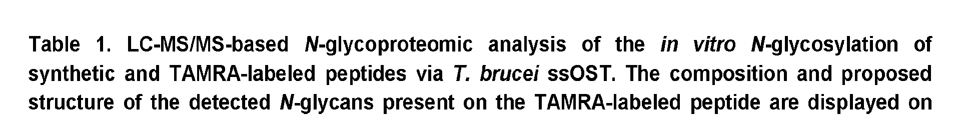

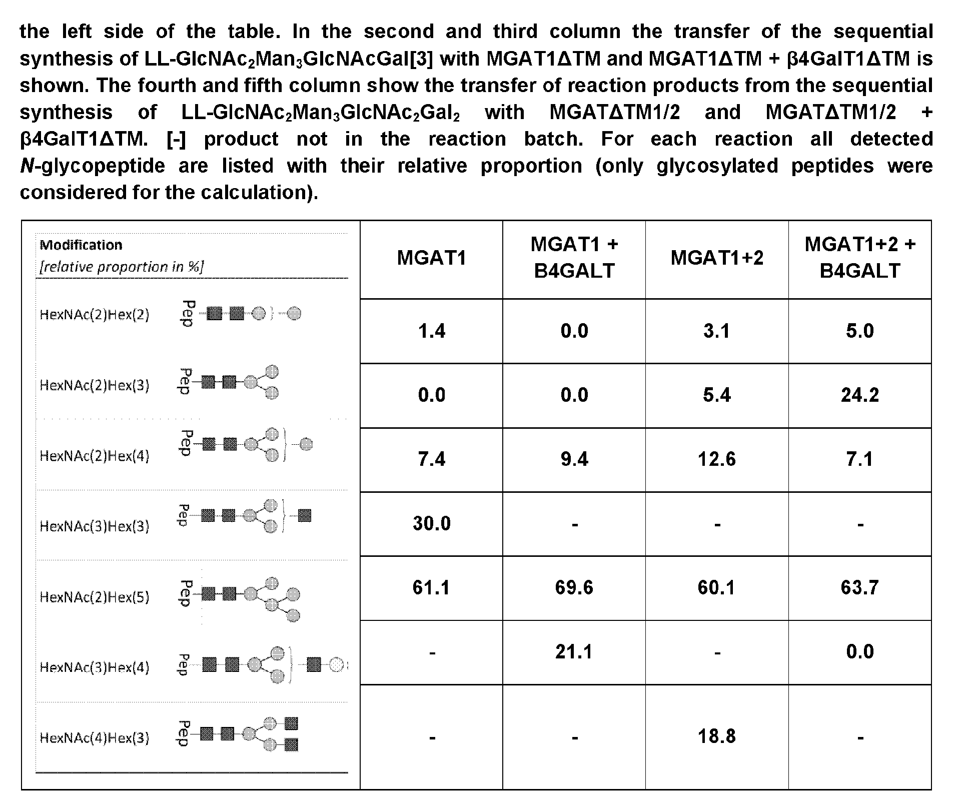

In the first run, MGAT1ΔTM was used to synthesize LL-GlcNAc₂Man₃GlcNAc₁[3]. After the subsequent *in vitro* glycosylation reaction using ssOST, the extracted ion chromatograms (EIS-MS) of identified glycopeptide spectra are depicted in Figure 7. Peptides with GlcNAc₂Man₂, GlcNAc₂Man₄, GlcNAc₂Man₅ and GlcNAc₂Man₃GlcNAc₁[3] *N*-glycan structures were identified. The fraction of glycopeptides containing GlcNAc₂Man₃GlcNAc₁[3] was 30% (see Table 1).

In the second run, the addition of MGAT1ΔTM and β4GaIT1ΔTM was used to generate LL-GlcNAc₂Man₃GlcNAc₁GaI₁[3]. GlcNAc₂Man₄, GlcNAc₂Man₅ and GlcNAc₂Man₃GlcNAc₁GaI₁[3] *N*-glycan structures were identified by LC-MS/MS measurement (Figure 7B). The *in vitro* glycosylation reaction yielded the GlcNAc₂Man₃GlcNAc₁Gal₁[3]-peptide with a proportion of 21.1% (Table 1). *N*-glycopeptides containing GlcNAc₂Man₃GlcNAcᵢ[3] were not detected by LC-MS/MS measurement. This finding is consistent with the LLO synthesis reaction run (see Figure 4). There, the side product LL-GlcNAc₂Man₃GlcNAc₁GaI₁[3] was not detected.

In the third run, MGAT1ΔTM and MGAT2ΔTM were applied to generate LL-GlcNAc₂Man₃GlcNAc₂ first, followed by using the ssOST to glycosylate the target peptide. The transfer of complex-type GlcNAc₂Man₃GlcNAc₂ to the synthetic peptide was successful (see Figure 7C and Table 1). Also, side-products such as peptides containing GlcNAc₂Man₂, GlcNAc₂Man₃, GlcNAc₂Man₄ and GlcNAc₂Man₅ were identified. The relative proportion of the GlcNAc₂Man₃GlcNAc₂-peptide was 18.8% of all glycosylated peptides in this reaction.

In the fourth run, after synthesizing LL-GlcNAc₂Man₃GlcNAc₂Gal₂, an *in vitro* reaction was initiated. The results show that this LLO is not transferred by the ssOST to synthetic peptides (Figure 7D & Table 1). Only the side-products GlcNAc₂Man₂, GlcNAc₂Man₃, GlcNAc₂Man₄ and GlcNAc₂Man₅ were identified.

### Example 5: Transfer of high-mannose LLOs to synthetic to a synthetic influenza hemaglutinin peptide by T. brucei STT3A

The reaction was conducted in a final volume of 50 µL containing reaction buffer (20 mM HEPES, 10 mM MnCI2, 150 mM NaCl, 0.035% (w/v) DDM, 0.007% (w/v) CHS and protease inhibitor), 25% (v/v) LL-GlcNAc₂Man₃/Man₅, 0.02 mM peptide and 20 nM OST. The OST was added step-wise at the beginning, after 4 h and 8 h of the reaction. The reaction was stopped after 24 h by heat (10 min, 90°C). Afterwards, a Tris-Tricine PAGE was performed and analyzed using a fluorescence scanner.

The peptide sequence was derived from Influenza A virus (strain A/Puerto Rico/8/1934 H1N1) Hemaglutinin (Uniprot Number: P03452; Amino acids 29-49) (SEQ ID NO: 2): TAMRA-STDTVDTVLEKNVTVTHSVNL-NH₂

## Claims

1. A method for producing a glycoprotein of general formula (**I**)
**[C-NH]ₒP** **(I)**
wherein C represents a carbohydrate of the following structure 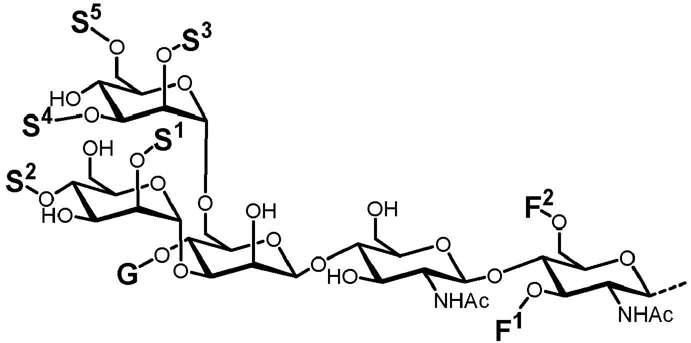
**o** is an integer representing the number of carbohydrates **C** which are bound to a peptide **P,**
**P** represents a peptide of at least 10 amino acids comprising at least **o**-times a consensus sequence of N-X-S/T, wherein X represents any amino acid except proline, and
**NH** represents an asparagine γ-amido group of the consensus sequence;
**F¹** and **F²** represent 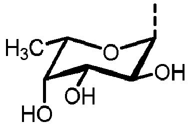 or -H; with the proviso that **F¹** and **F²** cannot be simultaneously 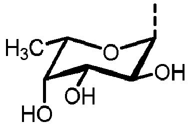
**G** represents 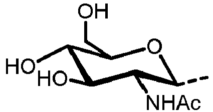 or -H;
**S¹** represents 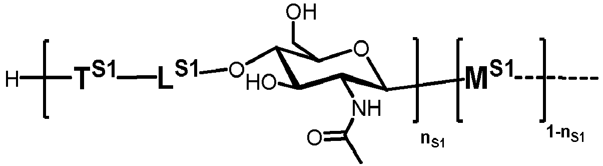
**S²** represents 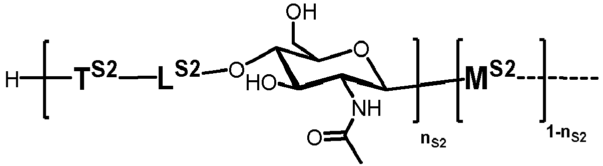
**S³** represents 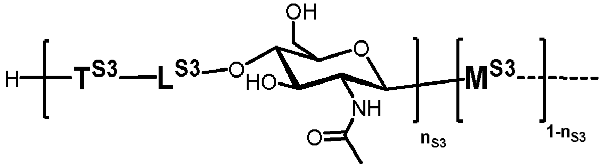
**S⁴** represents 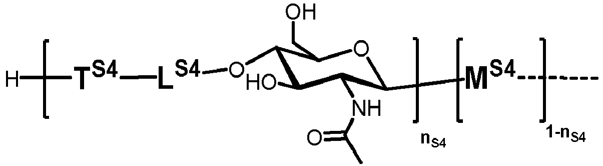
**S⁵** represents 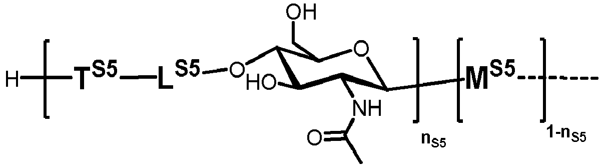
**M^{S1}** represents 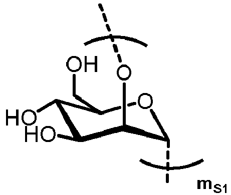
**M^{S2}** represents 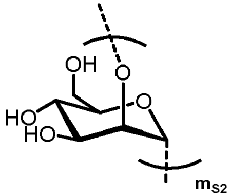
**M^{S3}** represents 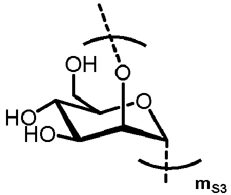
**M^{S4}** represents 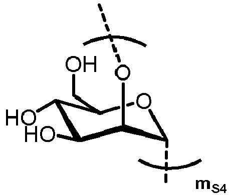 **M^{S5}** represents 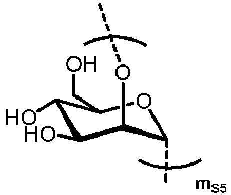
**L^{S1}** represents 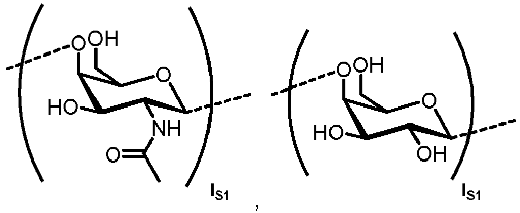 or 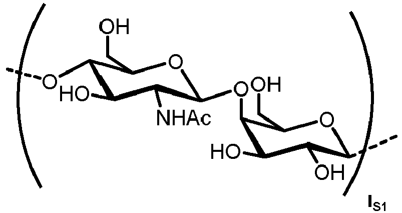
**L^{S2}** represents 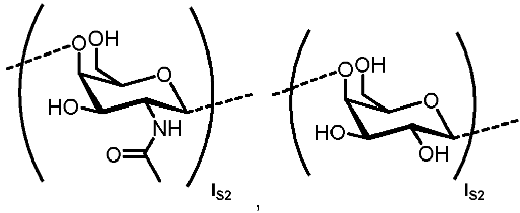 or 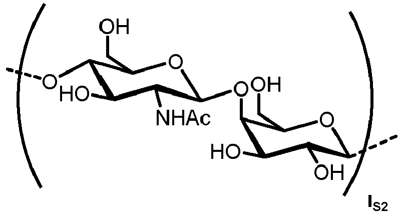
**L^{S3}** represents 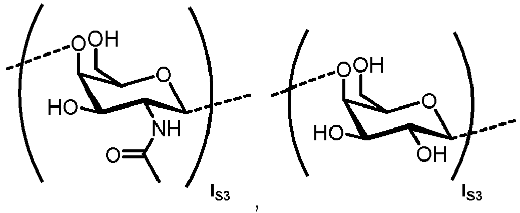 or 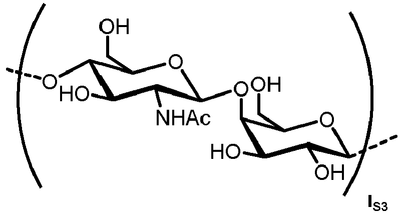
**L^{S4}** represents 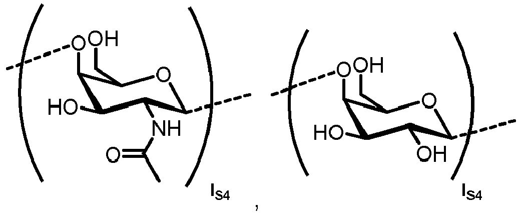 or 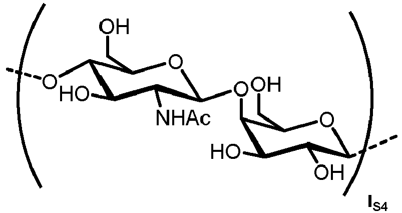
**L^{S5}** represents 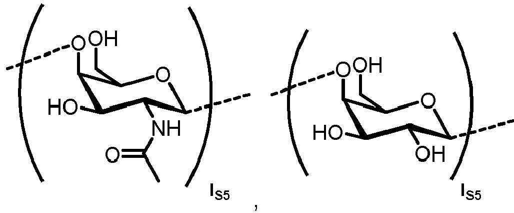 or 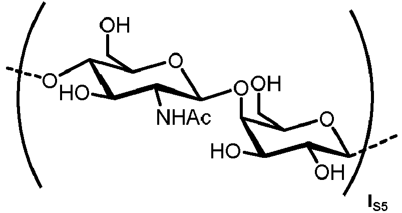
**T^{S1}**, **T^{S2}**, **T^{S3}**, **T^{S4}** and **T^{S5}** represent independently of each other: 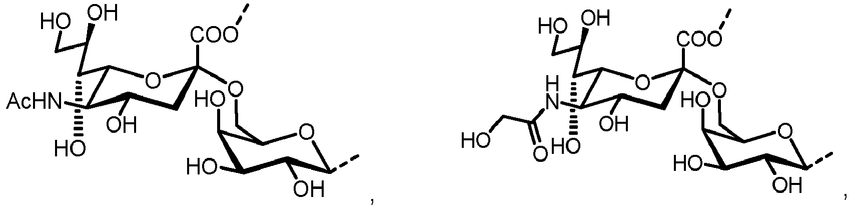 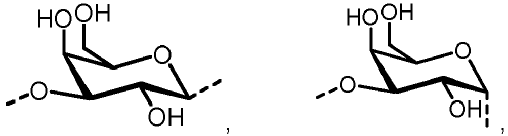 -SO₄- or a bond wherein m_{S1}, m_{S2}, m_{S3}, m_{S4}, M_{S5}, I_{S1}, I_{S2}, I_{S3}, I_{S4}, and I_{S5} represent independently of each other an integer selected from 0, 1, 2, 3, 4, 5, 6, 7, 8, 9, 10, 11, 12, 13, 14 and 15,
n_{S1}, n_{S2}, n_{S3}, n_{S4} and n_{S5} represent an integer selected from 0 and 1 ,
with the proviso that if n_{S3} = 1 then
n_{S1} = 1 and m_{S2} = m_{S4} = m_{S5} = 0; and
if n_{S3} = 0 then
i) n_{S2} = m_{S2} = m_{S3} = 0 , and
ii) m_{S1} + m_{S4} + m_{S5} ≥ 0 ;
comprising the steps:
A) providing a solution comprising a compound of formula (II) 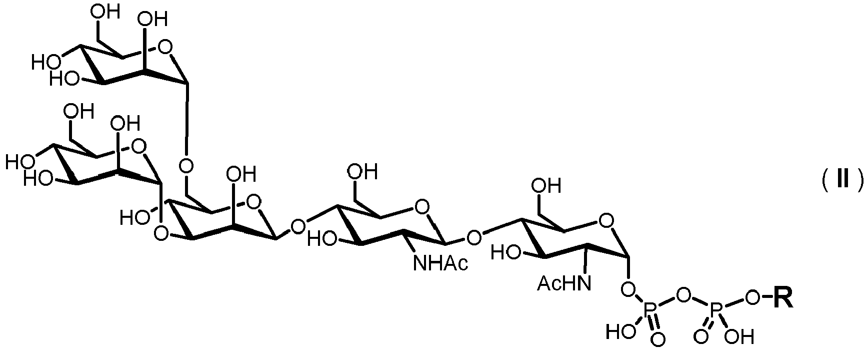 wherein R represents 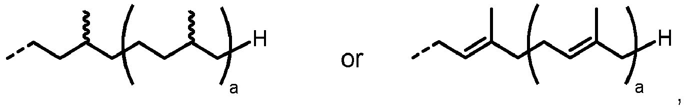 with a being an integer selected from 2, 3, 4, 5, 6, 7, 8, 9, 10, 11, 12, 13, 14 or 15,
B) mixing the solution with at least one nucleotide sugar and at least one glycosyltransferase enzyme and reacting a resulting mixture to produce a compound of formula (III) 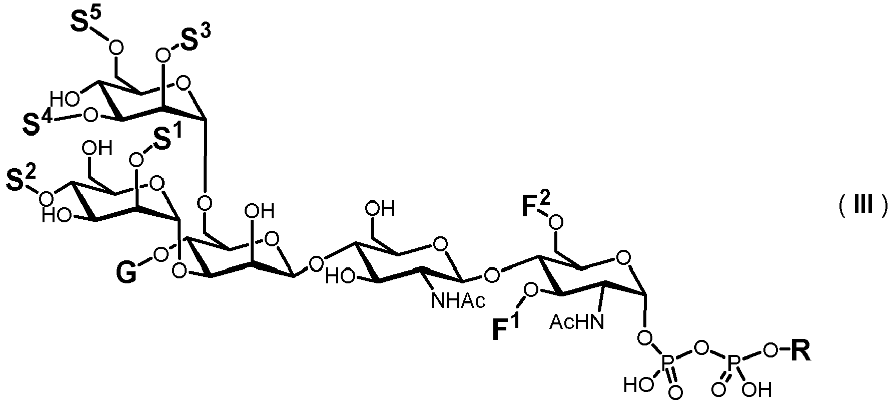 wherein the at least one nucleotide sugar is selected from GDP-mannose, UDP-galactose, UDP-GlcNAc, UDP-GaINAc, GDP-fucose, CMP-NeuAc and CMP-NeuGc,
wherein the at least one glycosyltransferase enzyme is selected from *N*-acetylglucosaminyltransferase, mannosyltransferase, glucosyltransferase, galactosyltransferase, fucosyltransferase and sialyltransferase;
C) reacting the compound of formula (**III**) with the peptide **P** in the presence of an oligosaccharyltransferase enzyme to produce the compound of formula (**I**).

2. The method according to claim 1, wherein the consensus sequence of peptide **P** is N-V-T or N-Y-T.

3. The method according to claim 1 or 2, wherein **P** represents a peptide having at least 20 amino acids.

4. The method according to any one of the claims 1 to 3, wherein the at least one glycosyltransferase enzyme is a transmembrane domain-deleted enzyme.

5. The method according to any one of the claims 1 to 4, wherein **P** is a therapeutic protein.

6. The method according to any one of the claims 1 to 5, wherein the oligosaccharyltransferase enzyme is STT3A protein from *Trypanosoma brucei.*

7. The method according to any one of the claims 1 to 6, wherein the *N*-acetylglucosaminyltransferase is an α-1,3-mannosyl-glycoprotein 2-β-*N*-acetylglucosaminyltransferase and/or an α-1,6-mannosyl-glycoprotein 2-β-*N*-acetylglucosaminyltransferase and/or a β-1,4-mannosyl-glycoprotein 4-β-*N*-acetylglucosaminyltransferase and/or an α-1,3-mannosyl-glycoprotein 4-β-*N*-acetylglucosaminyltransferase, wherein the mannosyltransferase is an α-1,2-mannosyltransferase and/or an α-1,3-mannosyltransferase and/or an α-1,6-mannosyltransferase and/or an α-1,3/1,6-mannosyltransferase, wherein the galactosyltransferase is an α-1,3-galactosyltransferase and/or a β-1,4-galactosyltransferase, wherein the sialyltransferase is an α-2,3-sialyltransferase and/or an α-2,6-sialyltransferase, wherein the fucosyltransferase is an α-1,2-fucosyltransferase and/or an α-1,3-fucosyltransferase and/or an α-1,6-fucosyltransferase, and wherein the glucosyltransferase is an α-1,2-glucosyltransferase and/or an α-1,3-glucosytransferase.

8. The method according to any one of the claims 1 to 7, wherein **T^{S1}**, **T^{S2}**, **T^{S3}**, **T^{S4}**, and **T^{S5}** represent a bond.

9. The method according to any one of the claims 1 to 8, wherein I_{S1}, I_{S2}, I_{S3}, I_{S4}, and I_{S5} represent independently of each other an integer selected from 0, 1, 2 and 3.

10. The method according to any one of the claims 1 to 9, wherein m_{S1}, m_{S2}, m_{S3}, m_{S4}, and m_{S5} represent independently of each other an integer selected from 0, 1, 2, 3, 4 and 5.

11. The method according to any one of the claims 1 to 10, wherein **F¹**, **F² and G** represent -H.

12. The method according to any one of the claims 1 to 11, wherein the peptide **P** is aglycosylated.

13. A method for producing a glycoprotein of general formula (**Ia**)
**[Ca-NH]ₒP** **(Ia)**
wherein **Ca** represents a carbohydrate of the following structure 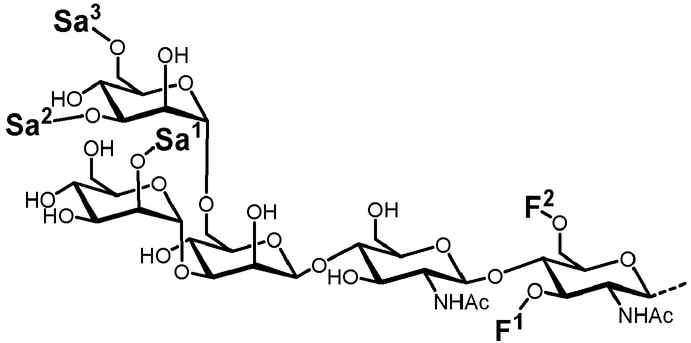
**o** is an integer representing the number of carbohydrates **C** which are bound to a peptide **P,**
**P** represents a peptide of at least 10 amino acids comprising at least **o**-times a consensus sequence of N-X-S/T, wherein X represents any amino acid except proline, and
**NH** represents an asparagine γ-amido group of the consensus sequence;
**F¹** and **F²** represent 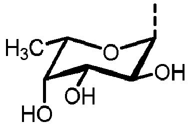 or -H; with the proviso that **F¹** and **F²** cannot be simultaneously 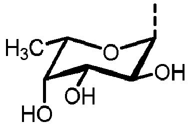
**Sa¹** represents 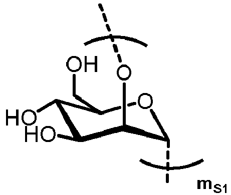
**Sa²** represents 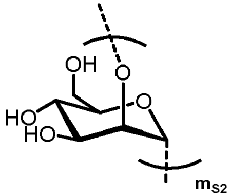
**Sa³** represents 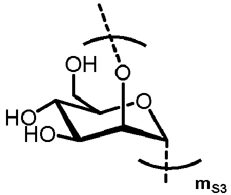 wherein m_{S1}, m_{S2}, and m_{S3} represent independently of each other an integer selected from 0, 1, 2, 3, 4, 5, 6, 7, 8, 9, 10, 11, 12, 13, 14 and 15,
comprising the steps:
Aa) providing a solution comprising a compound of formula (**IIIa**) 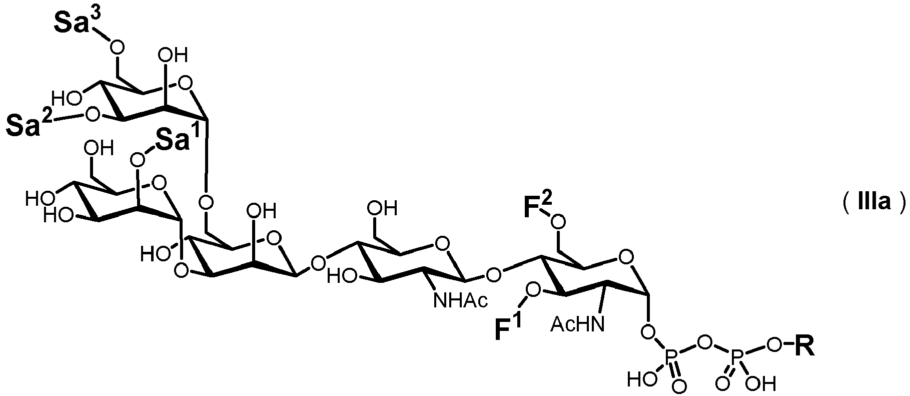 wherein **R** represents 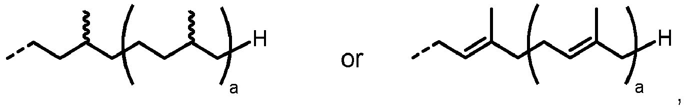 with a being an integer selected from 2, 3, 4, 5, 6, 7, 8, 9, 10, 11, 12, 13, 14 or 15,
Ba) reacting the compound of formula (**IIIa**) with the peptide **P** in the presence of an oligosaccharyltransferase enzyme to produce the compound of formula (**Ia**).

14. A method for producing a compound of general formula (**IIIb**) 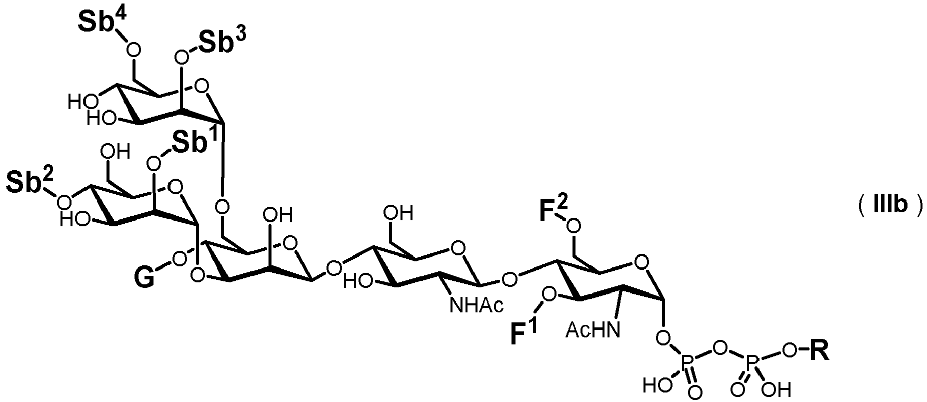 wherein
**F¹** and **F²** represent 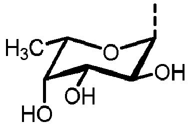 or -H; with the proviso that **F¹** and **F²** cannot be simultaneously 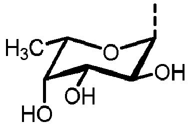
**G** represents 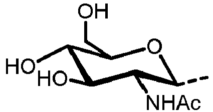 or -H;
**Sb¹** represents 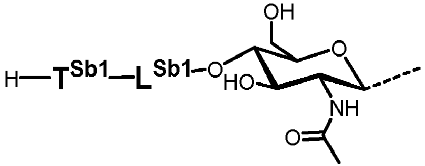
**Sb²** represents 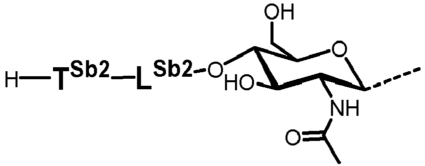 or -H
**Sb³** represents 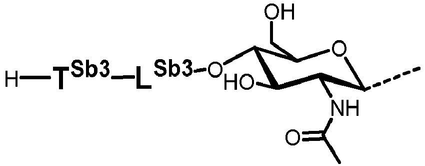
**Sb4** represents 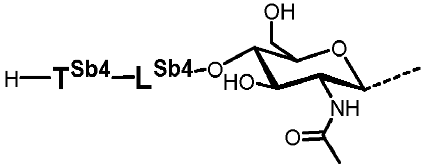 or -H
**L^{Sb1}** represents 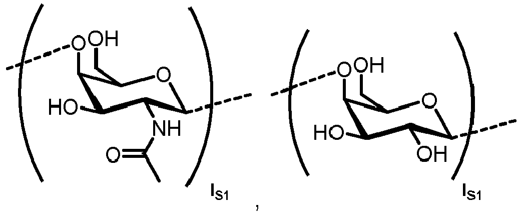 or 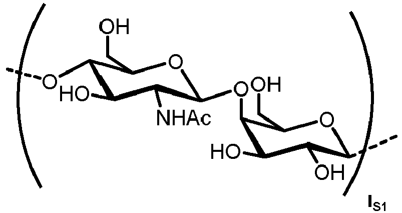
**L^{Sb2}** represents 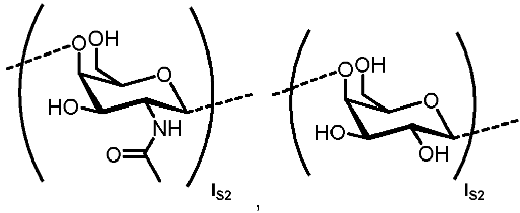 or 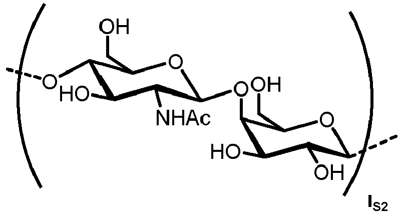
**L^{Sb3}** represents 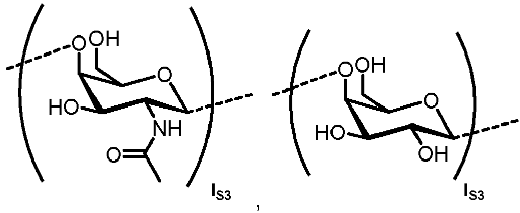 or 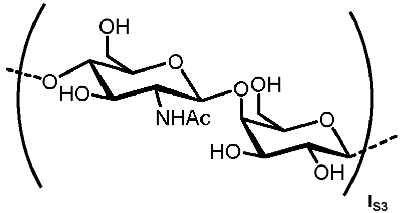
**L^{Sb4}** represents 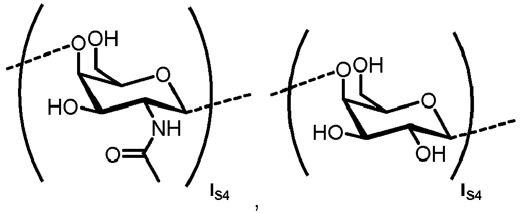 or 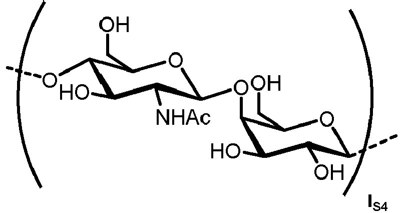
**T^{Sb1}**, **T^{Sb2}**, **T^{Sb3}** and **T^{Sb4}** represent independently of each other: 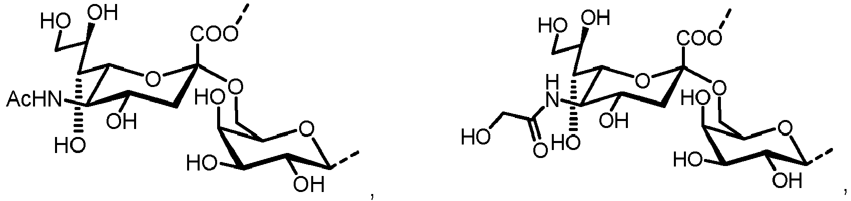 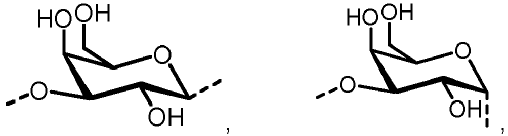 -SO₄- or a bond wherein I_{S1}, I_{S2}, I_{S3}, and I_{S4} represent independently of each other an integer selected from 0, 1, 2, 3, 4, 5, 6, 7, 8, 9, 10, 11, 12, 13, 14 and 15,
comprising the steps:
Ab) providing a solution comprising a compound of formula **(IIb)** 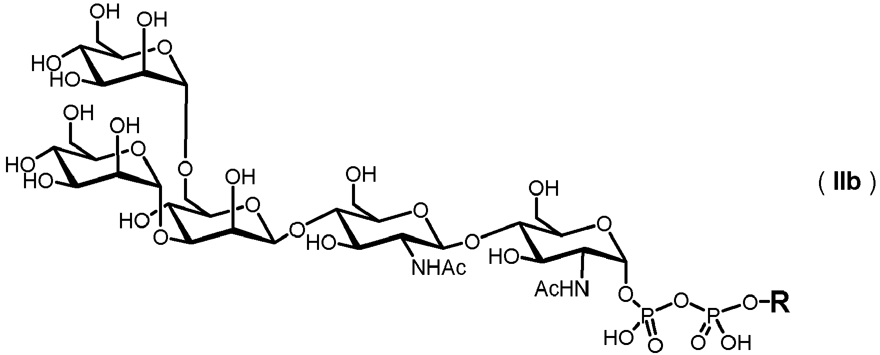 wherein **R** represents 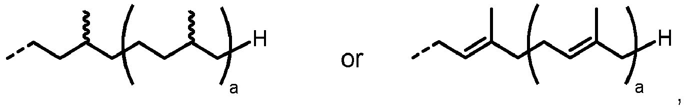 with a being an integer selected from 2, 3, 4, 5, 6, 7, 8, 9, 10, 11, 12, 13, 14 or 15,
Bb) mixing the solution with at least one nucleotide sugar and at least one glycosyltransferase enzyme and reacting a resulting mixture to produce the compound of formula **(IIIb),** wherein the at least one nucleotide sugar is selected from GDP-mannose, UDP-galactose, UDP-GlcNAc, UDP-GalNAc, GDP-fucose, CMP-NeuAc and CMP-NeuGc, and wherein the at least one glycosyltransferase enzyme is selected from *N*-acetylglucosaminyltransferase, mannosyltransferase, glucosyltransferase, galactosyltransferase, fucosyltransferase and sialyltransferase.

15. A method for producing a compound of general formula **(Ic)**
**[C-NH]ₒP** **(Ic)**
wherein **C** represents a carbohydrate of the following structure 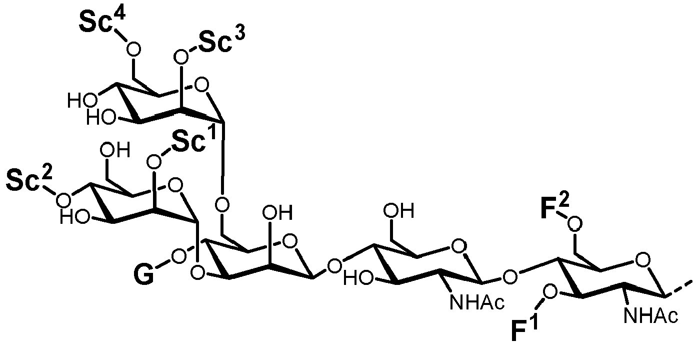
**o** is an integer representing the number of carbohydrates **C** which are bound to a peptide **P,**
**P** represents a peptide of at least 10 amino acids comprising at least **o**-times a consensus sequence of N-X-S/T, wherein X represents any amino acid except proline, and
**NH** represents an asparagine γ-amido group of the consensus sequence wherein
**F¹** and **F²** represent 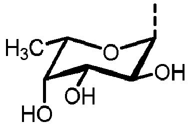 or -H; with the proviso that **F¹** and **F²** cannot be simultaneously 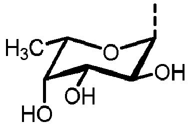
**G** represents 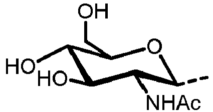 or -H;
**Sc¹** represents 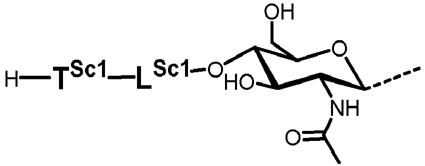
**Sc²** represents 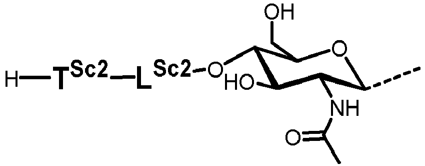 or -H
**Sc³** represents 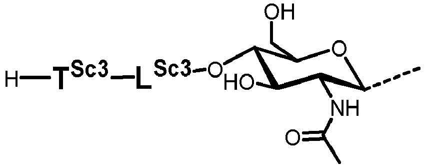
**Sc⁴** represents 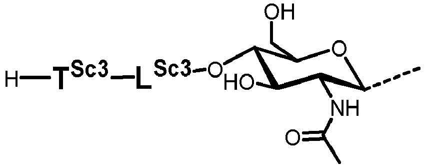 or -H
**L^{Sc1}** represents 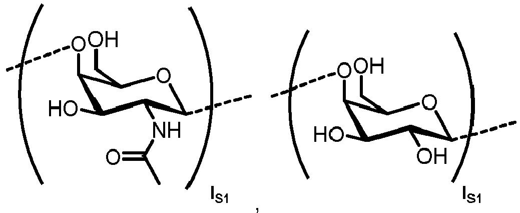 or 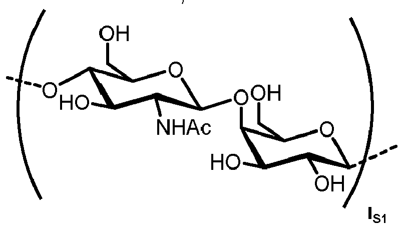
**L^{Sc2}** represents 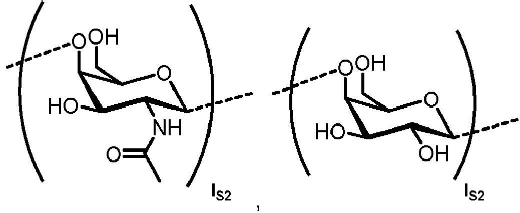 or 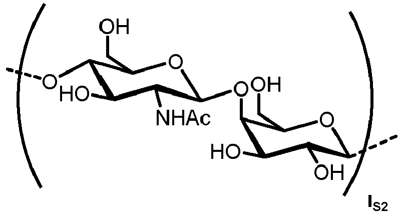
**L^{Sc3}** represents 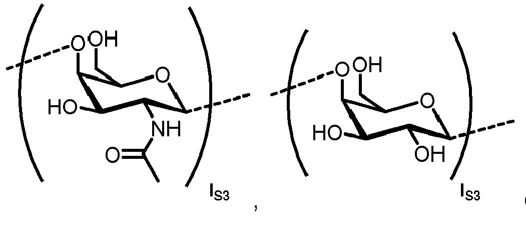 or 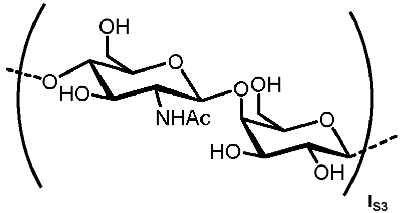
**L^{Sc4}** represents 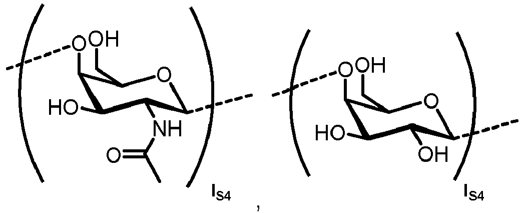 or 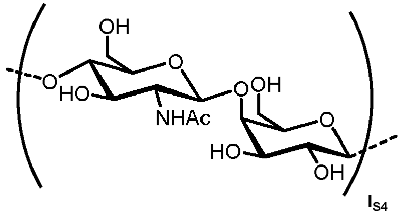
**T^{Sc1}, T^{Sc2}**, **T^{Sc3}**, and **T^{Sc4}** represent independently of each other: 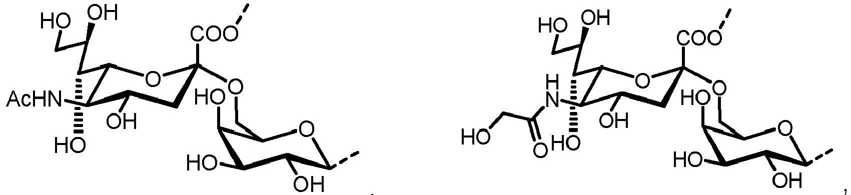 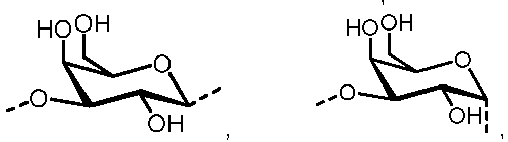 -SO₄- or a bond wherein I_{S1}, I_{S2}, I_{S3}, and I_{S4} represent independently of each other an integer selected from 0, 1, 2, 3, 4, 5, 6, 7, 8, 9, 10, 11, 12, 13, 14 and 15,
comprising the steps:
Ac) providing a solution comprising a compound of formula **(IIc)** 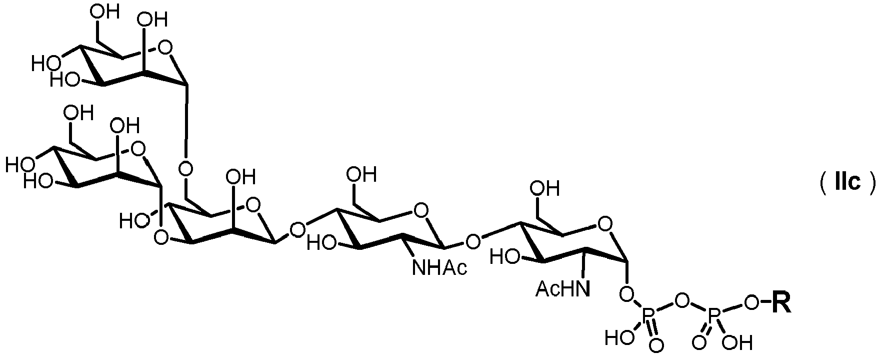 wherein **R** represents 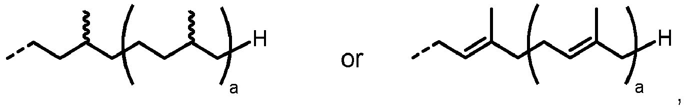 with a being an integer selected from 2, 3, 4, 5, 6, 7, 8, 9, 10, 11, 12, 13, 14 or 15,
Bc) reacting the compound of formula **(IIc)** with the peptide **P** in the presence of an oligosaccharyltransferase enzyme to produce the compound of formula **(IVc)**
**[Cc-NH]ₒP** **(IVc** )
wherein **Cc** represents a carbohydrate of the following structure 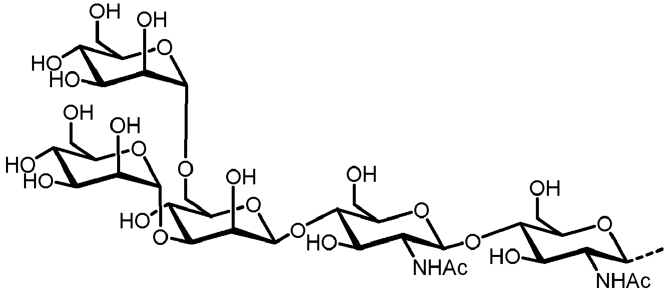
Cc) mixing the compound of formula **(IVc)** with at least one nucleotide sugar and at least one glycosyltransferase enzyme and reacting a resulting mixture to produce the compound of formula **(Ic),** wherein the at least one nucleotide sugar is selected from GDP-mannose, UDP-galactose, UDP-GlcNAc, UDP-GalNAc, GDP-fucose, CMP-NeuAc and CMP-NeuGc, and wherein the at least one glycosyltransferase enzyme is selected from *N*-acetylglucosaminyltransferase, mannosyltransferase, glucosyltransferase, galactosyltransferase, fucosyltransferase and sialyltransferase.
